# EUROPEAN PATENT SPECIFICATION

(11) **EP 3 622 936 B1**
(45) Date of publication and mention of the grant of the patent: **30.12.2020**
(21) Application number: 19195846.1
(22) Date of filing: 06.09.2019
(51) Int. Cl.: A61H 3/04

(54) **WALKING ASSIST DEVICE**
GEHHILFEVORRICHTUNG
DISPOSITIF D'ASSISTANCE À LA MARCHE

(30) Priority: 12.09.2018 JP 2018170555
(43) Date of publication of application: 18.03.2020
(73) Proprietor: JTEKT CORPORATION, Osaka-shi, Osaka 542-8502 (JP)
(72) Inventor: KANAYA, Manabu, Osaka-shi,, Osaka 542-8502 (JP); SHIBATA, Yoshiyuki, Osaka-shi,, Osaka 542-8502 (JP); TAKEUCHI, Shinji, Osaka-shi,, Osaka 542-8502 (JP)
(74) Representative: TBK

(56) References cited:
- EP-A1- 3 205 322
- WO-A1-2016/136370
- WO-A1-2017/104847
- DE-A1-102016 217 078
- KR-B1- 100 717 397
- US-B2- 9 510 992

## Description

### BACKGROUND OF THE INVENTION

### 1. Field of the Invention

The present invention relates to a walking assist device.

### 2. Description of the Related Art

In order for a user that can walk on his/her own to walk in a higher-quality walking state, it is very important to swing his/her arms correctly in synchronization with his/her legs in a correct posture with his/her body trunk straight without leaning on a walker.

For example, Japanese Patent Application Publication No. 2009-106446 (JP 2009-106446 A) describes a walking cart (corresponding to the walking assist device) that includes a pair of right and left front wheels, rear wheels, main frames (corresponding to the frame), side frames (corresponding to the rails), sliders (corresponding to the movable handles), handles (corresponding to the movable handles), and connecting rods. The sliders, to which the handles are fixed, are slidable back and forth along the side frames. The sliders are connected to the rear wheels via the connecting rods. Consequently, when a user slides the right and left sliders alternately back and forth by walking while grasping the right and left handles with his/her right and left hands and swinging his/her arms, the right and left rear wheels are rotationally driven. That is, the walking cart moves together with the user who walks while swinging his/her arms, and the power source of the walking cart is the force of the user to swing his/her arms back and forth.

In the walking cart described in JP 2009-106446 A, the range of front-rear swing of the arms is fixed by a link mechanism constituted from the handles, the sliders, the connecting rods, and the rear wheels, irrespective of the stride length. Thus, it is difficult for the user to adjust motion of the legs (stride length) and motion of the arms (range of arm swing) in conjunction with each other, and the user cannot walk with a range of front-rear swing of the arms that is appropriate for the user. In order to perform training for walking in a high-quality natural walking state, it is preferable to walk with a range of front-rear swing of the arms that is suitable for the user. As discussed above, however, the walking cart described in JP 2009-106446 A cannot correct the walking state of the user into a high-quality natural walking state in which the user swings his/her arms with a correct range in synchronization with his/her legs in a correct posture with his/her body trunk straight. Document EP 3205322 A1 discloses an electric vehicle in which a front wheel can run onto a step without need of an operation causing a large load to users. Further, document WO 2017/104847 A1 discloses a walk training system and walk training device.

### SUMMARY OF THE INVENTION

It is an object of the present invention to provide a walking assist device that can appropriately correct the walking state of a user.

An aspect of the present invention provides a walking assist device including:
a frame;
a pair of right and left arm portions provided on the frame;
a pair of right and left grasp portions provided on the pair of right and left arm portions, the grasp portions being graspable by a user and movable in a front-rear direction with respect to the frame;
a plurality of wheels provided at a lower end of the frame and including a drive wheel;
a drive unit that drives the drive wheel to cause the walking assist device to travel forward or rearward;
a grasp portion drive unit that is configured to move each of the grasp portions in the front-rear direction with respect to the frame;
a battery that serves as a power source for the drive unit and the grasp portion drive unit;
a control unit that controls the drive unit; and
a grasp portion state detection unit that detects a state of each of the grasp portions.

The control unit has:
a grasp portion state observation unit that observes a grasp portion state, which is a
   state of each of the grasp portions, based on a detection signal from the grasp portion state detection unit;
a walking state evaluation unit that evaluates a walking state of the user based on the grasp portion state observed using the grasp portion state observation unit; and
a correction adjustment unit that adjusts a control command for at least one of the drive unit and the grasp portion drive unit based on the walking state evaluated using the walking state evaluation unit.

With the walking assist device according to the aspect described above, the walking state of the user can be corrected appropriately.

In the walking assist device according to the aspect described above, the walking state evaluation unit may estimate an arm swing state of the user based on the observed grasp portion state; and the walking state evaluation unit may estimate the walking state based on the estimated arm swing state of the user.

With the walking assist device according to the aspect described above, the walking state of the user can be evaluated adequately by estimating the arm swing state of the user.

In the walking assist device according to the aspect described above, the grasp portion state observation unit may observe the grasp portion state during a predetermined observation period (predetermined time, a predetermined number of times of arm swing); and the walking state evaluation unit may evaluate the walking state based on the grasp portion state observed during the predetermined observation period.

With the walking assist device according to the aspect described above, the walking state of the user can be evaluated more accurately by estimating the arm swing state of the user after a longer period of observation.

In the walking assist device according to the aspect described above, the walking state evaluation unit may evaluate the walking state based on the grasp portion state during a period excluding: a predetermined post-start period that is a predetermined period immediately after the user starts walking using the walking assist device; a predetermined pre-end period that is a predetermined period immediately before the user finishes walking using the walking assist device; and a predetermined turn period that is a predetermined period around a right turn or a left turn made by the user using the walking assist device.

With the walking assist device according to the aspect described above, the walking state can be evaluated more accurately since an unstable walking state that occurs immediately after the start of walk, immediately before the end of walk, etc. is excluded.

In the walking assist device according to the aspect described above, the grasp portion state may include a right stroke length that is a front-rear stroke length of the right grasp portion with respect to the frame and a left stroke length that is a front-rear stroke length of the left grasp portion with respect to the frame; the walking state evaluation unit may evaluate it being necessary to make a correction of at least one of the right stroke length and the left stroke length in the case where a deviation between the right stroke length and the left stroke length is equal to or more than a predetermined length deviation; and the correction adjustment unit may adjust the control command for the grasp portion drive unit such that the right stroke length and the left stroke length are equal to each other in the case where the walking state evaluation unit evaluates it being necessary to make the correction.

With the walking assist device according to the aspect described above, the range of right arm swing (right stroke length) and the range of left arm swing (left stroke length) during walk of the user can be corrected so as to be equal to each other, which approximates a more ideal walking state.

In the walking assist device according to the aspect described above, the grasp portion state may include a right stroke range that is a front-rear stroke range of the right grasp portion in position in the front-rear direction with respect to the frame, a left stroke range that is a front-rear stroke range of the left grasp portion in position in the front-rear direction with respect to the frame, a right stroke middle position that is a middle position, in the front-rear direction, of the right stroke range, and a left stroke middle position that is a middle position, in the front-rear direction, of the left stroke range; the walking state evaluation unit may evaluate it being necessary to make a correction of at least one of the right stroke middle position and the left stroke middle position in the case where a distance, in the front-rear direction, between the right stroke middle position and the left stroke middle position is equal to or more than a predetermined distance deviation; and the correction adjustment unit may adjust the control command for the grasp portion drive unit such that the right stroke middle position and the left stroke middle position are the same position in the front-rear direction in the case where the walking state evaluation unit evaluates it being necessary to make the correction.

With the walking assist device according to the aspect described above, the position (right stroke middle position), in the front-rear direction, of right arm swing of the user and the position (left stroke middle position), in the front-rear direction, of left arm swing of the user can be corrected so as to match each other, which makes a correction such that the user walks with his/her body directed forward with respect to the travel direction.

In the walking assist device according to the aspect described above, the walking state evaluation unit may extract, from a storage unit that stores reference stroke information including a reference stroke length that matches user personal information including gender, age, and body information of the user, the reference stroke information corresponding to the user; the walking state evaluation unit may calculate a target stroke length based on the reference stroke length included in the extracted reference stroke information; the walking state evaluation unit may evaluate, for the calculated target stroke length, whether or not it is necessary to make a correction of each of the right stroke length and the left stroke length; and in the case where the walking state evaluation unit evaluates it being necessary to make the correction, the correction adjustment unit may adjust the control command for the grasp portion drive unit such that the stroke length, for which the walking state evaluation unit evaluates it being necessary to make the correction, is brought to the target stroke length.

With the walking assist device according to the aspect described above, the range of right arm swing (right stroke length) and the range of left arm swing (left stroke length) can be corrected so as to be equal to the target stroke length that is based on the reference stroke length that matches the user, which approximates a more ideal walking state.

The walking assist device according to the aspect described above may further include a teaching information extraction unit that extracts teaching information including teachings about a correction of the walking state of the user, and a teaching information output unit that outputs at least one of a sound and an image based on the teaching information. In the walking assist device, the walking state evaluation unit may estimate a posture of the user during walk using the walking assist device based on the observed grasp portion state, and evaluate the walking state including the estimated posture of the user; the teaching information extraction unit may extract, from a storage unit that stores teaching information including teachings about a correction of the walking state of the user, the teaching information based on the walking state evaluated by the walking state evaluation unit; and the teaching information output unit may output at least one of a sound and an image based on the extracted teaching information.

With the walking assist device according to the aspect described above, the teaching information that is based on the walking state allows the user to easily understand the current walking state, and allows the user to correct the walking state by himself/herself.

The walking assist device according to the aspect described above may further include a determination data acquisition unit and a learning unit. In the walking assist device, the grasp portion state observation unit may observe, as a state variable, at least one of positions, in the front-rear direction, of the grasp portions with respect to the frame, inclination directions and inclination angles of the grasp portions with respect to the frame, and pressures applied to the grasp portions; the determination data acquisition unit may acquire determination data for determining a deviation between a target walking state, which is based on a reference walking state that is a walking state serving as a reference for the user, and an actual walking state of the user and fluctuations in the actual walking state of the user; and the learning unit may learn, in accordance with a training data set constituted of a combination of the state variable and the determination data, at least one of the control command adjusted by the correction adjustment unit and the teaching information extracted by the teaching information extraction unit.

With the walking assist device according to the aspect described above, the user can be corrected into a more adequate walking state by learning the control command and the teaching information.

In the walking assist device according to the aspect described above, the state variable may include at least one of: grasp portion position data that indicate positions of the grasp portions with respect to the frame, and that are detected by a grasp portion position detection unit; grasp portion inclination data that indicate inclination directions and inclination angles of the grasp portions with respect to the frame, and that are detected by a grasp portion inclination detection unit provided to the grasp portions; and grasp portion pressure data that indicate a pressure applied from the user to the grasp portions as the user grasps the grasp portions, and that are detected by a grasp portion pressure detection unit provided to the grasp portions.

With the walking assist device according to the aspect described above, the user can be corrected into a more adequate walking state by observing and learning a state in which the user grasps the grasp portions as a variety of states that need to be observed for learning.

In the walking assist device according to the aspect described above, the learning unit may learn the control command and the teaching information in accordance with the training data set; and the learning unit may have a reward calculation section that calculates a reward based on the determination data, and a function update section that updates a function for estimating an appropriate set of the control command and the teaching information for reducing at least one of the deviation and the fluctuations from a current state variable based on the reward.

With the walking assist device according to the aspect described above, the learning unit can learn the control command and the teaching information, calculate a reward based on the determination data, and update an action value function for estimating an appropriate set of the control command and the teaching information.

In the walking assist device according to the aspect described above, the learning unit may update an action value data map corresponding to the set of the control command and the teaching information based on the state variable and the reward.

With the walking assist device according to the aspect described above, the learning unit can correct the user into a more adequate walking state by updating and learning the action value data map corresponding to the set of the control command and the teaching information using the action value function.

In the walking assist device according to the aspect described above, the learning unit may further include an intention determination section that determines, based on a result of the learning unit performing learning in accordance with the training data set, a command for the set of the control command and the teaching information.

With the walking assist device according to the aspect described above, the learning unit can determine the control command and the teaching information using the intention determination section.

In the walking assist device according to the aspect described above, the learning unit may be connected to the control unit via a network.

With the walking assist device according to the aspect described above, the learning unit can be provided outside the walking assist device, and the user can be corrected into a more adequate walking state by performing learning based on more information that can be acquired from a plurality of walking assist devices.

### BRIEF DESCRIPTION OF THE DRAWINGS

The foregoing and further features and advantages of the invention will become apparent from the following description of example embodiments with reference to the accompanying drawings, wherein like numerals are used to represent like elements and wherein:
FIG. 1 is a perspective view illustrating the overall configuration of a walking assist device;
FIG. 2 is a perspective view illustrating the configuration and the function of a movable handle, a fixed handle, and a rail;
FIG. 3 is a sectional view of the movable handle as seen in the III-III direction in FIG. 2;
FIG. 4 is a sectional view of the movable handle as seen in the IV-IV direction in FIG. 2;
FIG. 5 is a perspective view of the fixed handle in FIG. 2 as enlarged;
FIG. 6 is a sectional view of the fixed handle as seen in the VI-VI direction in FIG. 5;
FIG. 7 is a block diagram illustrating inputs and outputs of a control unit of the walking assist device;
FIG. 8 illustrates operation modes of the walking assist device determined based on outputs of various detection units;
FIG. 9 illustrates conditions for transitioning from a determination mode to various operation modes in FIG. 8 and conditions for returning to the determination mode;
FIG. 10 is a flowchart illustrating the procedure of the overall process for the control unit of the walking assist device;
FIG. 11A and FIG. 11B are flowcharts illustrating processes in determined operation modes;
FIG. 12 is a flowchart illustrating the procedure of processes in an assist mode 3 and a training mode 3 in the control unit of the walking assist device;
FTG. 13A and FTG. 13B are flowcharts illustrating the procedure of processes in an assist mode 2 and a training mode 2 in the control unit of the walking assist device;
FIG. 14A and FIG. 14B are flowcharts illustrating the procedure of processes in a training mode 1 in the control unit of the walking assist device;
FIG. 15A and FIG. 15B are flowcharts illustrating the procedure of processes in an assist mode 1 in the control unit of the walking assist device;
FIG. 16 is a flowchart illustrating the procedure of a process for determination of the direction of a device turning force in the control unit of the walking assist device;
FIG. 17 is a flowchart illustrating the procedure of a process for determination of a turn in the control unit of the walking assist device;
FIG. 18A and FIG. 18B are flowcharts illustrating the procedure of a process for determination of the deviation between the travel speed and the walking speed of a user in the control unit of the walking assist device;
FIG. 19 illustrates mode transition conditions for transitioning among the operation modes based on a body state, an atmospheric state, and a vehicle body state;
FIG. 20 illustrates conditions for transitioning to the various operation modes in the case where the operation mode is automatically switched;
FIG. 21 is a flowchart illustrating the procedure of a process for evaluation of a walking state and a process for adjustment for correcting control commands in the control unit of the walking assist device;
FIG. 22A and FIG. 22B are flowcharts illustrating the procedure of a process for evaluation of stroke lengths in the control unit of the walking assist device;
FIG. 23 is a flowchart illustrating the procedure of a process for evaluation of stroke middle positions in the control unit of the walking assist device;

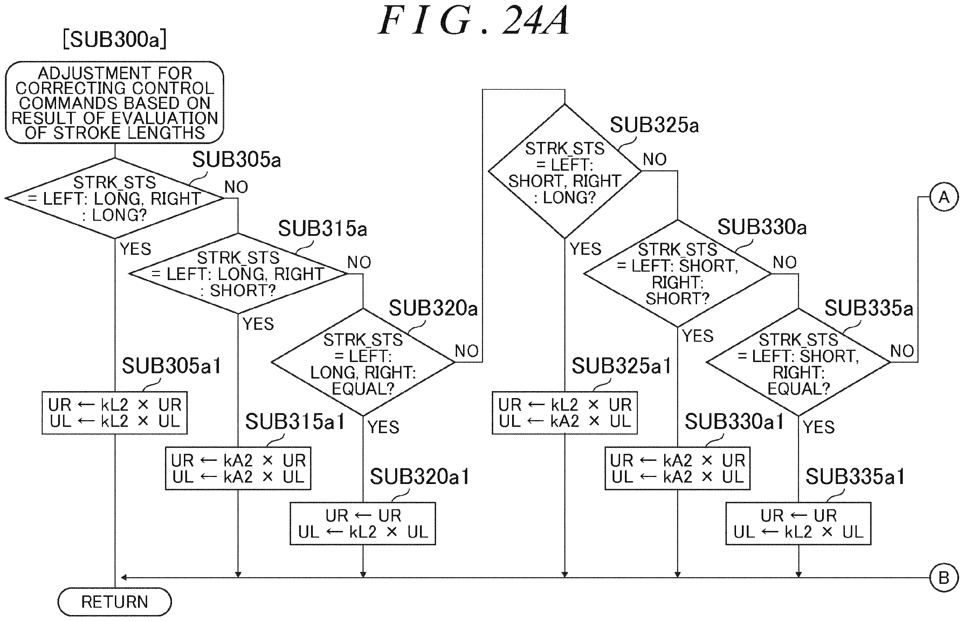

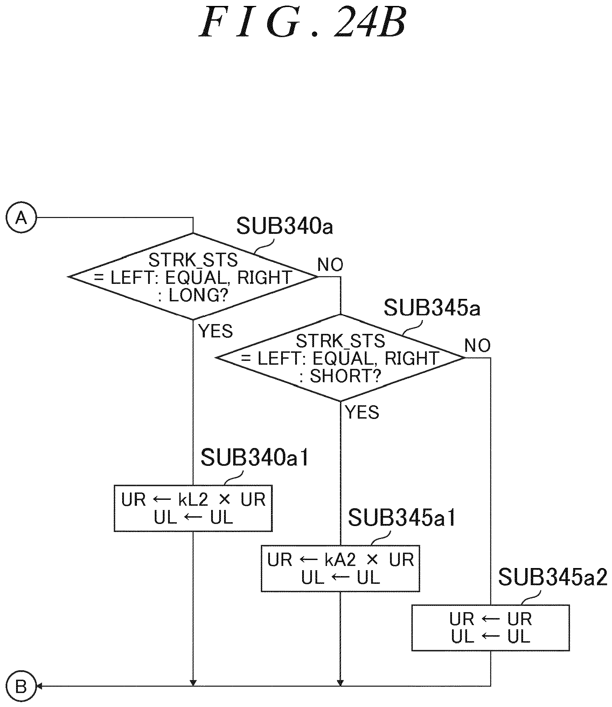

FIG. 24A and FIG. 24B are flowcharts illustrating the procedure of a process for adjustment for correcting control commands in accordance with the result of evaluation of stroke lengths in the control unit of the walking assist device;

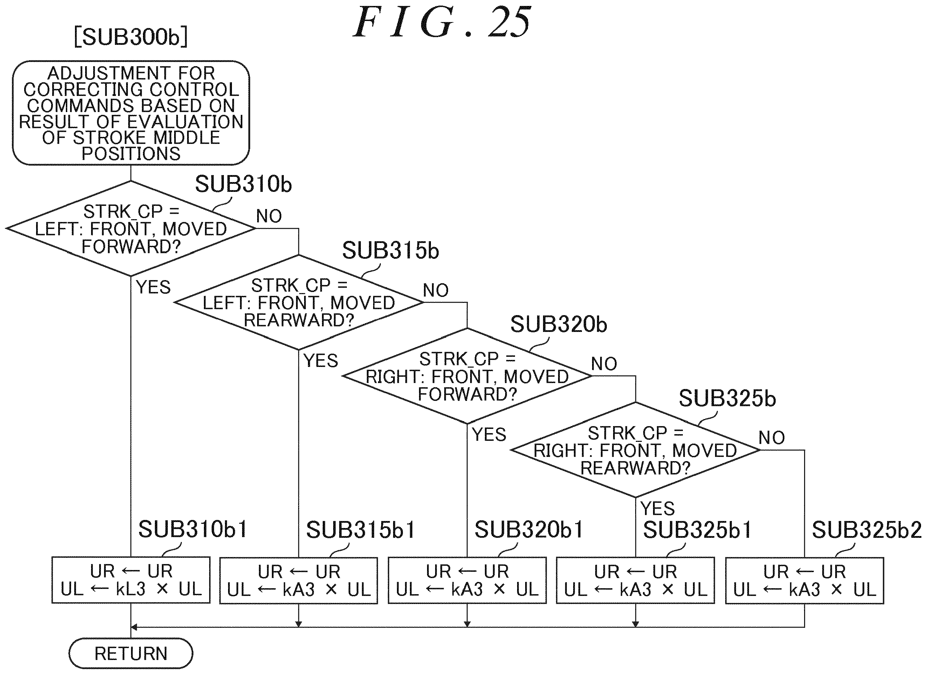

FIG. 25 is a flowchart illustrating the procedure of a process for adjustment for correcting control commands based on the result of evaluation of stroke middle positions in the control unit of the walking assist device;
FIG. 26 is a flowchart illustrating the procedure of a process for adjusting control commands and teaching information by a learning unit in the control unit of the walking assist device;
FIG. 27 illustrates a process for evaluation and correction of stroke lengths; and
FIG. 28 illustrates a process for evaluation and correction of stroke middle positions.

### DETAILED DESCRIPTION OF EMBODIMENTS

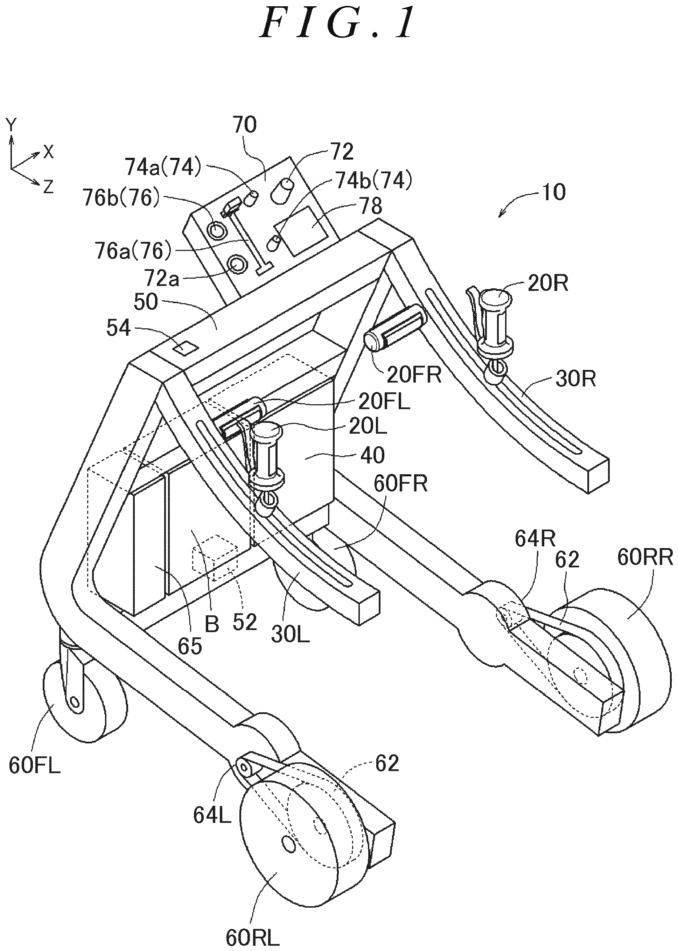

An embodiment of the present invention will be described below with reference to the drawings. The X axis, the Y axis, and the Z axis in the drawings are orthogonal to each other. In FIG. 1, the Z-axis direction indicates the direction from a front wheel 60FR to a rear wheel 60RR, and the X-axis direction indicates the direction from the left to the right in a frame 50. In the frame 50, the X-axis direction is referred to as "right", the direction opposite to the X-axis direction is referred to as "left", the direction opposite to the Z-axis direction is referred to as "front", and the Z-axis direction is referred to as "rear". In addition, the Y-axis direction is referred to as "upper", and the direction opposite to the Y-axis direction is referred to as "lower". The angular speed for rotation as seen in the X-axis direction is referred to as the pitch angular speed, the angular speed for rotation as seen in the Y-axis direction is referred to as the yaw angular speed, and the angular speed for rotation as seen in the Z-axis direction is referred to as the roll angular speed. The magnitude of the angular speed for clockwise rotation as seen in the direction of each of the X axis, the Y axis, and the Z axis is defined as "positive", and the magnitude of the angular speed for counterclockwise rotation as seen in the direction of each of the X axis, the Y axis, and the Z axis is defined as "negative".

A schematic configuration of the embodiment of the present invention will be described with reference to FIG. 1. FIG. 1 illustrates a walking assist device 10 according to the present embodiment. The walking assist device 10 has rails 30R and 30L (corresponding to the arm portion), a control unit 40, the frame 50, front wheels 60FR and 60FL, rear wheels 60RR and 60RL, drive units 64R and 64L (e.g. electric motors), a control panel 70, a battery B, and a regenerated power collecting unit 65.

As illustrated in FIG. 1, the frame 50 is shaped symmetrically in the right-left direction, and the rail 30R and the rail 30L are provided on the right side and the left side, respectively, of the frame 50 so as to extend along the front-rear direction of the frame 50. A user enters a space between the rail 30R and the rail 30L from the open side of the frame 50, and operates the walking assist device 10. The front wheels 60FR and 60FL are follower wheels (turnable caster wheels) provided at the lower front end of the frame 50.

The frame 50 is provided with an outside temperature sensor 54 that detects an outside temperature, and a three-axis acceleration and angular speed sensor 52 that detects inclination of the walking assist device 10 in each of the X-axis direction, the Y-axis direction, and the Z-axis direction. The rear wheels 60RR and 60RL are drive wheels provided at the lower rear end of the frame 50, and are driven by the drive units 64R and 64L, respectively, via belts 62. In the example illustrated in FIG. 1, a pair of right and left rear wheels are provided as the drive wheels, and are independently driven by the respective drive units. The rear wheels 60RR and 60RL can cause the walking assist device 10 to travel forward, travel rearward, make a right turn, and make a left turn.

The rail 30R has a movable handle 20R (corresponding to the grasp portion) and a fixed handle 20FR (corresponding to the grasp portion) that can be grasped by the user. The rail 30L has a movable handle 20L (corresponding to the grasp portion) and a fixed handle 20FL (corresponding to the grasp portion) that can be grasped by the user. The movable handle 20R is provided on the rail 30R, and is movable in the front-rear direction along the rail 30R in accordance with swing of an arm during walk of the user. The movable handle 20L is provided on the rail 30L, and is movable in the front-rear direction along the rail 30L in accordance with swing of an arm during walk of the user.

The rails 30R and 30L of the frame 50 are provided with the fixed handles 20FR and 20FL, respectively. The rails 30R and 30L are not limited to being shaped to be concavely curved upward, and may have a straight shape.

As illustrated in FTG. 1, the control panel 70 is provided at a position at which the control panel 70 is easily operable by the user at the upper portion of the frame 50, for example. The control panel 70 has a main switch 72, an assist amount adjustment volume 74a, a load amount adjustment volume 74b, a manual mode switching unit 76a, an automatic mode switching unit switch 76b, and a monitor 78 (corresponding to the teaching information output unit).

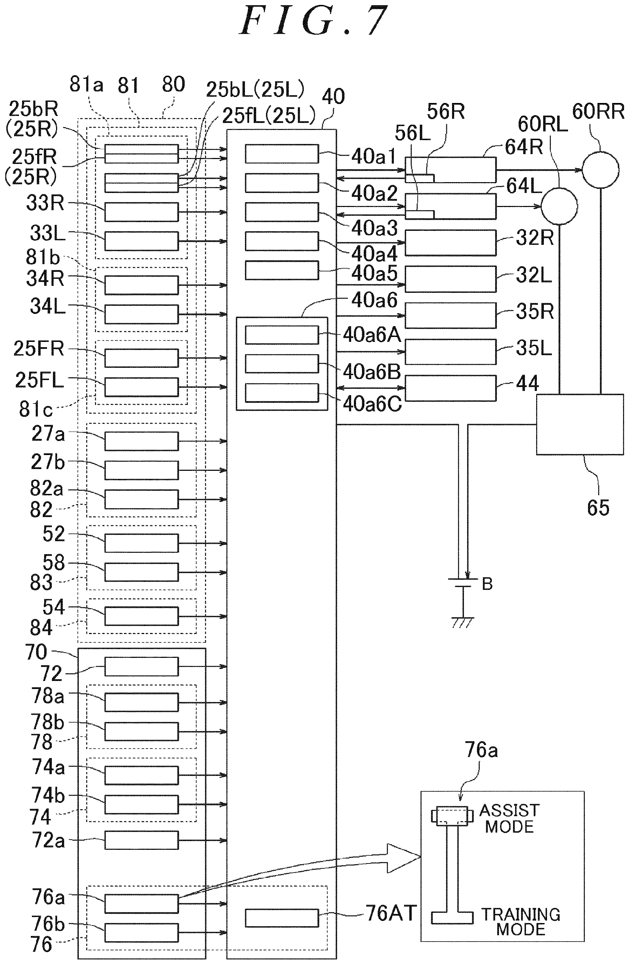

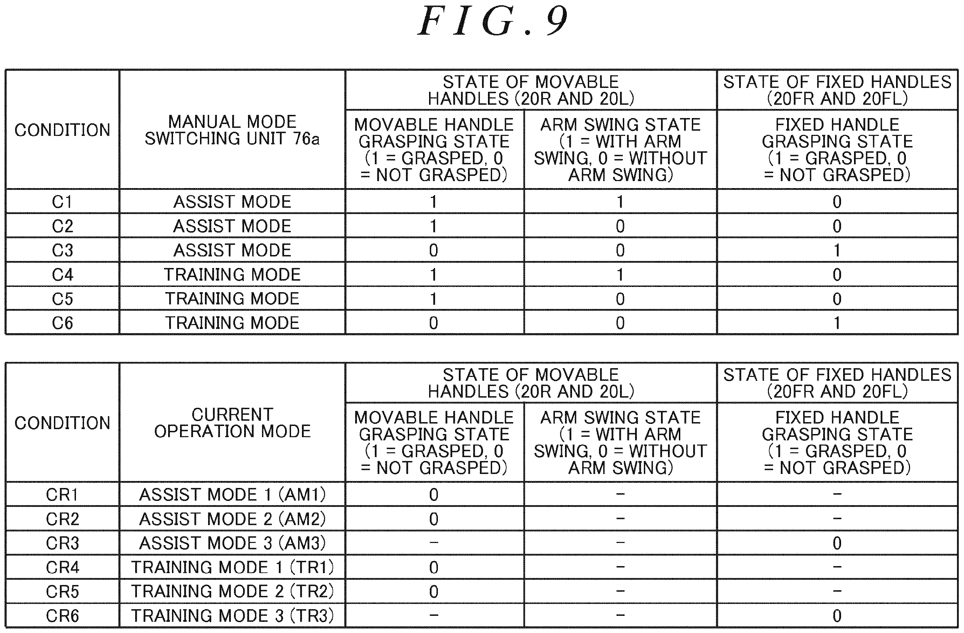

The walking assist device 10 has, as operation modes, a training mode, in which a load is applied to operation of the body of the user performed as the user walks, and an assist mode, in which the load on operation of the body of the user performed as the user walks is alleviated. The operation mode switching unit 76 has the manual mode switching unit 76a, the automatic mode switching unit switch 76b, and an automatic mode switching unit 76AT (see FIG. 7). The manual mode switching unit 76a switches the operation mode of the walking assist device 10 through a manual operation by the user. The manual mode switching unit 76a allows selection of one of two operation modes including an "assist mode" and a "training mode" (see FIG. 9).

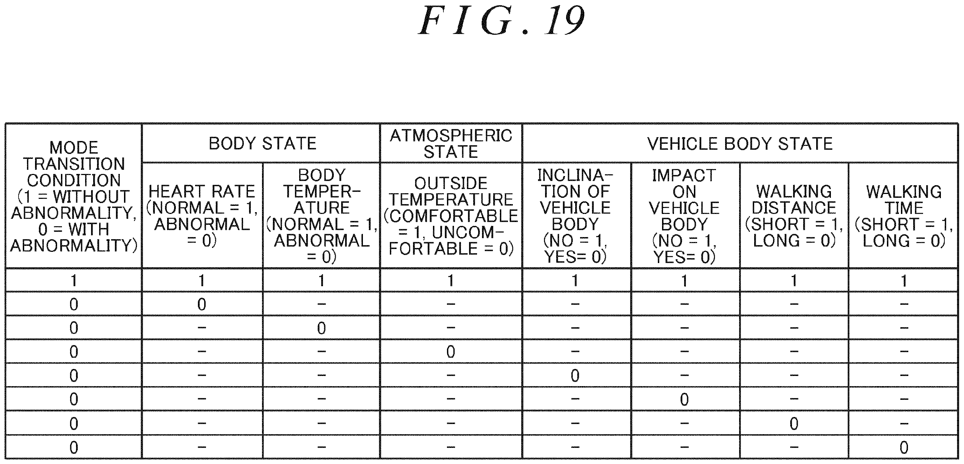

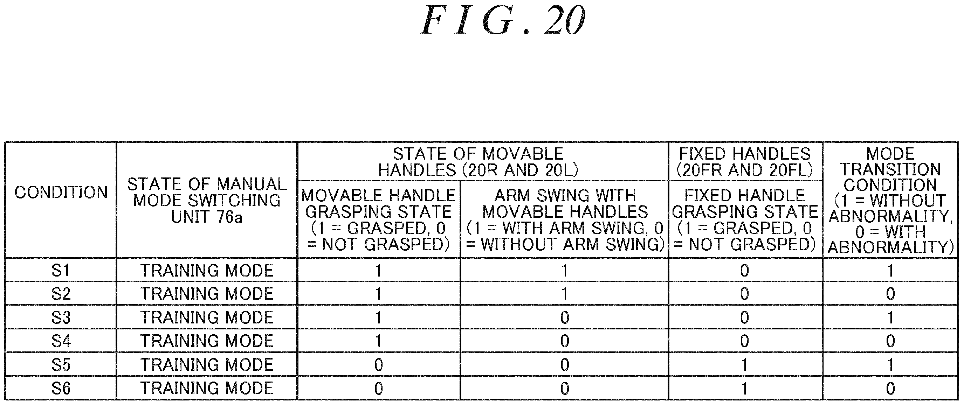

The automatic mode switching unit switch 76b is a switch that permits the control unit 40 to automatically switch the operation mode. In the case where the automatic mode switching unit switch 76b is on, the automatic mode switching unit 76AT of the control unit 40 automatically switches the operation mode based on information selected through the manual mode switching unit 76a and conditions in FIGS. 19 and 20.

The assist amount adjustment volume 74a is used to adjust the magnitude (assist amount) of an assist force in the assist mode. The load amount adjustment volume 74b is used to adjust the magnitude (load amount) of a load in the training mode.

The monitor 78 has a sound output unit 78a and an image output unit 78b. The monitor 78 communicates, to the user, operation mode information and, besides, the charge amount of the battery B, a walking history, information on the body state of the user, a body information history of the user, a surrounding atmospheric state, a load amount and assist amount, an operation history of the walking assist device 10, a vehicle body state, for example, using at least one of the sound output unit 78a and the image output unit 78b. The monitor 78 also communicates teachings to the user by outputting at least one of a sound from the sound output unit 78a and an image from the image output unit 78b.

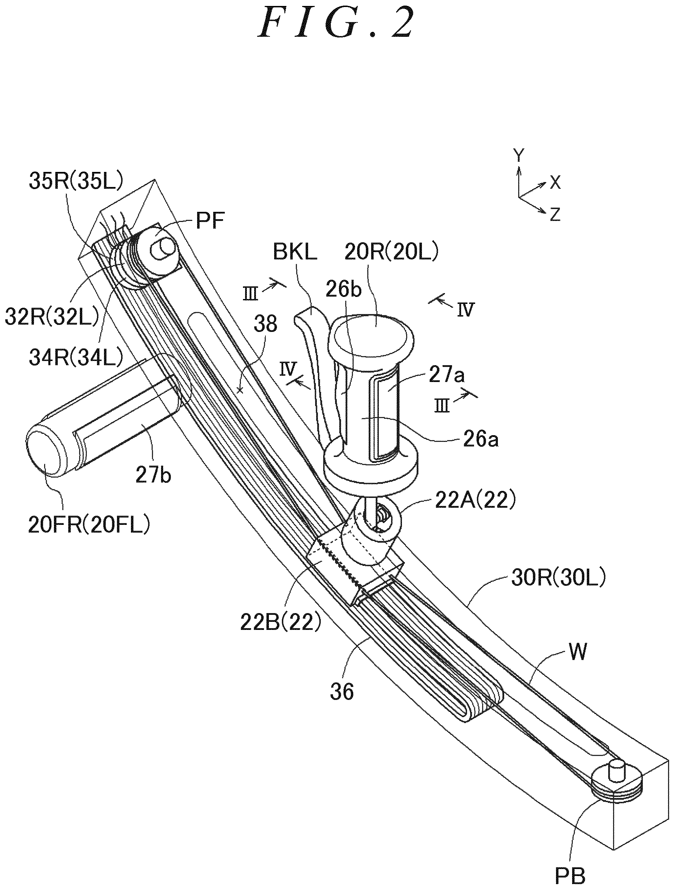

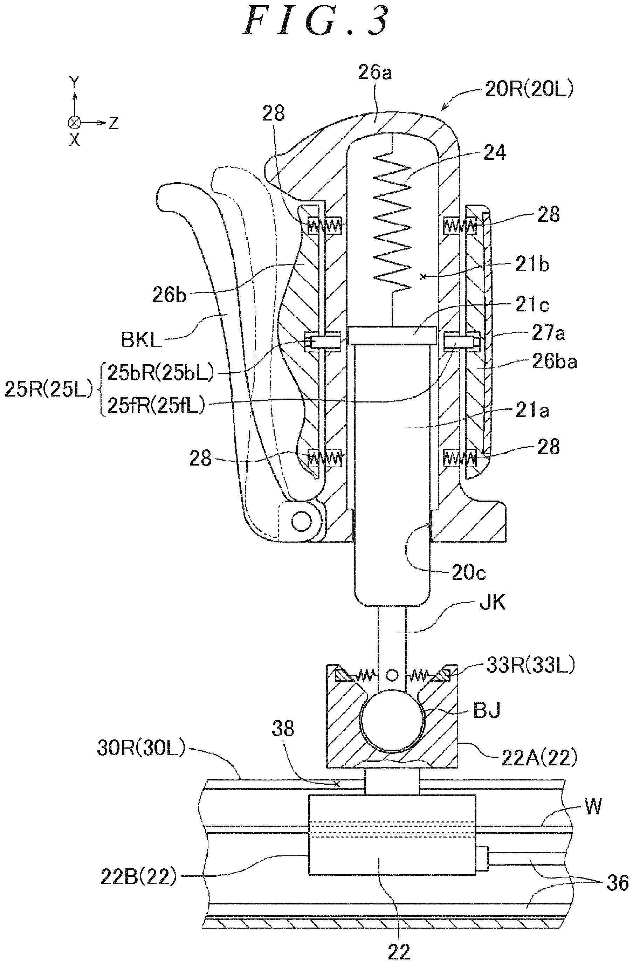

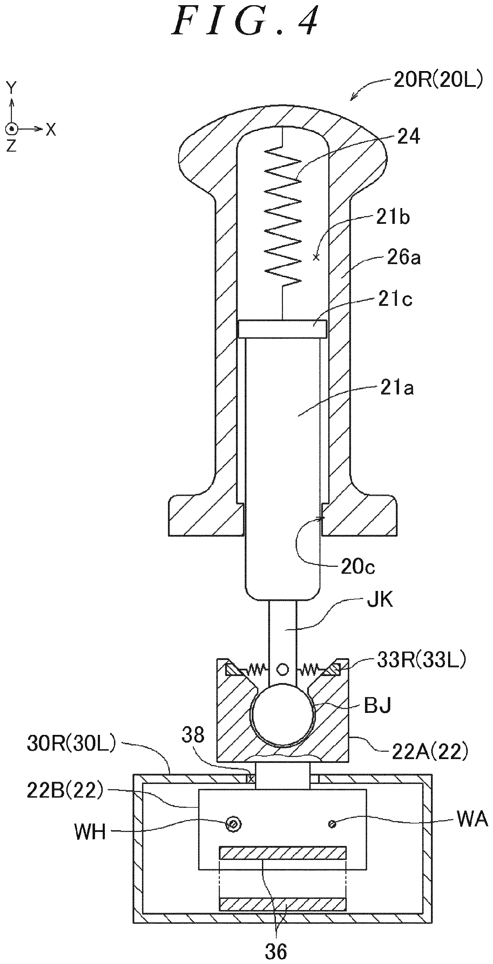

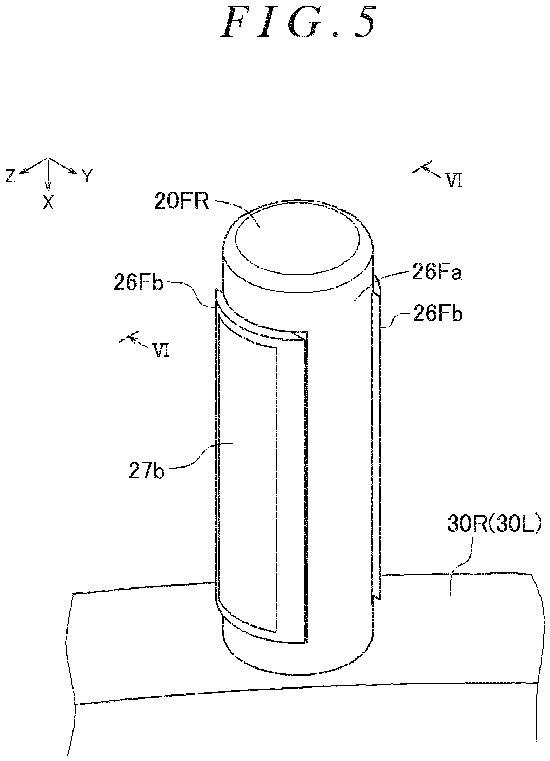

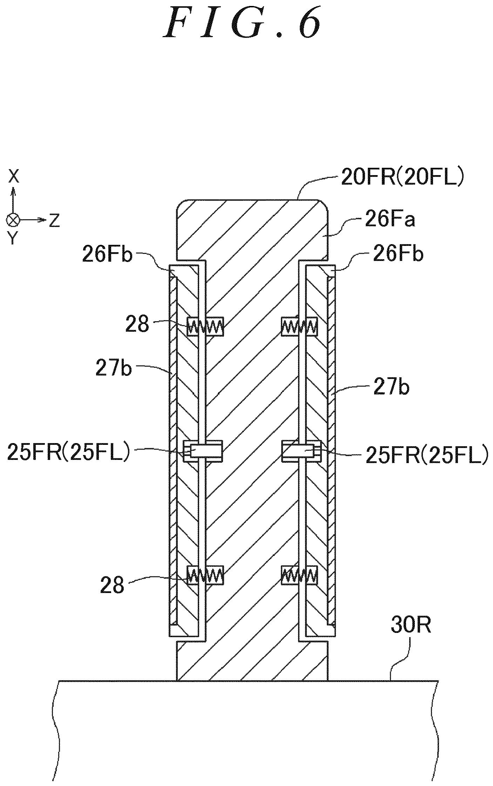

The structure of the walking assist device 10 will be described in detail with reference to FIGS. 2 to 6. The walking assist device 10 has a symmetrical structure between the right and the left of the frame 50 except for the control panel 70, the control unit 40, the battery B, and the regenerated power collecting unit 65. Therefore, the structure on the right side will be mainly described, while omitting description on the structure on the left side. FIG. 2 is a perspective view illustrating the configuration and the function of the movable handle 20R, the fixed handle 20FR, and the rail 30R. FIG. 3 is a sectional view of the movable handle 20R as seen in the III-III direction in FIG. 2. FIG. 4 is a sectional view of the movable handle 20R as seen in the IV-IV direction in FIG. 2. FIG. 5 is a perspective view of the fixed handle 20FR in FIG. 2 as enlarged. FIG. 6 is a sectional view of the fixed handle 20FR as seen in the VI-VI direction in FIG. 5.

As illustrated in FIG. 2, the rail 30R has the movable handle 20R, pulleys PB and PF, and a wire W. The rail 30R is shaped to be concavely curved upward, and has a rail slit portion 38 that opens upward, extends along the front-rear direction, and defines the movable range of the movable handle 20R. The rail 30R is provided with the pulleys PB and PF at respective ends in the front-rear direction. The wire W is wound around the pulley PF, which is provided on the front side, and the pulley PB, which is provided on the rear side, so that the pulleys PF and PB are rotated in conjunction with each other. A grasp portion drive unit 32R (e.g. an electric motor), a grasp portion position detection unit 34R (e.g. an encoder), and a handle movement limiting unit 35R are provided coaxially with the pulley PF. As illustrated in FIG. 4, the wire is fixed to a wire connection portion WA of an anchor portion 22B, and the wire is inserted through a wire hole WH without being fixed. The movable handle 20R is connected to the anchor portion 22B. Consequently, the grasp portion drive unit 32R can assist movement of the movable handle 20R, or apply a load to movement of the movable handle 20R, by rotating the pulley PF to rotate the wire W between the pulleys PB and PF. The grasp portion position detection unit 34R outputs the amount of rotation of the pulley PF that accompanies movement of the movable handle 20R on the rail 30R to the control unit 40.

As illustrated in FIG. 3, the movable handle 20R has a handle shaft portion 21a, a shaft portion fitting hole 21b, a slider 22, a grip portion 26a, a switch grip portion 26b, and a brake lever BKL. The slider 22 is composed of a handle holding portion 22A and an anchor portion 22B.

As illustrated in FIG. 3, one end of an urging unit 24 is connected to the handle shaft portion 21a, and the other end thereof is connected to the bottom portion of the shaft portion fitting hole 21b. A flange portion 21c that extends in the circumferential direction is provided at the end portion of the handle shaft portion 21a to which the urging unit 24 is connected. An inner flange portion 20c is provided on an inside wall surface at an opening of the shaft portion fitting hole 21b. Consequently, the grip portion 26a is slidable up and down along the longitudinal direction of the handle shaft portion 21a without separating from the handle shaft portion 21a. That is, the movable handle 20R has an expansion and contraction mechanism that enables expansion and contraction in the projecting direction.

A handle support shaft JK is provided on the side of the handle shaft portion 21a to which the urging unit 24 is not connected. The distal end of the handle support shaft JK is formed in a generally spherical shape, and forms a ball joint together with a recess provided in the handle holding portion 22A. Consequently, the movable handle 20R can be tilted to the front, rear, right, and left within a range defined by an opening with respect to the handle holding portion 22A (see FIGS. 3 and 4). A grasp portion inclination detection unit 33R that detects the amount of this tilt is provided at the opening of the handle holding portion 22A, and disposed on the front, rear, right, and left with respect to the handle support shaft JK. The grasp portion inclination detection unit 33R may be a pressure sensor that detects a pressure in accordance with expansion and contraction of springs provided between the side surfaces of the handle support shaft JK and the opening of the handle holding portion 22A, for example.

As illustrated in FIG. 3, the switch grip portion 26b is provided such that a predetermined gap is formed between the grip portion 26a and the switch grip portion 26b by grip urging units 28 (e.g. springs).

A grasp portion pressure detection unit 25R has a grasp portion front pressure detection unit 25fR and a grasp portion rear pressure detection unit 25bR. The grasp portion pressure detection unit 25R measures a pressure input to the right movable handle 20R. A grasp portion pressure detection unit 25L has a grasp portion front pressure detection unit 25fL and a grasp portion rear pressure detection unit 25bL. The grasp portion pressure detection unit 25L measures a pressure input to the left movable handle 20L.

The grasp portion front pressure detection units 25fR and 25fL and the grasp portion rear pressure detection units 25bR and 25bL are each a pressure sensor that detects a grasp portion pressure that is a pressure input to the movable handles 20R and 20L, respectively. The grasp portion front pressure detection units 25fR and 25fL and the grasp portion rear pressure detection units 25bR and 25bL may each be a load sensor that detects a load.

The grasp portion front pressure detection unit 25fR detects a grasp portion front pressure that is a pressure directed forward and input to the corresponding right movable handle 20R. The grasp portion rear pressure detection unit 25bR detects a grasp portion rear pressure that is a pressure directed rearward and input to the corresponding right movable handle 20R. The grasp portion front pressure detection unit 25fL detects a grasp portion front pressure that is a pressure directed forward and input to the corresponding left movable handle 20L. The grasp portion rear pressure detection unit 25bL detects a grasp portion rear pressure that is a pressure directed rearward and input to the corresponding left movable handle 20L.

The grasp portion front pressure detection unit 25fR outputs a signal that matches an applied pressure (see FIG. 3) with a switch grip portion 26ba moved toward the grip portion 26a when the user grasps the right movable handle 20R. The grasp portion front pressure detection unit 25fR is turned off when a pressure is not applied. The grasp portion front pressure detection unit 25fL outputs a signal that matches an applied pressure (see FIG. 3) with a switch grip portion 26ba moved toward the grip portion 26a when the user grasps the left movable handle 20L. The grasp portion front pressure detection unit 25fL is turned off when a pressure is not applied.

The grasp portion rear pressure detection unit 25bR outputs a signal that matches an applied pressure (see FIG. 3) with the switch grip portion 26b moved toward the grip portion 26a when the user grasps the right movable handle 20R. The grasp portion rear pressure detection unit 25bR is turned off when a pressure is not applied. The grasp portion rear pressure detection unit 25bL outputs a signal that matches an applied pressure (see FIG. 3) with the switch grip portion 26b moved toward the grip portion 26a when the user grasps the left movable handle 20L. The grasp portion rear pressure detection unit 25bL is turned off when a pressure is not applied.

As illustrated in FIG. 3, a heart rate and body temperature sensor 27a is provided at a part of the grip portion 26a. The heart rate and body temperature sensor 27a measures the heart rate and the body temperature of the user in predetermined cycles in the case where the user grasps the movable handle 20R (20L). The heart rate of the user may be measured by measuring the blood flow at a portion grasped by his/her hand using infrared radiation, for example. The body temperature of the user may be measured by measuring variations in the resistance of a thermistor that is varied in accordance with temperature variations, or variations in infrared radiation emitted by the portion that is grasped by the user, for example.

One end of the brake lever BKL is connected to the lower front side of the grip portion 26a. A mechanism that locks rotation of the front wheels 60FR and 60FL and the rear wheels 60RR and 60RL when the brake lever BKL is grasped and pulled toward the grip portion 26a by the user, that maintains the locked state, and unlocks such rotation when the brake lever BKL is further pulled is provided (not illustrated).

As illustrated in FIG. 2, the rail 30R is provided with the handle movement limiting unit 35R that permits and prohibits movement of the movable handle 20R with respect to the frame 50. The handle movement limiting unit 35R has a lock mechanism that locks rotation of the grasp portion drive unit 32R, for example. The handle movement limiting unit 35R prohibits movement of the handle by locking rotation of the grasp portion drive unit 32R, and permits movement of the handle with respect to the rail (i.e. with respect to the frame) by unlocking rotation of the grasp portion drive unit 32R.

As illustrated in FIGS. 2 and 4, one end of the wire W is inserted through the wire hole WH that is provided in the anchor portion 22B, and the other end of the wire W is connected (fixed) to the wire connection portion WA. The movable handle 20R is movable on the rail 30R with a constricted portion that connects between the handle holding portion 22A and the anchor portion 22B sliding in the rail slit portion 38.

A signal cable 36 transfers detection signals from the grasp portion pressure detection unit 25R and the grasp portion inclination detection unit 33R to the control unit 40 with one end of the signal cable 36 connected to the anchor portion 22B and with the other end thereof connected to the control unit 40. The signal cable 36 may be a cable that is flexible such as a flexible cable, for example. The control unit 40 can detect the position of the movable handle 20R on the rail 30R based on a detection signal from the grasp portion position detection unit 34R.

As illustrated in FIG. 5, the fixed handle 20FR (20FL) has a grip portion 26Fa and a switch grip portion 26Fb. A heart rate and body temperature sensor 27b measures the heart rate and the body temperature of the user in predetermined cycles in the case where the user grasps the fixed handle 20FR (20FL). Measurement of the heart rate and the body temperature of the user by the heart rate and body temperature sensor 27b is the same as that by the heart rate and body temperature sensor 27a, and therefore is not described.

As illustrated in FIG. 6, the switch grip portion 26Fb is provided such that a predetermined gap is formed between the grip portion 26Fa and the switch grip portion 26Fb by grip urging units 28 (e.g. springs). A grasp portion pressure detection unit 25FR outputs a detection signal that is proportional to a pressure with the switch grip portion 26Fb moved toward the grip portion 26Fa when the user grasps the fixed handle 20FR, and is turned off when a pressure is not applied. The grasp portion pressure detection unit 25FR may be any component that outputs a detection signal that is proportional to an applied pressure such as a pressure sensor, for example.

The function of the walking assist device 10 and the processes in the various operation modes will be described in detail with reference to FIGS. 7 to 18.

FIG. 7 is a block diagram illustrating inputs and outputs of the control unit 40 (e.g. a control device that includes a CPU) of the walking assist device 10 (see FIG. 1). As illustrated in FIG. 7, the control unit 40 receives, as inputs, information from a state detection unit 80, information stored in a storage unit 44, and information from the control panel 70. The control unit 40 controls the grasp portion drive units 32R and 32L (electric motors), the handle movement limiting units 35R and 35L, the drive units 64R and 64L, and the monitor 78 based on the input information.

The control unit 40 has a grasp portion state observation unit 40a1, a walking state evaluation unit 40a2, a correction adjustment unit 40a3, a teaching information extraction unit 40a4, a determination data acquisition unit 40a5, and a learning unit 40a6.

As illustrated in FIG. 7, the state detection unit 80 is composed of a grasp portion state detection unit 81, a body state detection unit 82, a vehicle body state detection unit 83, and an atmospheric state detection unit 84.

The grasp portion state detection unit 81 is composed of a movable handle acting force detection unit 81a, a movable handle movement amount detection unit 81b, and a fixed handle acting force detection unit 81c.

The movable handle acting force detection unit 81a has the grasp portion pressure detection units 25R and 25L, the grasp portion inclination detection unit 33R, and a grasp portion inclination detection unit 33L.

The grasp portion pressure detection unit 25R has the grasp portion front pressure detection unit 25fR and the grasp portion rear pressure detection unit 25bR (see FIG. 3). The grasp portion front pressure detection unit 25fR detects a grasping force applied to the front side of the movable handle 20R that is grasped by the user as a pressure (right grasp portion front pressure), and outputs a detection signal that matches the right grasp portion front pressure to the control unit 40. The grasp portion rear pressure detection unit 25bR detects a grasping force applied to the rear side of the movable handle 20R that is grasped by the user as a pressure (right grasp portion rear pressure), and outputs a detection signal that matches the right grasp portion rear pressure to the control unit 40.

The grasp portion pressure detection unit 25L has the grasp portion front pressure detection unit 25fL and the grasp portion rear pressure detection unit 25bL (see FIG. 3). The grasp portion front pressure detection unit 25fL detects a grasping force applied to the front side of the movable handle 20L that is grasped by the user as a pressure (left grasp portion front pressure), and outputs a detection signal that matches the left grasp portion front pressure to the control unit 40. The grasp portion rear pressure detection unit 25bL detects a grasping force applied to the rear side of the movable handle 20L that is grasped by the user as a pressure (left grasp portion rear pressure), and outputs a detection signal that matches the left grasp portion rear pressure to the control unit 40.

The grasp portion front pressure detection units 25fR and 25fL output a detection signal that matches a movable handle acting force (a force obtained by subtracting the grasp portion rear pressure from the grasp portion front pressure) to the control unit 40.

The grasp portion pressure detection unit 25R outputs "1 = grasped" to the control unit 40 as a detection signal in the case where it is detected that the user is grasping the movable handle 20R, and outputs "0 = not grasped" in the case where it is detected that the user is not grasping the movable handle 20R. The grasp portion pressure detection unit 25L outputs "1 = grasped" to the control unit 40 as a detection signal in the case where it is detected that the user is grasping the movable handle 20L, and outputs "0 = not grasped" in the case where it is detected that the user is not grasping the movable handle 20L.

The grasp portion inclination detection unit 33R detects how much (inclination angle) the movable handle 20R is tilted to which (inclination direction) of the front, rear, right, and left with respect to the rail 30R, and outputs the detected angle and direction to the control unit 40. The grasp portion inclination detection unit 33L detects how much (inclination angle) the movable handle 20L is tilted to which (inclination direction) of the front, rear, right, and left with respect to the rail 30L, and outputs the detected angle and direction to the control unit 40. The inclination direction is defined as "front" in the case where the movable handle (20R, 20L) is tilted to the front with respect to the frame 50, "rear" in the case where the movable handle (20R, 20L) is tilted to the rear, "right" in the case where the movable handle (20R, 20L) is tilted to the right, and "left" in the case where the movable handle (20R, 20L) is tilted to the left. The inclination direction is defined as "neutral" in the case where the movable handle (20R, 20L) is not tilted with respect to the frame 50. The inclination angle is detected as an angle with respect to the frame 50, for example.

The movable handle movement amount detection unit 81b has the grasp portion position detection unit 34R and a grasp portion position detection unit 34L.

The grasp portion position detection unit 34R detects a right grasp portion position HPR that is the position of the movable handle 20R on the rail 30R, and outputs a detection signal that matches the position to the control unit 40. The grasp portion position detection unit 34L detects a left grasp portion position HPL that is the position of the movable handle 20L on the rail 30L, and outputs a detection signal that matches the position to the control unit 40.

The movable handle movement amount detection unit 81b detects the amount of movement, in a predetermined time, of the movable handles 20R and 20L with respect to the rails 30R and 30L (see FIG. 1) made as the user walks while grasping the movable handles 20R and 20L and swinging his/her arms, and outputs a signal that matches the detected amount to the control unit 40.

The fixed handle acting force detection unit 81c has grasp portion pressure detection units 25FR and 25FL.

The grasp portion pressure detection unit 25FR outputs "1 = grasped" to the control unit 40 as a detection signal in the case where it is detected that the user is grasping the fixed handle 20FR, and outputs "0 = not grasped" in the case where it is detected that the user is not grasping the fixed handle 20FR. The grasp portion pressure detection unit 25FL outputs "1 = grasped" to the control unit 40 as a detection signal in the case where it is detected that the user is grasping the fixed handle 20FL, and outputs "0 = not grasped" in the case where it is detected that the user is not grasping the fixed handle 20FL.

The grasp portion pressure detection unit 25FR detects a fixed handle acting force that is a force to push forward and pull rearward the fixed handle 20FR (see FIG. 1) grasped by the user, and outputs a signal that matches a detected state to the control unit 40. The grasp portion pressure detection unit 25FL detects a fixed handle acting force that is a force to push forward and pull rearward the fixed handle 20FL (see FIG. 1) grasped by the user, and outputs a signal that matches a detected state to the control unit 40.

The body state detection unit 82 is a unit that detects the body state of the user, and has heart rate and body temperature sensors 27a and 27b and a body information history 82a. The body state detection unit 82 detects the body state of the user, e.g. the heart rate and the body temperature of the user, through the heart rate and body temperature sensors 27a and 27b, and outputs a signal that matches the detected state to the control unit 40.

The body state detection unit 82 stores a history of body information (e.g. the heart rate, the body temperature, and the number of footsteps) on the user in the body information history 82a. The number of footsteps is calculated based on information from the movable handle movement amount detection unit 81b by determining that the user makes two steps when he/she swings his/her arms back and forth once in the front-rear direction, for example.

The vehicle body state detection unit 83 is a unit that detects the state of the walking assist device 10 including an operation history of the walking assist device 10, and has a travel speed acquisition unit 56R, a travel speed acquisition unit 56L, the three-axis acceleration and angular speed sensor 52, and operation history information 58.

The travel speed acquisition unit 56R and the travel speed acquisition unit 56L are connected to the drive units 64R and 64L, respectively, and output a detection signal corresponding to travel speeds (VdR and VdL) at which the rear wheels 60RR and 60RL (see FIG. 1) travel forward and rearward, respectively, to the control unit 40.

The three-axis acceleration and angular speed sensor 52 measures an acceleration for each of the axes in the three directions, namely the X axis, the Y axis, and the Z axis, and measures an angular speed of rotation about each of the axes in the three directions. In the case where the walking assist device 10 is traveling on an inclined surface, for example, the three-axis acceleration and angular speed sensor 52 outputs a detection signal that matches the tilt of the vehicle with respect to the inclined surface for each of the X axis, the Y axis, and the Z axis to the control unit 40. The three-axis acceleration and angular speed sensor 52 also detects variations in the acceleration applied to the vehicle body of the walking assist device 10 (impact on the vehicle body), and outputs a signal that matches the detected variations in the acceleration to the control unit 40. The three-axis acceleration and angular speed sensor 52 also detects the pitch angular speed, the yaw angular speed, and the roll angular speed of the vehicle body of the walking assist device 10, and outputs a signal that matches the detected angular speeds to the control unit 40.

The three-axis acceleration and angular speed sensor 52 also functions as a turning force measurement unit that detects a device turning force (yaw angular speed) that is a force that turns the walking assist device 10, and outputs a signal that matches the detected yaw angular speed to the control unit 40.

The vehicle body state detection unit 83 stores an operation history (e.g. the walking distance and the walking time) of the walking assist device 10 in the operation history information 58, and detects the state of the walking assist device 10 (e.g. the travel speed of the walking assist device, the tilt of the vehicle body, and the travel speed).

The atmospheric state detection unit 84 is a unit that detects the atmospheric state (e.g. the outside temperature) around the user, and has the outside temperature sensor 54. The atmospheric state detection unit 84 detects the outside temperature through the outside temperature sensor 54, and outputs a signal that matches the detected state to the control unit 40.

The control unit 40 calculates forward-direction evaluation speeds (VRhf and VLhf), which are speeds of movement in the forward direction of the movable handles 20R and 20L with respect to the frame 50, and rearward-direction evaluation speeds (VRhb and VLhb), which are speeds of movement in the rearward direction of the movable handles 20R and 20L with respect to the frame 50, based on the amounts of movement of the movable handles 20R and 20L (see FIGS. 1 and 2). The magnitude of the speeds of movement of the movable handles 20R and 20L with respect to the frame 50 is defined as "positive" in the case of movement in the forward direction, and defined as "negative" in the case of movement in the rearward direction.

The forward-direction evaluation speeds (VRhf and VLhf), or the rearward-direction evaluation speeds (VRhb and VLhb), are calculated through integration from the speeds of movement of the movable handles (20R and 20L) for a case where the user swings his arm forward, or rearward, for example. Specifically, the evaluation speed is derived in accordance with the following procedure. The processes are the same for the right and left movable handles, and therefore only the forward-direction evaluation speed (VRhf) and the rearward-direction evaluation speed (VRhb) of the right movable handle 20R will be described.

Derivation of the forward-direction evaluation speed (VRhf) of the right movable handle 20R: The control unit 40 calculates the speed of movement of the movable handle 20R based on the amount of movement of the movable handle 20R measured at predetermined intervals. The control unit 40 integrates (integration process) only the speeds of forward movement (speeds of movement having a "positive" magnitude) at which the movable handle 20R moves forward, among the calculated speeds (right grasp portion movement speeds) of movement of the movable handle 20R. The control unit 40 derives the forward-direction evaluation speed (VRhf) by dividing the speed of forward movement of the movable handle 20R, which is obtained through integration, by a predetermined time (averaging process).

Derivation of the rearward-direction evaluation speed (VRhb) of the right movable handle 20R: The control unit 40 calculates the speed of movement of the movable handle 20R based on the amount of movement of the movable handle 20R measured at predetermined intervals. The control unit 40 integrates (integration process) only the speeds of rearward movement (speeds of movement having a "negative" magnitude) at which the movable handle 20R moves rearward, among the calculated speeds (right grasp portion movement speeds) of movement of the movable handle 20R. The control unit 40 derives the rearward-direction evaluation speed (VRhb) by dividing the speed of rearward movement of the movable handle 20R, which is obtained through integration, by a predetermined time (averaging process).

The control unit 40 drives the rear wheels 60RR and 60RL, which are drive wheels, by controlling the drive units 64R and 64L so as to achieve target travel speeds (VR and VL) that are targets for travel of the walking assist device 10. The target travel speed VR is a target travel speed at which the rear wheel 60RR of the walking assist device 10 is caused to travel based on operation by the user, and the target travel speed VL is a target travel speed at which the rear wheel 60RL of the walking assist device 10 is caused to travel based on operation by the user (see FIG. 1).

The control unit 40 drives the movable handles 20R and 20L (see FIG. 2) by controlling the grasp portion drive units 32R and 32L so as to achieve target movement speeds (UR and UL). The target movement speed UR is calculated based on the speed at which the user moves the movable handle 20R on the rail 30R (see FIG. 2). The target movement speed UL is calculated based on the speed at which the user moves the movable handle 20L on the rail 30L (see FIG. 2). The target movement speeds (UR and UL) are each set to a speed that serves as a load on the grasp portion movement speed (right grasp portion movement speed, left grasp portion movement speed) in the case where a load is to be imposed on the movable handle, and set to a speed that assists the grasp portion movement speed in the case where the movable handle is to be assisted.

A load amount and assist amount change unit 74 has the assist amount adjustment volume 74a and the load amount adjustment volume 74b. The assist amount adjustment volume 74a outputs a detection signal that matches the adjustment amount (assist adjustment amount) for adjusting the magnitude (assist amount) of an assist force in the assist mode to the control unit 40. The load amount adjustment volume 74b outputs a detection signal that matches the adjustment amount (load adjustment amount) for adjusting the magnitude (load amount) of a load in the training mode to the control unit 40. In the assist mode, the load amount and assist amount change unit 74 changes the assist amount based on information from the state detection unit 80 and the assist adjustment amount. In the training mode, the load amount and assist amount change unit 74 changes the load amount based on information from the state detection unit 80 and the load adjustment amount.

The storage unit 44 is a unit that stores information, and stores and reads information in response to a request from the control unit 40. The storage unit 44 stores information such as information acquired by the state detection unit 80, the result of computation performed by the control unit 40, the operation history of the walking assist device 10, the assist amount in the assist mode in the past during walk of the user, and the load amount in the training mode. The storage unit 44 stores reference stroke information including a reference stroke length that matches user personal information including gender, age, and body information of the user. The storage unit 44 also stores teaching information including teachings about a correction of the walking state of the user.

The control panel 70 provides switches and the monitor 78 that are necessary for the user to operate the walking assist device 10. The user makes the walking assist device 10 ready for travel by turning on the main switch 72. The user can adjust the load amount in the training mode and the assist amount in the assist mode using the assist amount adjustment volume 74a and the load amount adjustment volume 74b. The user can select a desired operation mode ("assist mode" and "training mode") by operating the manual mode switching unit 76a. In the case where the automatic mode switching unit switch 76b is turned on, the control unit 40 automatically switches the operation mode between the operation mode selected by the user and a predetermined operation mode.

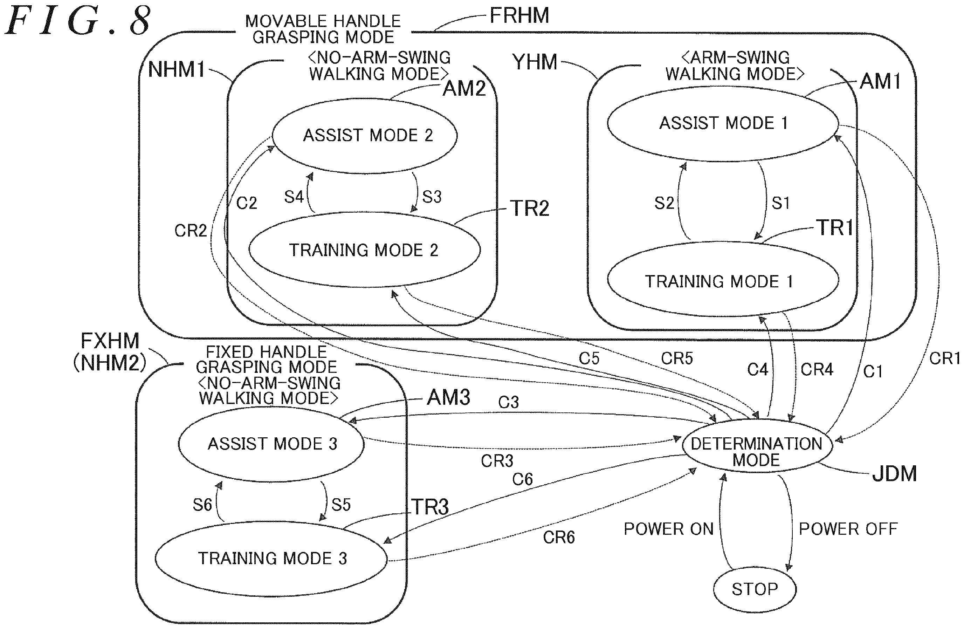

The determination of the operation mode of the walking assist device 10 (see FIG. 1) by the control unit 40 (see FIG. 7) and the processes based on the determined operation mode will be described in detail with reference to FIGS. 8 to 18.

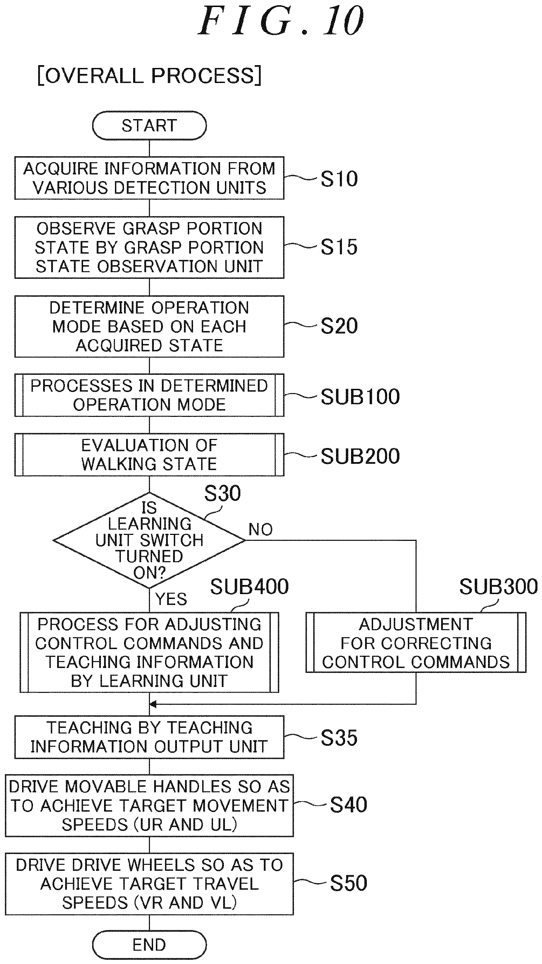

FIG. 8 is a state transition diagram illustrating the operation modes of the walking assist device 10 determined based on outputs of the various detection units. FTG. 9 illustrates conditions for transitioning from a determination mode JDM to various operation modes in FIG. 8 and conditions for returning to the determination mode JDM. FIG. 10 is a flowchart illustrating the procedure of the overall process for the control unit 40 of the walking assist device 10.

FIG. 8 illustrates the operation modes of the walking assist device 10 determined based on outputs of the various detection units. As illustrated in FIG. 8, the walking assist device 10 has operation modes including the determination mode JDM, an assist mode 1 (AM1), an assist mode 2 (AM2), an assist mode 3 (AM3), a training mode 1 (TR1), a training mode 2 (TR2), and a training mode 3 (TR3).

When the main switch 72 (see FIG. 7) is turned on (power is turned on), the control unit 40 reads the operation history stored in the storage unit 44, and writes the operation history into the operation history information 58. After that, the control unit 40 causes the walking assist device 10 to transition to the determination mode JDM. After a transition to the determination mode JDM, the control unit 40 acquires each state through the state detection unit 80, and causes the walking assist device 10 to transition to an operation mode based on the acquired state. When the main switch 72 is turned off (power is turned off), the control unit 40 stores information (e.g. the walking distance and the walking time) about the operation history in the operation history information 58 in the storage unit 44, and finishes the operation.

As illustrated in FIG. 8, the operation modes include a fixed handle grasping mode FXHM and a movable handle grasping mode FRHM. In the fixed handle grasping mode FXHM, the user walks while causing the walking assist device 10 to travel by grasping the fixed handles 20FR and 20FL (see FIG. 1). In the movable handle grasping mode FRHM, the user walks while causing the walking assist device 10 to travel by grasping the movable handles 20R and 20L (see FIG. 1).

The movable handle grasping mode FRHM includes a no-arm-swing walking mode NHM1, in which the user grasps the movable handles 20R and 20L but does not swing his/her arms, and an arm-swing walking mode YHM, in which the user swings his/her arms. The fixed handle grasping mode FXHM, in which the user grasps the fixed handles 20FR and 20FL, is a no-arm-swing walking mode NHM2.

The no-arm-swing walking mode NHM1 of the movable handle grasping mode FRHM, in which the user grasps the movable handles 20R and 20L that are fixed at a predetermined position on the rails 30R and 30L (see FIG. 1), corresponds to the fixed handle grasping mode FXHM (no-arm-swing walking mode NHM2). In the arm-swing walking mode YHM, the user walks while causing the walking assist device 10 to travel by grasping the movable handles 20R and 20L and moving the movable handles 20R and 20L along the front-rear direction of the rails 30R and 30L.

The arm-swing walking mode YHM of the movable handle grasping mode FRHM includes the training mode 1 (TR1) and the assist mode 1 (AM1). The no-arm-swing walking mode NHM1 of the movable handle grasping mode FRHM includes the assist mode 2 (AM2) and the training mode 2 (TR2). The fixed handle grasping mode FXHM includes the assist mode 3 (AM3) and the training mode 3 (TR3).

In the assist mode 1 (AM1), the load on operation of the body of the user of the walking assist device 10 can be alleviated. Specifically, the movable handles 20R and 20L can be moved by the grasp portion drive units 32R and 32L applying an assist force that is larger by a predetermined amount than an assist force with which operation (arm swing) of the body of the user performed as the user walks is operation (arm swing) in a no-load state to movement of the movable handles 20R and 20L in the front-rear direction. In addition, the walking assist device 10 can be caused to travel with an assist force that is larger by a predetermined amount than an assist force with which operation (walk) of the body of the user performed as the user walks is operation (walk) in a no-load state. Consequently, the load on operation (walk, arm swing) of the body of the user performed as the user walks can be alleviated.

In the assist mode 2 (AM2) and the assist mode 3 (AM3), the load on operation of the body of the user of the walking assist device 10 can be alleviated. Specifically, the walking assist device 10 can be caused to travel with an assist force that is larger by a predetermined amount than an assist force with which operation (walk) of the body of the user performed as the user walks is operation (walk) in a no-load state. Consequently, the load on operation (walk) of the body of the user performed as the user walks can be alleviated.

In the training mode 1 (TR1), the walking assist device 10 is caused to travel while causing the regenerated power collecting unit 65 to operate. The regenerated power collecting unit 65 is connected to the rear wheels 60RR and 60RL (see FIG. 1), and converts rotational energy into electric power to be collected (see FIGS. 1 and 7). In the training mode 1 (TR1), the walking assist device 10 can be caused to travel by applying a load to movement of the movable handles 20R and 20L in the front-rear direction through the grasp portion drive units 32R and 32L. Consequently, a load can be applied to operation (walk, arm swing) of the body of the user performed as the user walks.

In the training mode 2 (TR2), the walking assist device 10 is caused to travel while causing the regenerated power collecting unit 65 to operate. Thus, it is necessary for the user to push or pull the walking assist device 10 with a stronger force than in the assist mode 2 (AM2) in order to cause the walking assist device 10 to travel. Consequently, a load can be applied to operation (walk) of the body of the user performed as the user walks.

In the training mode 3 (TR3), the walking assist device 10 is caused to travel while causing the regenerated power collecting unit 65 to operate. Thus, it is necessary for the user to push or pull the walking assist device 10 with a stronger force than in the assist mode 3 (AM3) in order to cause the walking assist device 10 to travel. Consequently, a load can be applied to operation (walk) of the body of the user performed as the user walks.

FIG. 9 illustrates conditions for transitioning from the determination mode JDM to various operation modes in FIG. 8 and conditions for returning to the determination mode JDM. In FIG. 9, conditions C1 to C6 are conditions for transitioning from the determination mode JDM to the various operation modes in FIG. 8, and conditions CR1 to CR6 are conditions for returning from the various operation modes to the determination mode JDM. In FIG. 9, the symbol "-" indicates that the state may be either "0" or "1".

A transition to the various operation modes is determined in accordance with the manual mode switching unit 76a (see FIG. 7), the state (see FIG. 1) of the movable handles (20R and 20L), and the state (see FIG. 1) of the fixed handles (20FR and 20FL). The conditions for transitioning from the various operation modes to the determination mode JDM are determined in accordance with the current operation mode, the state of the movable handles (20R and 20L), and the state of the fixed handles (20FR and 20FL).

In FIG. 9, the movable handle grasping state is "1 = grasped" in the case where the grasp portion pressure detection units 25R and 25L (see FIG. 3) detects that the user is grasping either of the movable handles 20R and 20L, and "0 = not grasped" in the case where the grasp portion pressure detection units 25R and 25L detects that the user is not grasping either of the movable handles 20R and 20L.

The fixed handle grasping state is "1 = grasped" in the case where the grasp portion pressure detection units 25FR and 25FL (see FIG. 6) detects that the user is grasping either of the fixed handles 20FR and 20FL, and "0 = not grasped" in the case where the grasp portion pressure detection units 25FR and 25FL detects that the user is not grasping either of the fixed handles 20FR and 20FL.

The state of arm swing with the movable handles 20R and 20L is "1 = with arm swing" in the case where a detection signal with movement of the movable handle 20R or 20L is output from one of the grasp portion position detection unit 34R and the grasp portion position detection unit 34L, and "0 = without arm swing" otherwise.

In the case where one of the conditions C1 to C6 is met, the control unit 40 changes the operation mode to an operation mode corresponding to the condition. Determination of a transition from the determination mode JDM to the various operation modes will be described in detail below.

In the case where the manual mode switching unit 76a selects the "assist mode", the movable handle grasping state is "1 = grasped", the arm swing state is "1 = with arm swing", and the fixed handle grasping state is "0 = not grasped", the condition C1 is met, and the control unit 40 causes the operation mode to transition from the determination mode JDM to the assist mode 1 (AM1).

In the case where the manual mode switching unit 76a selects the "assist mode", the movable handle grasping state is "1 = grasped", the arm swing state is "0 = without arm swing", and the fixed handle grasping state is "0 = not grasped", the condition C2 is met, and the control unit 40 causes the operation mode to transition from the determination mode JDM to the assist mode 2 (AM2).

In the case where the manual mode switching unit 76a selects the "assist mode", the movable handle grasping state is "0 = not grasped", the arm swing state is "0 = without arm swing", and the fixed handle grasping state is "1 = grasped", the condition C3 is met, and the control unit 40 causes the operation mode to transition from the determination mode JDM to the assist mode 3 (AM3).

In the case where the manual mode switching unit 76a selects the "training mode", the movable handle grasping state is "1 = grasped", the arm swing state is "1 = with arm swing", and the fixed handle grasping state is "0 = not grasped", the condition C4 is met, and the control unit 40 causes the operation mode to transition from the determination mode JDM to the training mode 1 (TR1).

In the case where the manual mode switching unit 76a selects the "training mode", the movable handle grasping state is "1 = grasped", the arm swing state is "0 = without arm swing", and the fixed handle grasping state is "0 = not grasped", the condition C5 is met, and the control unit 40 causes the operation mode to transition from the determination mode JDM to the training mode 2 (TR2).

In the case where the manual mode switching unit 76a selects the "training mode", the movable handle grasping state is "0 = not grasped", the arm swing state is "0 = without arm swing", and the fixed handle grasping state is "1 = grasped", the condition C6 is met, and the control unit 40 causes the operation mode to transition from the determination mode JDM to the training mode 3 (TR3).

In the case where one of the conditions CR1 to CR6 is met, the control unit 40 finishes the current operation mode (see FIG. 8), and causes the operation mode to transition to the determination mode JDM. Determination of a transition from the various operation modes to the determination mode JDM will be described in detail below.

In the case where the current mode is the "assist mode 1 (AM1)" and the movable handle grasping state is "0 = not grasped", the condition CR1 is met irrespective of the other states, and the control unit 40 causes the operation mode to transition from the assist mode 1 (AM1) to the determination mode JDM.

In the case where the current mode is the "assist mode 2 (AM2)" and the movable handle grasping state is "0 = not grasped", the condition CR2 is met irrespective of the other states, and the control unit 40 causes the operation mode to transition from the assist mode 2 (AM2) to the determination mode JDM.

In the case where the current mode is the "assist mode 3 (AM3)" and the fixed handle grasping state is "0 = not grasped", the condition CR3 is met irrespective of the other states, and the control unit 40 causes the operation mode to transition from the assist mode 3 (AM3) to the determination mode JDM.

In the case where the current mode is the "training mode 1 (TR1)" and the movable handle grasping state is "0 = not grasped", the condition CR4 is met irrespective of the other states, and the control unit 40 causes the operation mode to transition from the training mode 1 (TR1) to the determination mode JDM.

In the case where the current mode is the "training mode 2 (TR2)" and the movable handle grasping state is "0 = not grasped", the condition CR5 is met irrespective of the other states, and the control unit 40 causes the operation mode to transition from the training mode 2 (TR2) to the determination mode JDM.

In the case where the current mode is the "training mode 3 (TR3)" and the fixed handle grasping state is "0 = not grasped", the condition CR6 is met irrespective of the other states, and the control unit 40 causes the operation mode to transition from the training mode 3 (TR3) to the determination mode JDM.

FIG. 10 is a flowchart illustrating the procedure of the overall process for the control unit 40 (see FIG. 7) of the walking assist device 10 (see FIG. 1). The process procedure for the control unit 40 of the walking assist device 10 will be described with reference to the flowchart in FIG. 10. The operation mode in each process is not given the symbol in FTG. 8 except where it is necessary for convenience of description.

The overall process for the control unit 40 is constituted from: processes for calculation of target movement speeds for the movable handles and calculation of a target travel speed at which the walking assist device 10 is caused to travel (steps S10 to SUB400); teaching by the teaching information output unit (step S35); and processes for drive of the movable handles and drive of the drive wheels (steps S40 and S50). The control unit 40 executes the overall process at intervals of a predetermined time (e.g. at intervals of several milliseconds) when started.

In step S10, the control unit 40 acquires information (detection signal) from the various detection units (grasp portion state detection unit 81, body state detection unit 82, vehicle body state detection unit 83, and atmospheric state detection unit 84) (see FIG. 7), calculates forward-direction evaluation speeds VRhf and VLhf and rearward-direction evaluation speeds VRhb and VLhb, and proceeds to step S15.

In step S15, the grasp portion state observation unit 40a1 observes a grasp portion state, which is the state of each of the movable handles (20R and 20L), based on the detection signal from the grasp portion state detection unit 81, and proceeds to step S20.

In step S20 (determination of the operation mode based on each acquired state), the control unit 40 reads each state acquired through the state detection unit 80 (see FIG. 7) and stored in the storage unit 44, determines the operation mode (see FIG. 8) for which the condition is met in accordance with FIG. 9 based on such information, and proceeds to step SUB100.

In step SUB200, the walking state evaluation unit 40a2 (see FIG. 7) evaluates the walking state of the user based on the grasp portion state observed using the grasp portion state observation unit 40a1, and proceeds to step S30.

In step S30, the control unit 40 proceeds to step SUB400 (process for adjusting control commands and teaching information by the learning unit) in the case where it is determined that a learning unit switch 72a (see FIGS. 1 and 3) is turned on (Yes), and proceeds to step SUB300 (adjustment for correcting control commands) in the case where it is determined that the learning unit switch 72a is not turned on (No).

In step SUB300, the correction adjustment unit 40a3 (see FIG. 7) adjusts control commands for the grasp portion drive units 32R and 32L based on the walking state (step SUB200) that is evaluated using the walking state evaluation unit 40a2, and proceeds to step S35 (teaching by the teaching information output unit).

In step SUB400, the learning unit 40a6 (see FIG. 7) learns the control commands that are adjusted by the correction adjustment unit 40a3 and the teaching information that is extracted by the teaching information extraction unit 40a4 (see FIG. 7) in accordance with a training data set constituted of a combination of a state variable and determination data, and proceeds to step S35 (teaching by the teaching information output unit). The state variable is a variable that indicates the state of the user and that includes at least one of the respective positions (right grasp portion position HPR and left grasp portion position HPL) of the movable handles 20R and 20L with respect to the respective rails 30R and 30L, the respective inclination directions and inclination angles of the movable handles 20R and 20L with respect to the respective rails 30R and 30L, and the respective pressures (right grasp portion front pressure, right grasp portion rear pressure, left grasp portion front pressure, and left grasp portion rear pressure) applied to the movable handles 20R and 20L. The determination data are data for determining the deviation between a target walking state, which is based on a reference walking state that is a walking state serving as a reference for the user, and the actual walking state of the user and fluctuations in the actual walking state of the user.

In step S35, the teaching information extraction unit 40a4 extracts teaching information corresponding to the walking state that is evaluated in step SUB200 from the storage unit 44, outputs a sound and an image corresponding to the extracted teaching information from the monitor 78 (teaching information output unit) (see FIG. 7), and proceeds to step S40.

In step S40, the control unit 40 drives the grasp portion drive units 32R and 32L so as to bring the movement speeds of the movable handles (20R and 20L) to the target movement speeds (UR and UL), and proceeds to step S50.

In step S50, the control unit 40 drives the rear wheels 60RR and 60RL by controlling the drive units 64R and 64L such that the target travel speeds (VR and VL) for the walking assist device 10 are set to target forward travel speeds (VfdR and VfdL), which are the target travel speeds for forward travel, in the case of forward travel, set to target reverse travel speeds (VbdR and VbdL), which are the target travel speeds for reverse travel, in the case of reverse travel, and set to "0" otherwise, and finishes the overall process.

Step S10 (acquisition of information from each detection unit) will be described in detail below.

In step S10, the control unit 40 acquires information (detection signal) from the state detection unit 80 (grasp portion state detection unit 81, body state detection unit 82, vehicle body state detection unit 83, and atmospheric state detection unit 84), and stores a variety of detected states (input states) in the storage unit 44. The control unit 40 calculates forward-direction evaluation speeds VRhf and VLhf and rearward-direction evaluation speeds VRhb and VLhb based on the information acquired through the state detection unit 80, and stores such evaluation speeds in the storage unit 44. The control unit 40 finishes the acquisition of each state through the state detection unit (step S10), and returns to the overall process.

For example, the control unit 40 detects and stores the following input states in the storage unit 44 in step S10.

The grasp portion state (state of the fixed handles 20FR and 20FL and the movable handles 20R and 20L) includes the following.
(1) Fixed handle grasping state (whether or not the handle is grasped) and fixed handle acting force.
(2) Movable handle grasping state (whether or not the handle is grasped, grasp portion front pressure, and grasp portion rear pressure), movable handle acting force (a force obtained by subtracting the grasp portion rear pressure from the grasp portion front pressure), and arm swing state (amount of movement of the movable handle).
(3) Forward-direction evaluation speeds (VRhf and VLhf) and rearward-direction evaluation speeds (VRhb and VLhb).
(4) Right grasp portion position (HPR) and left grasp portion position (HPL).

The body state of the user includes the following.
(1) Heart rate and body temperature: the heart rate and the body temperature of the user during use of the walking assist device 10.

The vehicle body state of the walking assist device 10 includes the following.
(1) Travel speeds (VdR and VdL): the travel speeds of the rear wheels 60RR and 60RL to travel forward or rearward (corresponding to the rotational speeds of the rear wheels 60RR and 60RL).
(2) Acceleration: acceleration applied to the walking assist device 10 for each of the axes in the three directions, namely the X axis, the Y axis, and the Z axis.
(3) Angular speeds: angular speeds for rotation about each of the axes in the three directions, namely the X axis, the Y axis, and the Z axis (pitch angular speed, yaw angular speed, and roll angular speed).
(4) Accumulated walking time: accumulated time of walk of the user with the walking assist device 10 stored in the storage unit 44.
(5) Accumulated walking distance: accumulated distance of walk of the user with the walking assist device 10 stored in the storage unit 44.

The surrounding atmospheric state includes the following.
(1) Outside temperature: the temperature of outside air around the walking assist device 10.

The output information from the control panel 70 includes the following.
(1) State of main switch 72: whether the main switch of the walking assist device 10 is on (operation enabled) or off (operation disabled).
(2) State of manual mode switching unit 76a: the operation mode of the walking assist device 10 selected by the user.
(3) State of automatic mode switching unit switch 76b: whether the switch is on (automatic operation mode switching enabled) or off (automatic operation mode switching disabled).
(4) Assist adjustment amount: the adjustment amount for adjusting the magnitude of an assist force in the assist mode.
(5) Load adjustment amount: the adjustment amount for adjusting the magnitude of a load in the training mode.
(6) Learning unit switch 72a: whether the switch configured to cause the learning unit 40a6 to operate is on (operation enabled) or off (operation disabled).

Step S15 (observation of the grasp portion state by the grasp portion state observation unit) will be described in detail below. The grasp portion state observation unit 40a1 observes the following grasp portion state, which is the state of the movable handles 20R and 20L (grasp portions), based on the detection signal from the grasp portion state detection unit 81. Observed values as the observed grasp portion state and calculated values calculated based on the observed values are stored in the storage unit 44.

The grasp portion state observation unit 40a1 evaluates the walking state based on the grasp portion state during a period excluding a predetermined post-start period, which is a predetermined period immediately after the user starts walking using the walking assist device 10, a predetermined pre-end period, which is a predetermined period immediately before the user finishes walking using the walking assist device 10, and a predetermined turn period, which is a predetermined period before and after a right turn or a left turn made by the user using the walking assist device 10.

The state about the grasp portion pressure includes the following.
(1) Right grasp portion front pressure: a pressure that matches a grasping force observed based on a detection signal from the grasp portion front pressure detection unit 25fR and applied to the front side of the movable handle 20R that is grasped by the user.
(2) Right grasp portion rear pressure: a pressure that matches a grasping force observed based on a detection signal from the grasp portion rear pressure detection unit 25bR and applied to the rear side of the movable handle 20R that is grasped by the user.
(3) Left grasp portion front pressure: a pressure that matches a grasping force observed based on a detection signal from the grasp portion front pressure detection unit 25fL and applied to the front side of the movable handle 20L that is grasped by the user.
(4) Left grasp portion rear pressure: a pressure that matches a grasping force observed based on a detection signal from the grasp portion rear pressure detection unit 25bL and applied to the rear side of the movable handle 20L that is grasped by the user.

The state about the inclination of the grasp portion includes the following.
(1) Right grasp portion inclination direction: the inclination direction of the movable handle 20R that is observed based on a detection signal from the grasp portion inclination detection unit 33R.
(2) Right grasp portion inclination angle: the inclination angle of the movable handle 20R that is observed based on a detection signal from the grasp portion inclination detection unit 33R.
(3) Left grasp portion inclination direction: the inclination direction of the movable handle 20L that is observed based on a detection signal from the grasp portion inclination detection unit 33L.
(4) Left grasp portion inclination angle: the inclination angle of the movable handle 20L that is observed based on a detection signal from the grasp portion inclination detection unit 33L.

The state about the position and the speed of the grasp portion includes the following.
(1) Right grasp portion position HPR: the position of the movable handle 20R, which is grasped by the user, on the rail 30R that is observed based on a detection signal from the grasp portion position detection unit 34R.
(2) Left grasp portion position HPL: the position of the movable handle 20L, which is grasped by the user, on the rail 30L that is observed based on a detection signal from the grasp portion position detection unit 34L.
(3) Right grasp portion movement speed: the speed of movement of the movable handle 20R along the rail 30R that is calculated from the right grasp portion position HPR, the previously observed right grasp portion position HPR that is stored in the storage unit 44, and a process interval time. The speed of forward movement of the movable handle 20R is defined as positive, and the speed of rearward movement thereof is defined as negative.
(4) Left grasp portion movement speed: the speed of movement of the movable handle 20L along the rail 30L that is calculated from the left grasp portion position HPL, the previously observed left grasp portion position HPL that is stored in the storage unit 44, and a process interval time. The speed of forward movement of the movable handle 20L is defined as positive, and the speed of rearward movement thereof is defined as negative.

The state about the stroke of the grasp portion includes the following.
(1) Right stroke front end position: the right grasp portion position HPR that is observed with (previously observed right grasp portion movement speed > 0) and (right grasp portion movement speed ≤ 0).
(2) Left stroke front end position: the left grasp portion position HPL that is observed with (previously observed left grasp portion movement speed > 0) and (left grasp portion movement speed ≤ 0).
(3) Right stroke rear end position: the right grasp portion position HPR that is observed with (previously observed right grasp portion movement speed < 0) and (right grasp portion movement speed ≥ 0).
(4) Left stroke rear end position: the left grasp portion position HPL that is observed with (previously observed left grasp portion movement speed < 0) and (left grasp portion movement speed ≥ 0).
(5) Right stroke front end time: the time when the right stroke front end position is observed.
(6) Right stroke rear end time: the time when the right stroke rear end position is observed.
(7) Left stroke front end time: the time when the left stroke front end position is observed.
(8) Left stroke rear end time: the time when the left stroke rear end position is observed.
(9) Right stroke length: the front-rear stroke length of the movable handle 20R along the rail 30R that is calculated by subtracting the right stroke rear end position from the right stroke front end position.
(10) Left stroke length: the front-rear stroke length of the movable handle 20L along the rail 30L that is calculated by subtracting the left stroke rear end position from the left stroke front end position.
(11) Right stroke range: an observed front-rear stroke range of the position of the movable handle 20R in the front-rear direction of the rail 30R.
(12) Left stroke range: an observed front-rear stroke range of the position of the movable handle 20L in the front-rear direction of the rail 30L.
(13) Right stroke middle position: the middle position, in the front-rear direction, of the right stroke range.
(14) Left stroke middle position: the middle position, in the front-rear direction, of the left stroke range.
(15) Right stroke cycle: the cycle (cycle of arm swing of the user) of movement of the movable handle 20R on the rail 30R that is calculated from the right stroke rear end time and the right stroke front end time.
(16) Left stroke cycle: the cycle (cycle of arm swing of the user) of movement of the movable handle 20L on the rail 30L that is calculated from the left stroke rear end time and the left stroke front end time.

The average of each of the states of the stroke of the grasp portion is exemplified as follows.

The grasp portion state observation unit 40a1 observes the grasp portion state during a predetermined observation period (predetermined time, predetermined number of times of arm swing), and stores the observed state in the storage unit 44. The grasp portion state observation unit 40a1 calculates the average grasp portion state based on the stored grasp portion state.
(1) Average right stroke length: the average of 100 right stroke lengths calculated.
(2) Average left stroke length: the average of 100 left stroke lengths calculated.
(3) Average right stroke middle position: the average of 100 right stroke middle positions calculated.
(4) Average left stroke middle position: the average of 100 left stroke middle positions calculated.
(5) Average right stroke cycle: the average of 100 right stroke cycles calculated.
(6) Average left stroke cycle: the average of 100 left stroke cycles calculated.
(7) Average right stroke speed: the average stroke speed that is calculated from the average right stroke length and the average right stroke cycle.
(8) Average left stroke speed: the average stroke speed that is calculated from the average left stroke length and the average left stroke cycle.

The grasp portion state observation unit 40a1 observes, as the state variable, at least one of the respective positions (right grasp portion position HPR and left grasp portion position HPL) of the movable handles 20R and 20L with respect to the respective rails 30R and 30L, the respective inclination directions and inclination angles of the movable handles 20R and 20L with respect to the respective rails 30R and 30L, and the respective pressures (right grasp portion front pressure, right grasp portion rear pressure, left grasp portion front pressure, and left grasp portion rear pressure) applied to the movable handles 20R and 20L.

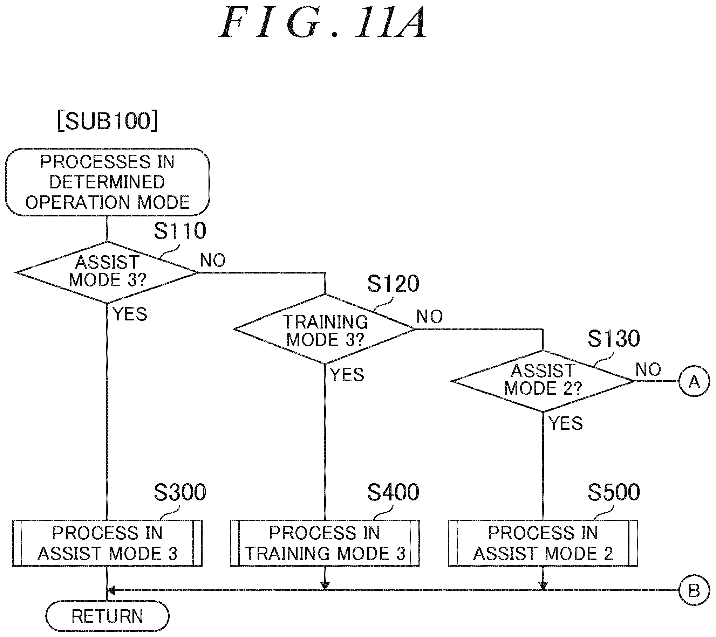

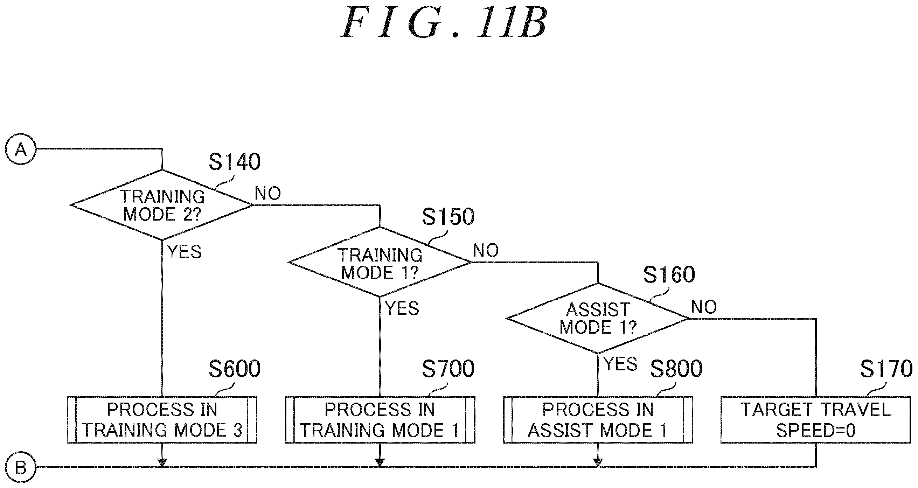

FIG. 11A and FIG. 11B are flowcharts illustrating processes in determined operation modes.

The control unit 40 performs processes in the determined operation mode based on the result of the determination that is made in step S20 (determination of the operation mode based on each acquired state).

In step S110, the control unit 40 proceeds to step S300 in the case where the determined operation mode is the assist mode 3 (AM3) (Yes), and proceeds to step S120 in the case where the determined operation mode is not the assist mode 3 (AM3) (No).

In step S120, the control unit 40 proceeds to step S400 in the case where the determined operation mode is the training mode 3 (TR3) (Yes), and proceeds to step S130 in the case where the determined operation mode is not the training mode 3 (TR3) (No).

In step S130, the control unit 40 proceeds to step S500 in the case where the determined operation mode is the assist mode 2 (AM2) (Yes), and proceeds to step S140 in the case where the determined operation mode is not the assist mode 2 (AM2) (No).

In step S140, the control unit 40 proceeds to step S600 in the case where the determined operation mode is the training mode 2 (TR2) (Yes), and proceeds to step S150 in the case where the determined operation mode is not the training mode 2 (TR2) (No).

In step S150, the control unit 40 proceeds to step S700 in the case where the determined operation mode is the training mode 1 (TR1) (Yes), and proceeds to step S160 in the case where the determined operation mode is not the training mode 1 (TR1) (No).

In step S160, the control unit 40 proceeds to step S800 in the case where the determined operation mode is the assist mode 1 (AM1) (Yes), and proceeds to step S170 in the case where the determined operation mode is not the assist mode 1 (AM1) (No).

In step S170, the control unit 40 sets the target travel speed for the walking assist device 10 to 0 (determination mode), and returns to the overall process.

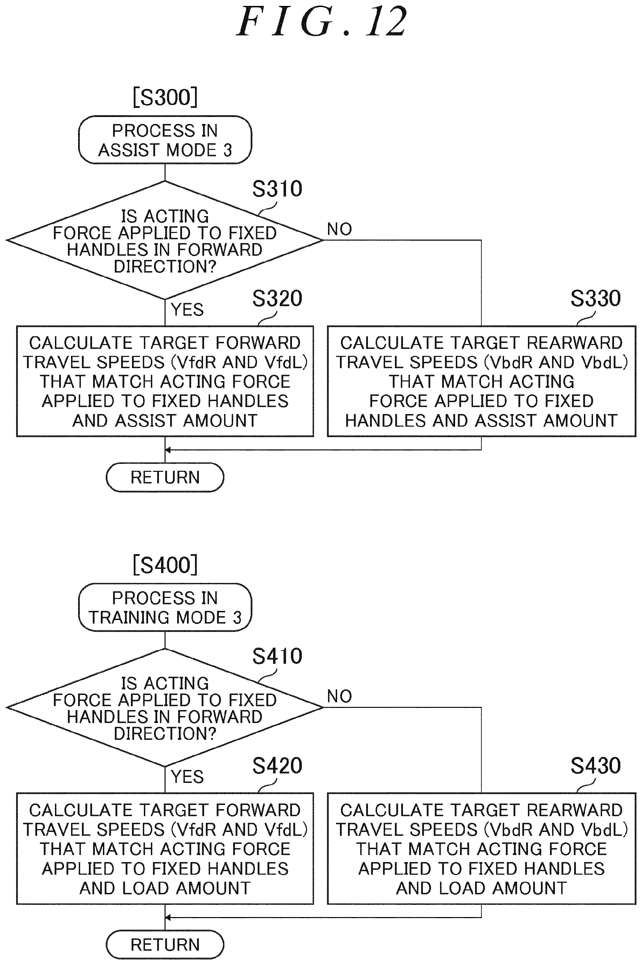

FIG. 12 is a flowchart illustrating the procedure of processes in the assist mode 3 (AM3) in the control unit 40 of the walking assist device 10 (see FIGS. 1, 7, and 8). Step S300 (processes in the assist mode 3) will be described with reference to the flowchart in FIG. 12.

In step S310, the control unit 40 proceeds to step S320 in the case where the acting force of the user applied to the fixed handles 20FR and 20FL is in the forward direction (Yes) based on information from the fixed handle acting force detection unit 81c, and proceeds to step S330 in the case where the acting force of the user applied to the fixed handles 20FR and 20FL is not in the forward direction (No).

In step S320, the control unit 40 calculates the target forward travel speeds (VfdR and VfdL) that match the acting force applied to the fixed handles 20FR and 20FL and the assist amount that is derived by the load amount and assist amount change unit 74, finishes the processes in the assist mode 3 (step S300), and returns to the overall process.

In step S330, the control unit 40 calculates the target rearward travel speeds (VbdR and VbdL) that match the acting force applied to the fixed handles 20FR and 20FL and the assist amount that is derived by the load amount and assist amount change unit 74, finishes the processes in the assist mode 3 (step S300), and returns to the overall process.

In the assist mode 3 (AM3) (see FIG. 8), the walking assist device 10 can be caused to travel with an assist force that is larger by a predetermined amount than an assist force with which operation (walk) of the body of the user performed as the user walks is operation in a no-load state. Consequently, the load on operation (walk) of the body of the user performed as the user walks can be alleviated.

FIG. 12 is a flowchart illustrating the procedure of processes in the training mode 3 (TR3) in the control unit 40 of the walking assist device 10 (see FIGS. 1, 7, and 8). Step S400 (processes in the training mode 3) will be described with reference to the flowchart in FIG. 12. The walking assist device 10 is not caused to generate an assist force for the acting force of the user with the regenerated power collecting unit 65 operating.

In step S410, the control unit 40 proceeds to step S420 in the case where the acting force of the user applied to the fixed handles 20FR and 20FL is in the forward direction (Yes) based on information from the fixed handle acting force detection unit 81c, and proceeds to step S430 in the case where the acting force of the user applied to the fixed handles 20FR and 20FL is not in the forward direction (No).

In step S420, the control unit 40 calculates the target forward travel speeds (VfdR and VfdL) that match the acting force applied to the fixed handles 20FR and 20FL, finishes the processes in the training mode 3 (step S400), and returns to the overall process.

In step S430, the control unit 40 calculates the target rearward travel speeds (VbdR and VbdL) that match the acting force applied to the fixed handles 20FR and 20FL, finishes the processes in the training mode 3 (step S400), and returns to the overall process.

In the training mode 3 (TR3) (see FTG. 8), since the walking assist device 10 is caused to travel with the regenerated power collecting unit 65 operating, it is necessary for the user to push or pull the walking assist device 10 with a stronger force than that in the assist mode 3 (AM3) in order to cause the walking assist device 10 to travel. Consequently, a load can be applied to operation (walk) of the body of the user performed as the user walks.

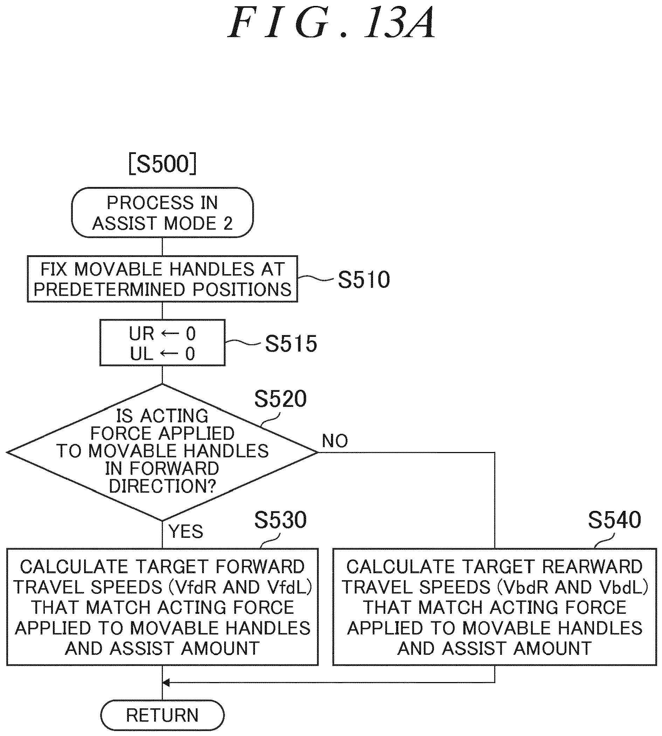

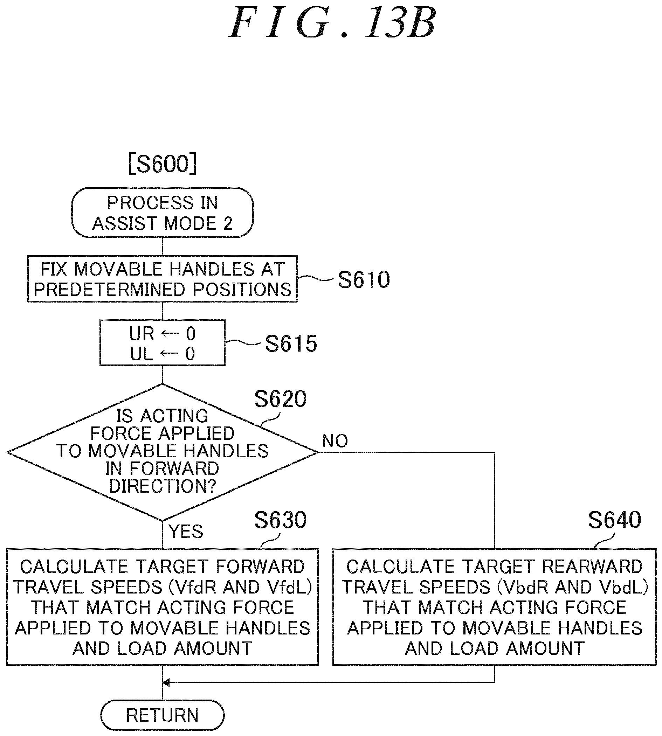

FIG. 13A and FIG. 13B are flowcharts illustrating the procedure of processes in the assist mode 2 (AM2) in the control unit 40 of the walking assist device 10 (see FIGS. 1, 7, and 8). Step S500 (processes in the assist mode 2) will be described with reference to the flowchart in FIG. 13A and FIG. 13B.

In step S510, the control unit 40 fixes the movable handles 20R and 20L at predetermined positions by limiting movement thereof on the rails 30R and 30L due to drive of the grasp portion drive units 32R and 32L using the handle movement limiting units 35R and 35L, and proceeds to step S515.

In step S515, the control unit 40 sets each of the target movement speeds UR and UL to 0 (UR = 0 and UL = 0), and proceeds to step S520.

In step S520, the control unit 40 proceeds to step S530 in the case where the acting force of the user applied to the movable handles 20R and 20L is in the forward direction (Yes) based on information from the movable handle acting force detection unit 81a, and proceeds to step S540 in the case where the acting force of the user applied to the movable handles 20R and 20L is not in the forward direction (No).

In step S530, the control unit 40 calculates the target forward travel speeds (VfdR and VfdL) that match the acting force applied to the movable handles 20R and 20L and the assist amount that is derived by the load amount and assist amount change unit 74, finishes the processes in the assist mode 2 (step S500), and returns to the overall process.

In step S540, the control unit 40 calculates the target rearward travel speeds (VbdR and VbdL) that match the acting force applied to the movable handles 20R and 20L and the assist amount that is derived by the load amount and assist amount change unit 74, finishes the processes in the assist mode 2 (step S500), and returns to the overall process.

In the assist mode 2 (AM2), the walking assist device 10 can be caused to travel with an assist force that is larger by a predetermined amount than an assist force with which operation (walk) of the body of the user performed as the user walks is operation in a no-load state. Consequently, the load on operation (walk) of the body of the user performed as the user walks can be alleviated.

FIG. 13A and FIG. 13B are flowcharts illustrating the procedure of processes in the training mode 2 (TR2) in the control unit 40 of the walking assist device 10 (see FIGS. 1, 7, and 8). Step S600 (processes in the training mode 2) will be described with reference to the flowchart in FIG. 13A and FIG. 13B. The walking assist device 10 is not caused to generate an assist force in accordance with the acting force of the user with the regenerated power collecting unit 65 operating.

In step S610, the control unit 40 fixes the movable handles 20R and 20L at predetermined positions by limiting movement thereof on the rails 30R and 30L due to drive of the grasp portion drive units 32R and 32L using the handle movement limiting units 35R and 35L, and proceeds to step S615.

In step S615, the control unit 40 sets each of the target movement speeds UR and UL to 0 (UR = 0 and UL = 0), and proceeds to step S620.

In step S620, the control unit 40 proceeds to step S630 in the case where the acting force of the user applied to the movable handles 20R and 20L is in the forward direction (Yes) based on information from the movable handle acting force detection unit 81a, and proceeds to step S640 in the case where the acting force of the user applied to the movable handles 20R and 20L is not in the forward direction (No).

In step S630, the control unit 40 calculates the target forward travel speeds (VfdR and VfdL) that match the acting force applied to the movable handles 20R and 20L, finishes the processes in the training mode 2 (step S600), and returns to the overall process.

In step S640, the control unit 40 calculates the target rearward travel speeds (VbdR and VbdL) that match the acting force applied to the movable handles 20R and 20L, finishes the processes in the training mode 2 (step S600), and returns to the overall process.

In the training mode 2 (TR2) (see FIG. 8), since the walking assist device 10 is caused to travel with the regenerated power collecting unit 65 operating, it is necessary for the user to push or pull the walking assist device 10 with a stronger force than that in the assist mode 2 (AM2) in order to cause the walking assist device 10 to travel. Consequently, a load can be applied to operation (walk) of the body of the user performed as the user walks.

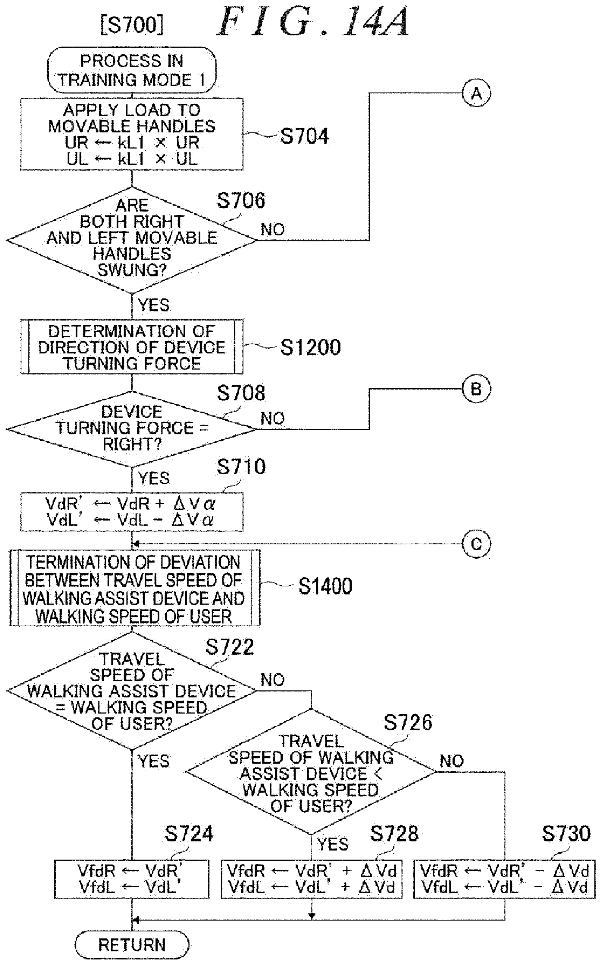

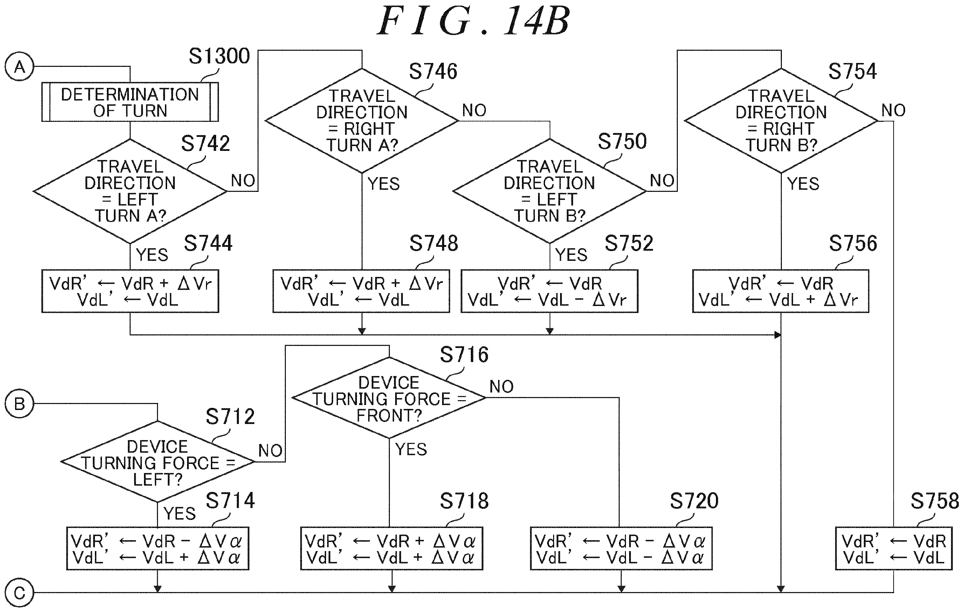

FIG. 14A and FIG. 14B are flowcharts illustrating the procedure of processes in the training mode 1 (TR1) in the control unit 40 of the walking assist device 10 (see FIGS. 1, 7, and 8). Step S700 (processes in the training mode 1) will be described with reference to the flowchart in FIG. 14A and FIG. 14B. An assist force is not generated for the acting force of the user with the regenerated power collecting unit 65 operating.

In step S704, the control unit 40 sets the target movement speed UR to kL1×UR (UR = kL1×UR), sets UL to kL1×UL (UL = kL1×UL), and proceeds to step S706. The speed coefficient kL1 is a speed coefficient (< 1) for applying a load with a load amount derived by the load amount and assist amount change unit 74 to movement of the movable handles 20R and 20L.

In step S706, the control unit 40 proceeds to step S1200 (determination of the direction of a device turning force) in the case where both the right movable handle 20R and the left movable handle 20L are moved, that is, both the right and left arms are swung (Yes), based on information from the movable handle movement amount detection unit 81b, and proceeds to step S1300 (determination of a turn) in the case where both the right and left arms are not swung (No).

In step S708, the control unit 40 proceeds to step S710 in the case where the direction of the device turning force of the walking assist device 10 is "right" (Yes), and proceeds to step S712 in the case where the direction of the device turning force of the walking assist device 10 is not "right" (No).

In step S710, the control unit 40 sets the target travel speed VdR' for the rear wheel 60RR, which serves as the right drive wheel of the walking assist device 10, as VdR' = VdR + ΔVα (predetermined speed), sets the target travel speed VdL' for the rear wheel 60RL, which serves as the left drive wheel, as VdL' = VdL - ΔVα, and proceeds to step S1400 (determination of the deviation between the travel speed of the walking assist device and the walking speed of the user). ΔVα is a predetermined speed corresponding to the magnitude of the device turning force (yaw angular speed), and is stored in the storage unit 44 in advance. Consequently, the device turning force toward the right is reduced by making the rotational speed of the right rear wheel 60RR higher than the rotational speed of the left rear wheel 60RL.

In step S712, the control unit 40 proceeds to step S714 in the case where the direction of the device turning force of the walking assist device 10 is "left" (Yes), and proceeds to step S716 in the case where the direction of the device turning force of the walking assist device 10 is not "left" (No).

In step S714, the control unit 40 sets the target travel speed VdR' for the rear wheel 60RR, which serves as the right drive wheel of the walking assist device 10, as VdR' = VdR - ΔVα, sets the target travel speed VdL' for the rear wheel 60RL, which serves as the left drive wheel, as VdL' = VdL + ΔVα, and proceeds to step S1400 (determination of the deviation between the travel speed of the walking assist device and the walking speed of the user). Consequently, the device turning force toward the right is reduced by making the rotational speed of the left rear wheel 60RL higher than the rotational speed of the right rear wheel 60RR.

In step S716, the control unit 40 proceeds to step S718 in the case where the direction of the device turning force of the walking assist device 10 is "front" (Yes), and proceeds to step S720 in the case where the direction of the device turning force of the walking assist device 10 is not "front" (No).

In step S718, the control unit 40 sets the target travel speed VdR' for the rear wheel 60RR, which serves as the right drive wheel of the walking assist device 10, as VdR' = VdR + ΔVα, sets the target travel speed VdL' for the rear wheel 60RL, which serves as the left drive wheel, as VdL' = VdL + ΔVα, and proceeds to step S1400 (determination of the deviation between the travel speed of the walking assist device and the walking speed of the user).

In step S720, the control unit 40 sets the target travel speed VdR' for the rear wheel 60RR, which serves as the right drive wheel of the walking assist device 10, as VdR' = VdR - ΔVα, sets the target travel speed VdL' for the rear wheel 60RL, which serves as the left drive wheel, as VdL' = VdL - ΔVα, and proceeds to step S1400 (determination of the deviation between the travel speed of the walking assist device and the walking speed of the user).

In step S742, the control unit 40 proceeds to step S744 in the case where the travel direction of the walking assist device 10 is "left turn A" (Yes), and proceeds to step S746 in the case where the travel direction of the walking assist device 10 is not "left turn A" (No).

In step S744, the control unit 40 sets the target travel speed VdR' for the rear wheel 60RR, which serves as the right drive wheel of the walking assist device 10, as VdR' = VdR + ΔVr (predetermined speed), sets the target travel speed VdL' for the rear wheel 60RL, which serves as the left drive wheel, as VdL' = VdL, and proceeds to step S1400 (determination of the deviation between the travel speed of the walking assist device and the walking speed of the user). ΔVr is a predetermined speed corresponding to the travel speeds (VdR and VdL), and is stored in the storage unit 44 in advance.

In step S746, the control unit 40 proceeds to step S748 in the case where the travel direction of the walking assist device 10 is "right turn A" (Yes), and proceeds to step S750 in the case where the travel direction of the walking assist device 10 is not "right turn A" (No).

In step S748, the control unit 40 sets the target travel speed VdR' for the rear wheel 60RR, which serves as the right drive wheel of the walking assist device 10, as VdR' = VdR - ΔVr, sets the target travel speed VdL' for the rear wheel 60RL, which serves as the left drive wheel, as VdL' = VdL, and proceeds to step S1400 (determination of the deviation between the travel speed of the walking assist device and the walking speed of the user).

In step S750, the control unit 40 proceeds to step S752 in the case where the travel direction of the walking assist device 10 is "left turn B" (Yes), and proceeds to step S754 in the case where the travel direction of the walking assist device 10 is not "left turn B" (No).

In step S752, the control unit 40 sets the target travel speed VdR' for the rear wheel 60RR, which serves as the right drive wheel of the walking assist device 10, as VdR' = VdR, sets the target travel speed VdL' for the rear wheel 60RL, which serves as the left drive wheel, as VdL' = VdL - ΔVr, and proceeds to step S1400 (determination of the deviation between the travel speed of the walking assist device and the walking speed of the user).

In step S754, the control unit 40 proceeds to step S756 in the case where the travel direction of the walking assist device 10 is "right turn B" (Yes), and proceeds to step S758 in the case where the travel direction of the walking assist device 10 is not "right turn B" (No).

In step S756, the control unit 40 sets the target travel speed VdR' for the rear wheel 60RR, which serves as the right drive wheel of the walking assist device 10, as VdR' = VdR, sets the target travel speed VdL' for the rear wheel 60RL, which serves as the left drive wheel, as VdL' = VdL + ΔVr, and proceeds to step S1400 (determination of the deviation between the travel speed of the walking assist device and the walking speed of the user).

In step S758, the control unit 40 sets the target travel speed VdR' for the rear wheel 60RR, which serves as the right drive wheel of the walking assist device 10, as VdR' = VdR, sets the target travel speed VdL' for the rear wheel 60RL, which serves as the left drive wheel, as VdL' = VdL, and proceeds to step S1400 (determination of the deviation between the travel speed of the walking assist device and the walking speed of the user).

In step S722, the control unit 40 proceeds to step S724 in the case where the travel speed of the walking assist device 10 is equal to the walking speed of the user (Yes), and proceeds to step S726 in the case where the travel speed of the walking assist device 10 is not equal to the walking speed of the user (No).

In step S724, the control unit 40 sets the target forward travel speed VfdR for the rear wheel 60RR, which serves as the right drive wheel of the walking assist device 10, as VfdR = VdR', sets the target forward travel speed VfdL for the rear wheel 60RL, which serves as the left drive wheel, as VfdL = VdL', finishes the processes in the training mode 1 (step S700), and returns to the overall process.

In step S726, the control unit 40 proceeds to step S728 in the case where the travel speed of the walking assist device 10 is lower than the walking speed of the user (Yes), and proceeds to step S730 in the case where the travel speed of the walking assist device 10 is not lower than the walking speed of the user (No).

In step S728, the control unit 40 sets the target forward travel speed VfdR for the rear wheel 60RR, which serves as the right drive wheel of the walking assist device 10, as VfdR = VdR' + ΔVd (predetermined speed), sets the target forward travel speed VfdL for the rear wheel 60RL, which serves as the left drive wheel, as VfdL = VdL' + ΔVd (predetermined speed), finishes the processes in the training mode 1 (step S700), and returns to the overall process. ΔVd is a predetermined speed corresponding to the magnitude of the target travel speeds (VdR' and VdL') for correcting the target travel speeds, and is stored in the storage unit 44 in advance.

In step S730, the control unit 40 sets the target forward travel speed VfdR for the rear wheel 60RR, which serves as the right drive wheel of the walking assist device 10, as VfdR = VdR' - ΔVd, sets the target forward travel speed VfdL for the rear wheel 60RL, which serves as the left drive wheel, as VfdL = VdL' - ΔVd, finishes the processes in the training mode 1 (step S700), and returns to the overall process.

In the training mode 1 (TR1) (see FIG. 8), the walking assist device 10 can be caused to travel by applying a load to movement of the movable handles 20R and 20L in the front-rear direction through the grasp portion drive units 32R and 32L. Consequently, a load can be applied to operation (arm swing) of the body of the user performed as the user walks.

In the case where it is detected that the right movable handle 20R is moving forward and the left movable handle 20L is moving rearward, the control unit 40 controls the rotational speed of the left rear wheel 60RL so as to become higher than the rotational speed of the right rear wheel 60RR in order to reduce the device turning force toward the left. In the case where it is detected that the right movable handle 20R is moving rearward and the left movable handle 20L is moving forward, the control unit 40 controls the rotational speed of the right rear wheel 60RR so as to become higher than the rotational speed of the left rear wheel 60RL in order to reduce the device turning force toward the right.

The control unit 40 determines that the user desires to turn the walking assist device 10 to the right in the case where the movable handle 20L is stationary and the movable handle 20R is moving rearward (right turn A) and in the case where the movable handle 20R is stationary and the movable handle 20L is moving forward (right turn B). In the case where the right turn A is determined, the control unit 40 controls the drive unit 64R such that the rear wheel 60RR, which serves as the right drive wheel, is at a speed that is the predetermined speed (ΔVr) lower than the travel speed (VdR). In the case where the right turn B is determined, the control unit 40 controls the drive unit 64L such that the rear wheel 60RL, which serves as the left drive wheel, is at a speed that is the predetermined speed (ΔVr) higher than the travel speed (VdL).

The control unit 40 determines that the user desires to turn the walking assist device 10 to the left in the case where the movable handle 20L is stationary and the movable handle 20R is moving forward (left turn A) and in the case where the movable handle 20R is stationary and the movable handle 20L is moving rearward (left turn B). In the case where the left turn A is determined, the control unit 40 controls the drive unit 64R such that the rear wheel 60RR, which serves as the right drive wheel, is at a speed that is the predetermined speed (ΔVr) higher than the travel speed (VdR). In the case where the left turn B is determined, the control unit 40 controls the drive unit 64L such that the rear wheel 60RL, which serves as the left drive wheel, is at a speed that is the predetermined speed (ΔVr) lower than the travel speed (VdL).

In the case where the travel speeds (VdR and VdL) of the walking assist device 10 and the walking speed of the user are equal to each other, the magnitudes of an evaluation speed Vhfd in the forward direction and an evaluation speed Vhbd in the rearward direction are equal to each other if the magnitudes of the speeds of front-rear arm swing by the user are equal to each other. In the case where the travel speed of the walking assist device 10 is lower than the walking speed of the user, on the other hand, the magnitude of the evaluation speed Vhfd in the forward direction is larger than the magnitude of the evaluation speed Vhbd in the rearward direction because of the difference between the walking speed of the user and the travel speed of the walking assist device 10. Thus, in order to correct the deviation between the travel speed of the walking assist device 10 and the walking speed of the user, in the case where the walking speed of the user is higher than the travel speed of the walking assist device 10, the control unit 40 sets the target travel speed VdR' for the rear wheel 60RR, which serves as the right drive wheel of the walking assist device 10, as VdR' = VdR' + ΔVd, and sets the target travel speed VdL' for the rear wheel 60RL, which serves as the left drive wheel, as VdL' = VdL' + ΔVd. Consequently, the deviation between the travel speed of the walking assist device 10 and the walking speed of the user can be corrected.

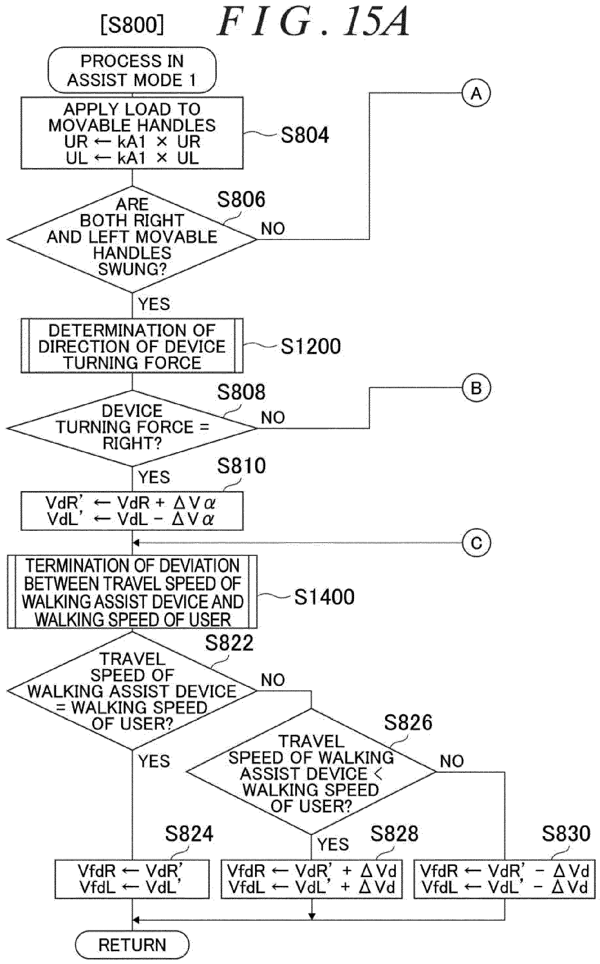

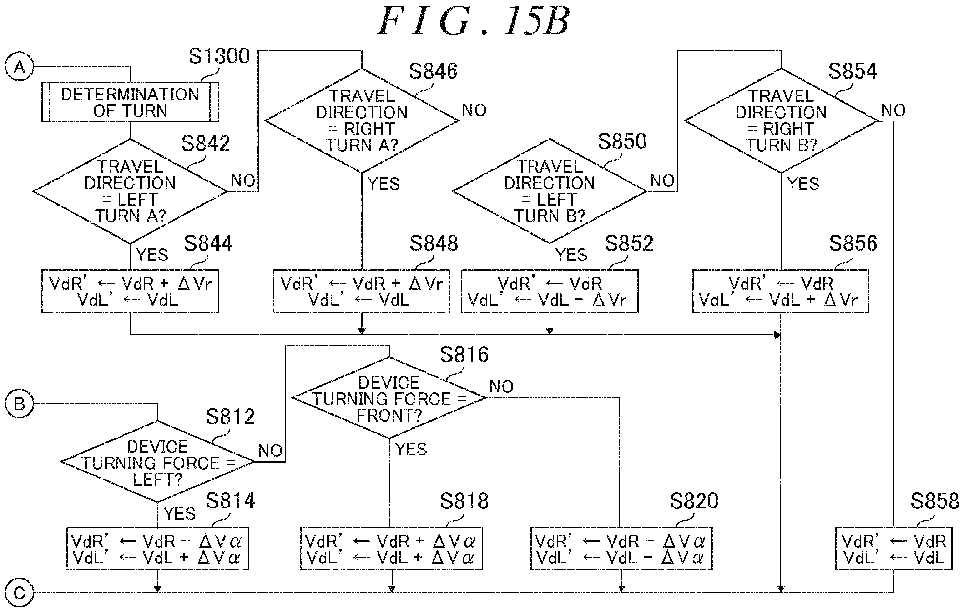

FIG. 15A and FIG. 15B are flowcharts illustrating the procedure of processes in the assist mode 1 (AM1) in the control unit 40 of the walking assist device 10 (see FIGS. 1, 7, and 8). Step S800 (processes in the assist mode 1) will be described with reference to the flowchart in FIG. 15A and FIG. 15B. The processes in the assist mode 1 are the same as those in step S700 (processes in the training mode 1) except for the control (step S804) for driving the grasp portion drive units (32R and 32L) so as to apply an assist force for assisting movement of the movable handles 20R and 20L.

In step S804, the control unit 40 sets the target movement speed UR to kA1×UR (UR = kA1×UR), sets UL to kA1×UL (UL = kA1×UL), and proceeds to step S806. The speed coefficient kA1 is a speed coefficient (> 1) for providing assist with an assist amount derived by the load amount and assist amount change unit 74 to movement of the movable handles 20R and 20L.

In step S806, the control unit 40 proceeds to step S1200 (determination of the direction of a device turning force) in the case where both the right movable handle 20R and the left movable handle 20L are moved, that is, both the right and left arms are swung (Yes), based on information from the movable handle movement amount detection unit 81b, and proceeds to step S1300 (determination of a turn) in the case where both the right and left arms are not swung (No).

In step S808, the control unit 40 proceeds to step S810 in the case where the direction of the device turning force of the walking assist device 10 is "right" (Yes), and proceeds to step S812 in the case where the direction of the device turning force of the walking assist device 10 is not "right" (No).

In step S810, the control unit 40 sets the target travel speed VdR' for the rear wheel 60RR, which serves as the right drive wheel of the walking assist device 10, as VdR' = VdR + ΔVα (predetermined speed), sets the target travel speed VdL' for the rear wheel 60RL, which serves as the left drive wheel, as VdL' = VdL - ΔVα, and proceeds to step S1400 (determination of the deviation between the travel speed of the walking assist device and the walking speed of the user). ΔVα is a predetermined speed corresponding to the magnitude of the device turning force (yaw angular speed), and is stored in the storage unit 44 in advance. Consequently, the device turning force toward the right is reduced by making the rotational speed of the right rear wheel 60RR higher than the rotational speed of the left rear wheel 60RL.

In step S812, the control unit 40 proceeds to step S814 in the case where the direction of the device turning force of the walking assist device 10 is "left" (Yes), and proceeds to step S816 in the case where the direction of the device turning force of the walking assist device 10 is not "left" (No).

In step S814, the control unit 40 sets the target travel speed VdR' for the rear wheel 60RR, which serves as the right drive wheel of the walking assist device 10, as VdR' = VdR - ΔVα, sets the target travel speed VdL' for the rear wheel 60RL, which serves as the left drive wheel, as VdL' = VdL + ΔVα, and proceeds to step S1400 (determination of the deviation between the travel speed of the walking assist device and the walking speed of the user). Consequently, the device turning force toward the left is reduced by making the rotational speed of the left rear wheel 60RL higher than the rotational speed of the right rear wheel 60RR.

In step S816, the control unit 40 proceeds to step S818 in the case where the direction of the device turning force of the walking assist device 10 is "front" (Yes), and proceeds to step S820 in the case where the direction of the device turning force of the walking assist device 10 is not "front" (No).

In step S818, the control unit 40 sets the target travel speed VdR' for the rear wheel 60RR, which serves as the right drive wheel of the walking assist device 10, as VdR' = VdR + ΔVα, sets the target travel speed VdL' for the rear wheel 60RL, which serves as the left drive wheel, as VdL' = VdL + ΔVα, and proceeds to step S1400 (determination of the deviation between the travel speed of the walking assist device and the walking speed of the user).

In step S820, the control unit 40 sets the target travel speed VdR' for the rear wheel 60RR, which serves as the right drive wheel of the walking assist device 10, as VdR' = VdR - ΔVα, sets the target travel speed VdL' for the rear wheel 60RL, which serves as the left drive wheel, as VdL' = VdL - ΔVα, and proceeds to step S1400 (determination of the deviation between the travel speed of the walking assist device and the walking speed of the user).

In step S842, the control unit 40 proceeds to step S844 in the case where the travel direction of the walking assist device 10 is "left turn A" (Yes), and proceeds to step S846 in the case where the travel direction of the walking assist device 10 is not "left turn A" (No).

In step S844, the control unit 40 sets the target travel speed VdR' for the rear wheel 60RR, which serves as the right drive wheel of the walking assist device 10, as VdR' = VdR + ΔVr (predetermined speed), sets the target travel speed VdL' for the rear wheel 60RL, which serves as the left drive wheel, as VdL' = VdL, and proceeds to step S1400 (determination of the deviation between the travel speed of the walking assist device and the walking speed of the user). ΔVr is a predetermined speed corresponding to the travel speeds (VdR and VdL), and is stored in the storage unit 44 in advance.

In step S846, the control unit 40 proceeds to step S848 in the case where the travel direction of the walking assist device 10 is "right turn A" (Yes), and proceeds to step S850 in the case where the travel direction of the walking assist device 10 is not "right turn A" (No).

In step S848, the control unit 40 sets the target travel speed VdR' for the rear wheel 60RR, which serves as the right drive wheel of the walking assist device 10, as VdR' = VdR - ΔVr, sets the target travel speed VdL' for the rear wheel 60RL, which serves as the left drive wheel, as VdL' = VdL, and proceeds to step S1400 (determination of the deviation between the travel speed of the walking assist device and the walking speed of the user).

In step S850, the control unit 40 proceeds to step S852 in the case where the travel direction of the walking assist device 10 is "left turn B" (Yes), and proceeds to step S854 in the case where the travel direction of the walking assist device 10 is not "left turn B" (No).

In step S852, the control unit 40 sets the target travel speed VdR' for the rear wheel 60RR, which serves as the right drive wheel of the walking assist device 10, as VdR' = VdR, sets the target travel speed VdL' for the rear wheel 60RL, which serves as the left drive wheel, as VdL' = VdL - ΔVr (predetermined speed), and proceeds to step S1400 (determination of the deviation between the travel speed of the walking assist device and the walking speed of the user).

In step S854, the control unit 40 proceeds to step S856 in the case where the travel direction of the walking assist device 10 is "right turn B" (Yes), and proceeds to step S858 in the case where the travel direction of the walking assist device 10 is not "right turn B" (No).

In step S856, the control unit 40 sets the target travel speed VdR' for the rear wheel 60RR, which serves as the right drive wheel of the walking assist device 10, as VdR' = VdR, sets the target travel speed VdL' for the rear wheel 60RL, which serves as the left drive wheel, as VdL' = VdL + ΔVr (predetermined speed), and proceeds to step S1400 (determination of the deviation between the travel speed of the walking assist device and the walking speed of the user).

In step S858, the control unit 40 sets the target travel speed VdR' for the rear wheel 60RR, which serves as the right drive wheel of the walking assist device 10, as VdR' = VdR, sets the target travel speed VdL' for the rear wheel 60RL, which serves as the left drive wheel, as VdL' = VdL, and proceeds to step S1400 (determination of the deviation between the travel speed of the walking assist device and the walking speed of the user).

In step S822, the control unit 40 proceeds to step S824 in the case where the travel speed of the walking assist device 10 is equal to the walking speed of the user (Yes), and proceeds to step S826 in the case where the travel speed of the walking assist device 10 is not equal to the walking speed of the user (No).

In step S824, the control unit 40 sets the target forward travel speed VfdR for the rear wheel 60RR, which serves as the right drive wheel of the walking assist device 10, as VfdR = VdR', sets the target forward travel speed VfdL for the rear wheel 60RL, which serves as the left drive wheel, as VfdL = VdL', finishes the processes in the assist mode 1 (step S800), and returns to the overall process.

In step S826, the control unit 40 proceeds to step S828 in the case where the travel speed of the walking assist device 10 is lower than the walking speed of the user (Yes), and proceeds to step S830 in the case where the travel speed of the walking assist device 10 is not lower than the walking speed of the user (No).

In step S828, the control unit 40 sets the target forward travel speed VfdR for the rear wheel 60RR, which serves as the right drive wheel of the walking assist device 10, as VfdR = VdR' + ΔVd (predetermined speed), sets the target forward travel speed VfdL for the rear wheel 60RL, which serves as the left drive wheel, as VfdL = VdL' + ΔVd, finishes the processes in the assist mode 1 (step S800), and returns to the overall process. ΔVd is a predetermined speed corresponding to the magnitude of the target travel speeds (VdR' and VdL') for correcting the target travel speeds, and is stored in the storage unit 44 in advance.

In step S830, the control unit 40 sets the target forward travel speed VfdR for the rear wheel 60RR, which serves as the right drive wheel of the walking assist device 10, as VfdR = VdR' - ΔVd, sets the target forward travel speed VfdL for the rear wheel 60RL, which serves as the left drive wheel, as VfdL = VdL' - ΔVd, finishes the processes in the assist mode 1 (step S800), and returns to the overall process.

In the assist mode 1 (AM1) (see FIG. 8), the movable handles 20R and 20L can be moved by the grasp portion drive units 32R and 32L applying an assist force that is larger by a predetermined amount than an assist force with which operation (arm swing) of the body of the user performed as the user walks is operation (arm swing) in a no-load state to movement of the movable handles 20R and 20L in the front-rear direction. In addition, the walking assist device 10 can be caused to travel with an assist force that is larger by a predetermined amount than an assist force with which operation (walk) of the body of the user performed as the user walks is operation (walk) in a no-load state.

In the case where it is detected that the right movable handle 20R is moving forward and the left movable handle 20L is moving rearward, the control unit 40 controls the rotational speed of the right rear wheel 60RR so as to become higher than the rotational speed of the left rear wheel 60RL in order to reduce the device turning force toward the right. In the case where it is detected that the right movable handle 20R is moving rearward and the left movable handle 20L is moving forward, the control unit 40 controls the rotational speed of the left rear wheel 60RL so as to become higher than the rotational speed of the right rear wheel 60RR in order to reduce the device turning force toward the left.

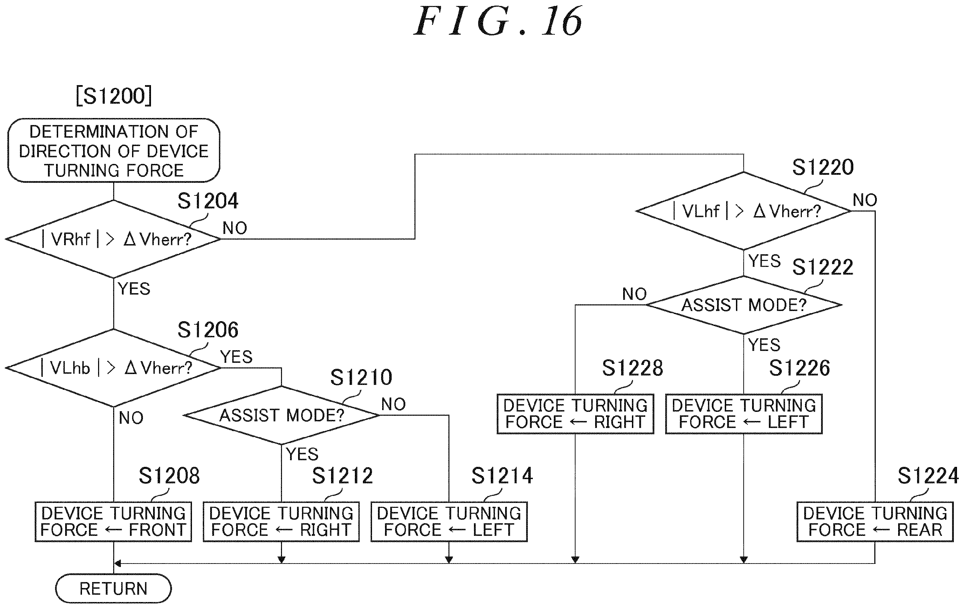

FIG. 16 is a flowchart illustrating the procedure of processes for determination of the direction of a device turning force in the control unit 40 of the walking assist device 10 (see FIGS. 1 and 7). Step S1200 (determination of the direction of a device turning force) will be described with reference to the flowchart in FIG. 16.

In step S1204, the control unit 40 proceeds to step S1206 in the case where the absolute value |VRhf| of the forward-direction evaluation speed of the right movable handle 20R is more than ΔVherr (Yes; it is determined that the right movable handle 20R is moving forward), and proceeds to step S1220 in the case where the absolute value |VRhf| is not more than ΔVherr (No). ΔVherr is a predetermined value determined in advance, and is stored in the storage unit 44.

In step S1206, the control unit 40 proceeds to step S1210 in the case where the absolute value |VLhb| of the rearward-direction evaluation speed of the left movable handle 20L is more than ΔVherr (Yes; it is determined that the left movable handle 20L is moving rearward), and proceeds to step S1208 in the case where the absolute value |VLhb| is not more than ΔVherr (No).

In step S1208, the control unit 40 sets the direction of a device turning force of the walking assist device 10 to "front", and finishes the determination of the direction of a device turning force (step S1200). The control unit 40 proceeds to step S708 in the case where it is called in step S700, and proceeds to step S808 in the case where it is called in step S800. In this case, the user is walking (determined as straight travel) while pushing the walking assist device 10 forward in the state of grasping the movable handles 20R and 20L without swinging the movable handles 20R and 20L in the front-rear direction.

In step S1210, the control unit 40 proceeds to step S1212 in the case where the operation mode of the walking assist device 10 is the "assist mode" (Yes), and proceeds to step S1214 in the case where the operation mode thereof is not the "assist mode" (No).

In step S1212, the control unit 40 sets the direction of a device turning force of the walking assist device 10 to "right", and finishes the determination of the direction of a device turning force (step S1200). The control unit 40 proceeds to step S708 when called in step S700, and proceeds to step S808 when called in step S800. In this case, the user is swinging the movable handle 20R forward and swinging the movable handle 20L rearward, and a device turning force toward the right is generated for the walking assist device 10 as a reaction to the assist force (determined as a device turning force toward the right).

In step S1214, the control unit 40 sets the direction of a device turning force of the walking assist device 10 to "left", and finishes the determination of the direction of a device turning force (step S1200). The control unit 40 proceeds to step S708 in the case where it is called in step S700, and proceeds to step S808 in the case where it is called in step S800. In this case, the user is swinging the movable handle 20R forward and swinging the movable handle 20L rearward, and a device turning force toward the left is generated for the walking assist device 10 as a reaction to the load (determined as a device turning force toward the left).

In step S1220, the control unit 40 proceeds to step S1222 in the case where the absolute value |VLhf| of the forward-direction evaluation speed is more than ΔVherr (Yes; it is determined that the left movable handle 20L is moving forward), and proceeds to step S1224 in the case where |VLhf| is not more than ΔVherr (No).

In step S1222, the control unit 40 proceeds to step S1226 in the case where the operation mode of the walking assist device 10 is the "assist mode" (Yes), and proceeds to step S1228 in the case where the operation mode thereof is not the "assist mode" (No).

In step S1224, the control unit 40 sets the direction of a device turning force of the walking assist device 10 to "rear", and finishes the determination of the direction of a device turning force (step S1200). The control unit 40 proceeds to step S708 when called in step S700, and proceeds to step S808 when called in step S800. In this case, the user is pulling the walking assist device 10 rearward in the state of grasping the movable handles 20R and 20L without swinging the movable handles 20R and 20L in the front-rear direction.

In step S1226, the control unit 40 sets the direction of a device turning force of the walking assist device 10 to "left", and finishes the determination of the direction of a device turning force (step S1200). The control unit 40 proceeds to step S708 when called in step S700, and proceeds to step S808 when called in step S800. In this case, the user is swinging the movable handle 20R rearward and swinging the movable handle 20L forward, and a device turning force toward the left is generated for the walking assist device 10 as a reaction to the assist force (determined as a device turning force toward the left).

In step S1228, the control unit 40 sets the direction of a device turning force of the walking assist device 10 to "right", and finishes the determination of the direction of a device turning force (step S1200). The control unit 40 proceeds to step S708 when called in step S700, and proceeds to step S808 when called in step S800. In this case, the user is swinging the movable handle 20R rearward and swinging the movable handle 20L forward, and a device turning force toward the right is generated for the walking assist device 10 as a reaction to the load (determined as a device turning force toward the right).

In the assist mode, in the case where it is detected that the right movable handle 20R is moving forward and the left movable handle 20L is moving rearward, the control unit 40 determines that a device turning force toward the right is generated. In the case where it is detected that the right movable handle 20R is moving rearward and the left movable handle 20L is moving forward, the control unit 40 determines that a device turning force toward the left is generated.

In the training mode, in the case where it is detected that the right movable handle 20R is moving forward and the left movable handle 20L is moving rearward, the control unit 40 determines that a device turning force toward the left is generated. In the case where it is detected that the right movable handle 20R is moving rearward and the left movable handle 20L is moving forward, the control unit 40 determines that a device turning force toward the right is generated.

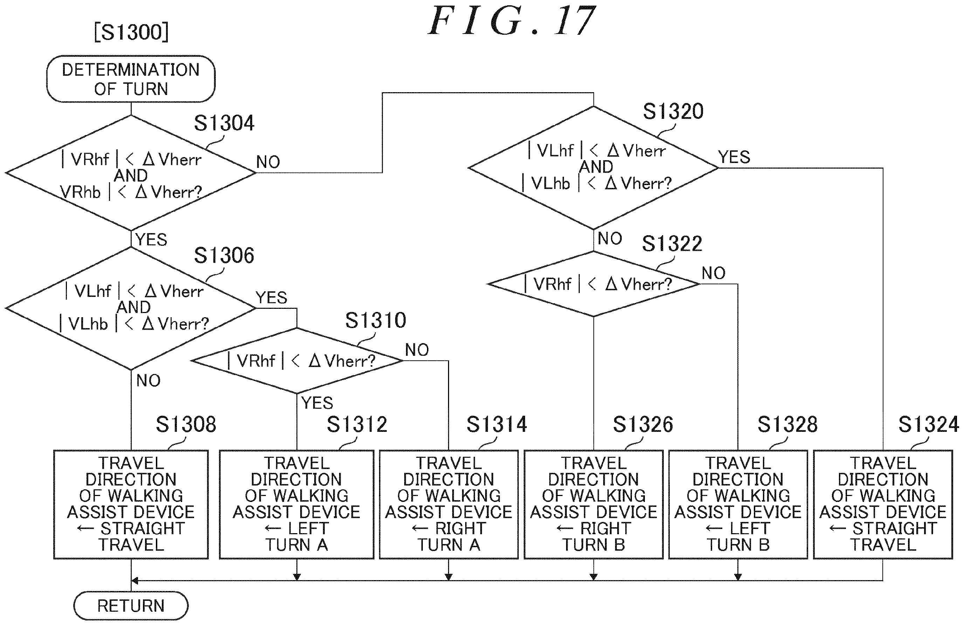

FIG. 17 is a flowchart illustrating the procedure of processes for determination of a turn in the control unit 40 of the walking assist device 10 (see FIGS. 1 and 7). Step S1300 (determination of a turn) will be described with reference to the flowchart in FIG. 17.

In step S1304, the control unit 40 proceeds to step S1320 in the case where the absolute value |VRhf| of the forward-direction evaluation speed of the right movable handle 20R is less than ΔVherr and the absolute value |VRhb| of the rearward-direction evaluation speed is less than ΔVherr (Yes; it is determined that the right movable handle 20R is stationary), and proceeds to step S1306 otherwise (No). ΔVherr is a predetermined value determined in advance, and is stored in the storage unit 44.

In step S1306, the control unit 40 proceeds to step S1310 in the case where the absolute value |VLhf| of the forward-direction evaluation speed of the left movable handle 20L is less than ΔVherr and the absolute value |VLhb| of the rearward-direction evaluation speed is less than ΔVherr (Yes; it is determined that the left movable handle 20L is stationary), and proceeds to step S1308 otherwise (No).

In step S1308, the control unit 40 sets the travel direction of the walking assist device 10 to "straight travel", and finishes the determination of a turn (step S1300). The control unit 40 proceeds to step S742 when called in step S700, and proceeds to step S842 when called in step S800. In this case, the movable handles 20R and 20L are moving in the front-rear direction, and the control unit 40 determines that the user desires straight travel (straight travel).

In step S1310, the process proceeds to step S1312 in the case where the absolute value |VRhf| of the forward-direction evaluation speed is more than ΔVherr (Yes; it is determined that the right movable handle 20R is moving forward), and proceeds to step S1314 in the case where the absolute value |VRhf| is not more than ΔVherr (No).

In step S1312, the control unit 40 sets the travel direction of the walking assist device 10 to "left turn A", and finishes the determination of a turn (step S1300). The control unit 40 proceeds to step S742 when called in step S700, and proceeds to step S842 when called in step S800. In this case, the movable handle 20L is stationary and the movable handle 20R is moving forward, and the control unit 40 determines that the user desires a left turn of the walking assist device 10 (left turn A).

In step S1314, the control unit 40 sets the travel direction of the walking assist device 10 to "right turn A", and finishes the determination of a turn (step S1300). The control unit 40 proceeds to step S742 when called in step S700, and proceeds to step S842 when called in step S800. In this case, the movable handle 20L is stationary and the movable handle 20R is moving rearward, and the control unit 40 determines that the user desires a right turn of the walking assist device 10 (right turn A).

In step S1320, the control unit 40 proceeds to step S1324 in the case where the absolute value |VLhf| of the forward-direction evaluation speed of the left movable handle 20L is less than ΔVherr and the absolute value |VLhb| of the rearward-direction evaluation speed is less than ΔVherr (Yes; it is determined that the left movable handle 20L is stationary), and proceeds to step S1322 otherwise (No).

In step S1322, the process proceeds to step S1326 in the case where the absolute value |VLhf| of the forward-direction evaluation speed is more than ΔVherr (Yes; it is determined that the left movable handle 20L is moving forward), and proceeds to step S1328 in the case where the absolute value |VLhf| is not more than ΔVherr (No).

In step S1324, the control unit 40 sets the travel direction of the walking assist device 10 to "straight travel", and finishes the determination of a turn (step S1300). The control unit 40 proceeds to step S742 when called in step S700, and proceeds to step S842 when called in step S800. In this case, both the movable handles 20R and 20L are stationary, and the control unit 40 determines that the user does not desire a turn (straight travel).

In step S1326, the control unit 40 sets the travel direction of the walking assist device 10 to "right turn B", and finishes the determination of a turn (step S1300). The control unit 40 proceeds to step S742 when called in step S700, and proceeds to step S842 when called in step S800. In this case, the movable handle 20R is stationary and the movable handle 20L is moving rearward, and the control unit 40 determines that the user desires a right turn of the walking assist device 10 (right turn B).

In step S1328, the control unit 40 sets the travel direction of the walking assist device 10 to "left turn B", and finishes the determination of a turn (step S1300). The control unit 40 proceeds to step S742 when called in step S700, and proceeds to step S842 when called in step S800. In this case, the movable handle 20R is stationary and the movable handle 20L is moving forward, and the control unit 40 determines that the user desires a left turn of the walking assist device 10 (left turn B).

The control unit 40 determines that the user desires to turn the walking assist device 10 to the right in the case where the movable handle 20L is stationary and the movable handle 20R is moving rearward (right turn A) and in the case where the movable handle 20R is stationary and the movable handle 20L is moving forward (right turn B). The control unit 40 determines that the user desires to turn the walking assist device 10 to the left in the case where the movable handle 20L is stationary and the movable handle 20R is moving forward (left turn A) and in the case where the movable handle 20R is stationary and the movable handle 20L is moving rearward (left turn B).

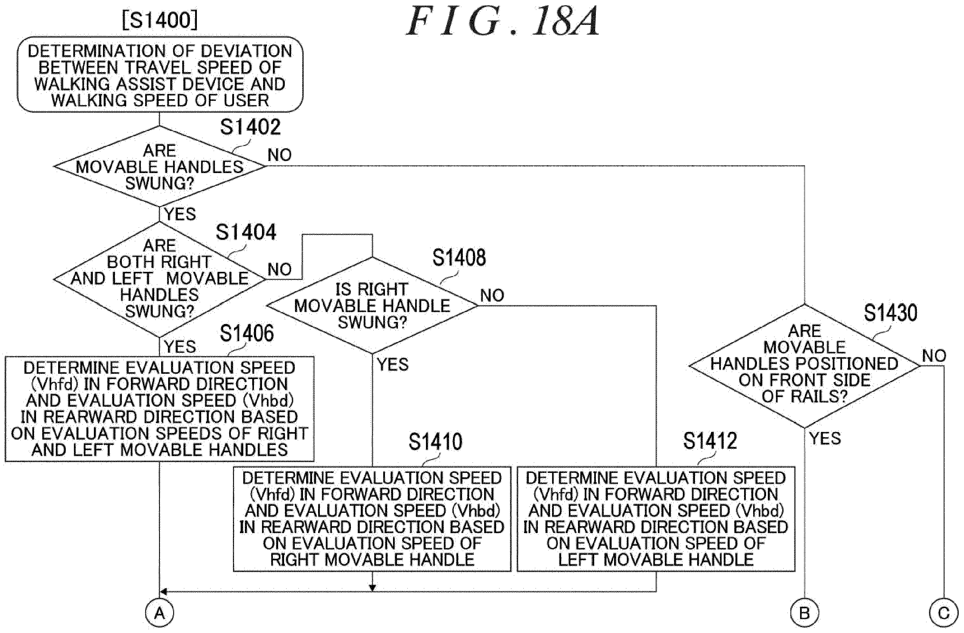

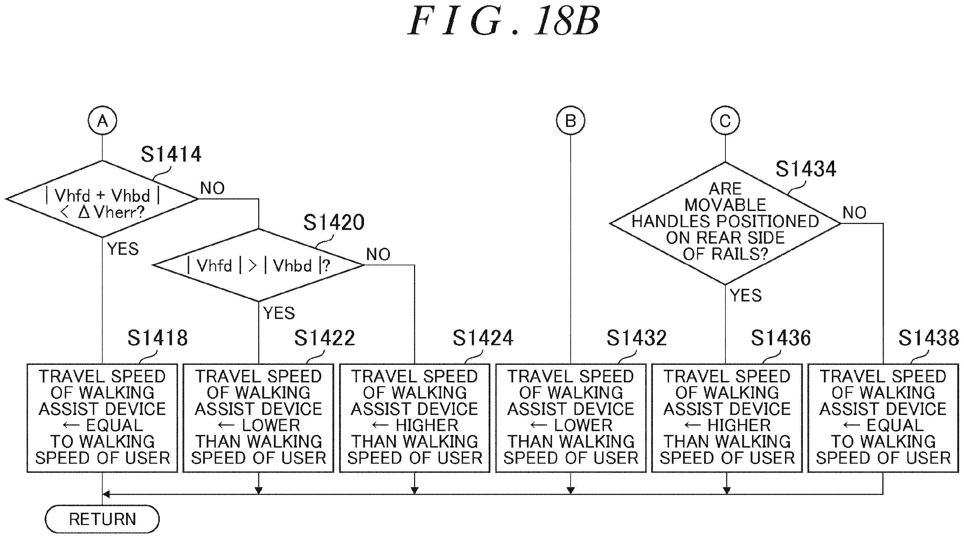

FIG. 18A and FIG. 18B are flowcharts illustrating the procedure of processes for determination of the deviation between the travel speed of the walking assist device 10 and the walking speed of the user in the control unit 40 of the walking assist device 10 (see FIGS. 1 and 7). Step S1400 (determination of the deviation between the travel speed of the walking assist device and the walking speed of the user) will be described with reference to the flowchart in FIG. 18A and FIG. 18B.

In step S1402, the control unit 40 proceeds to step S1404 in the case where the right movable handle 20R or the left movable handle 20L is moving, that is, either of the movable handles is swung (Yes), based on information from the movable handle movement amount detection unit 81b, and proceeds to step S1430 in the case where either of the movable handles is not swung (No).

In step S1404, the control unit 40 proceeds to step S1406 in the case where both the right movable handle 20R and the left movable handle 20L are moved, that is, both the right and left arms are swung (Yes), and proceeds to step S1408 in the case where either of the right and left arms is not swung (No).

In step S1406, the control unit 40 determines an evaluation speed Vhfd in the forward direction and an evaluation speed Vhbd in the rearward direction based on the forward-direction evaluation speeds (VRhf and VLhf) and the rearward-direction evaluation speeds (VRhb and VLhb) of the right and left movable handles 20R and 20L, and proceeds to step S1414. In the case where the amount of movement of the right movable handle 20R is "positive" and the amount of movement of the left movable handle 20L is "negative" (in the case where the right arm of the user is swung in the forward direction and the left arm of the user is swung in the rearward direction), the evaluation speed Vhfd in the forward direction is determined as the forward-direction evaluation speed VRhf, and the evaluation speed Vhbd in the rearward direction is determined as the rearward-direction evaluation speed VLhb. In the case where the amount of movement of the right movable handle 20R is "negative" and the amount of movement of the left movable handle 20L is "positive" (in the case where the left arm of the user is swung in the forward direction and the right arm of the user is swung in the rearward direction), the evaluation speed Vhfd in the forward direction is determined as the forward-direction evaluation speed VLhf, and the evaluation speed Vhbd in the rearward direction is determined as the rearward-direction evaluation speed VRhb.

In step S1408, the control unit 40 proceeds to step S1410 in the case where only the right movable handle 20R is moved, that is, the right arm is swung (Yes), based on information from the movable handle movement amount detection unit 81b, and proceeds to step S1412 in the case where the right arm is not swung (No).

In step S1410, the control unit 40 determines an evaluation speed (Vhfd = VRhf) in the forward direction and an evaluation speed (Vhbd = VRhb) in the rearward direction based on the evaluation speeds (forward-direction evaluation speed VRhf and rearward-direction evaluation speed VRhb) of the right movable handle 20R, and proceeds to step S1414.

In step S1412, the control unit 40 determines an evaluation speed (Vhfd = VLhf) in the forward direction and an evaluation speed (Vhbd = VLhb) in the rearward direction based on the evaluation speeds (forward-direction evaluation speed VLhf and rearward-direction evaluation speed VLhb) of the left movable handle 20L, and proceeds to step S1414.

In step S1414, the control unit 40 proceeds to step S1418 in the case where the absolute value |Vhfd + Vhbd| of the difference between the evaluation speed Vhfd in the forward direction and the evaluation speed Vhbd in the rearward direction is less than ΔVerr that is set in advance (Yes), and proceeds to step S1420 in the case where |Vhfd + Vhbd| is not less than ΔVerr that is set in advance (No). The evaluation speed Vhfd in the forward direction is defined as "positive", and the evaluation speed Vhbd in the rearward direction is defined as "negative". Therefore, the difference between such speeds is the sum thereof (Vhfd + Vhbd).

In step S1418, the control unit 40 sets the travel speed of the walking assist device 10 to be "equal to the walking speed of the user", and finishes the determination of the deviation between the travel speed of the walking assist device and the walking speed of the user (step S1400). The control unit 40 proceeds to step S722 when called in step S700, and proceeds to step S822 when called in step S800.

In step S1420, the control unit 40 proceeds to step S1422 in the case where the absolute value |Vhfd| of the evaluation speed in the forward direction is more than the absolute value |Vhbd| of the evaluation speed in the rearward direction (Yes), and proceeds to step S1424 in the case where the absolute value |Vhfd| of the evaluation speed in the forward direction is not more than the absolute value |Vhbd| of the evaluation speed in the rearward direction (No).

In step S1422, the control unit 40 sets the travel speed of the walking assist device 10 to be "lower than the walking speed of the user", and finishes the determination of the deviation between the travel speed of the walking assist device and the walking speed of the user (step S1400). The control unit 40 proceeds to step S722 when called in step S700, and proceeds to step S822 when called in step S800.

In step S1424, the control unit 40 sets the travel speed of the walking assist device 10 to be "higher than the walking speed of the user", and finishes the determination of the deviation between the travel speed of the walking assist device and the walking speed of the user (step S1400). The control unit 40 proceeds to step S722 when called in step S700, and proceeds to step S822 when called in step S800.

In step S1430, the control unit 40 proceeds to step S1432 in the case where it is determined based on the right grasp portion position HPR and the left grasp portion position HPL that the right movable handle 20R and the left movable handle 20L are positioned on the front side of the rails 30R and 30L (Yes), and proceeds to step S1434 in the case where it is not determined that the right movable handle 20R and the left movable handle 20L are positioned on the front side thereof (No).

In step S1432, the control unit 40 sets the travel speed of the walking assist device 10 to be "lower than the walking speed of the user", and finishes the determination of the deviation between the travel speed of the walking assist device 10 and the walking speed of the user (step S1400). The control unit 40 proceeds to step S722 when called in step S700, and proceeds to step S822 when called in step S800.

In step S1436, the control unit 40 sets the travel speed of the walking assist device 10 to be "higher than the walking speed of the user", and finishes the determination of the deviation between the travel speed of the walking assist device and the walking speed of the user (step S1400). The control unit 40 proceeds to step S722 when called in step S700, and proceeds to step S822 when called in step S800.

In step S1438, the control unit 40 sets the travel speed of the walking assist device 10 to be "equal to the walking speed of the user", and finishes the determination of the deviation between the travel speed of the walking assist device and the walking speed of the user (step S1400). The control unit 40 proceeds to step S722 when called in step S700, and proceeds to step S822 when called in step S800.

In the case where the travel speeds (VdR and VdL) of the walking assist device 10 and the walking speed of the user are equal to each other, the magnitudes of the evaluation speed Vhfd in the forward direction and the evaluation speed Vhbd in the rearward direction are equal to each other if the magnitudes of the speeds of front-rear arm swing by the user are equal to each other. In the case where the travel speed of the walking assist device 10 is lower than the walking speed of the user, on the other hand, the magnitude of the evaluation speed Vhfd in the forward direction is larger than the magnitude of the evaluation speed Vhbd in the rearward direction because of the difference between the walking speed of the user and the travel speed of the walking assist device 10. In the case where the travel speed of the walking assist device 10 is higher than the walking speed of the user, the magnitude of the evaluation speed Vhfd in the forward direction is smaller than the magnitude of the evaluation speed Vhbd in the rearward direction because of the difference between the walking speed of the user and the travel speed of the walking assist device 10. The control unit 40 increases the travel speeds (VdR and VdL) of the walking assist device 10 in the case where the travel speeds (VdR and VdL) of the walking assist device 10 are lower than the walking speed of the user, and decreases the travel speeds of the walking assist device 10 in the case where the travel speeds of the walking assist device 10 are higher than the walking speed of the user. Consequently, travel of the walking assist device 10 of the user can be controlled adequately in accordance with the speed of front-rear arm swing by the user by correcting the deviation between the travel speed of the walking assist device 10 and the walking speed of the user.

In the case where the user does not swing his/her arms back and forth, e.g. in the case where the user walks while pushing or pulling the walking assist device 10 with his/her both hands as with a walker according to the related art, the travel speed of the walking assist device 10 is controlled such that the movable handles 20R and 20L are positioned at the middle in the front-rear direction on the rails 30R and 30L. Consequently, travel of the walking assist device 10 of the walker can be controlled adequately, even in the case where the user does not swing his/her arms back and forth, by correcting the deviation between the travel speed of the walking assist device 10 and the walking speed of the user.

FIG. 19 illustrates mode transition conditions for transitioning among the operation modes based on the body state, the atmospheric state, and the vehicle body state. FIG. 20 illustrates conditions for transitioning to the various operation modes in the case where the operation mode is automatically switched. In the case where the automatic mode switching unit switch 76b is on, the control unit 40 determines the operation mode in accordance with the conditions indicated in FIGS. 9, 19, and 20 in step S20 (determination of the operation mode based on each acquired state) in FIG. 10 based on information selected using the manual mode switching unit 76a.

In the case where one of conditions S1 to S6 is met, the control unit 40 changes the operation mode to an operation mode corresponding to the condition. In FIGS. 19 and 20, the symbol "-" indicates that the state may be either "0" or "1".

In FIG. 19, the mode transition conditions are determined based on the body state, the atmospheric state, and the vehicle body state. The control unit 40 determines the mode transition condition as "1 = without abnormality" only in the case where all the states are "1", and as "0 = with abnormality" in the case where any of the conditions is "0".

Examples of the body state include the heart rate and the body temperature of the user. The control unit 40 compares the heart rate and the body temperature that are acquired by the heart rate and body temperature sensors 27a and 27b with predetermined values stored in advance in the storage unit 44, and determines the body state as "abnormal = 0" in the case where such predetermined values are exceeded, and as "normal = 1" otherwise.

Examples of the atmospheric state include the outside temperature. The control unit 40 compares the outside temperature that is acquired by the outside temperature sensor 54 with a predetermined value stored in advance in the storage unit 44, and determines the atmospheric state as "uncomfortable = 0" in the case where such a predetermined value is exceeded, and as "comfortable = 1" otherwise.

Examples of the vehicle body state include the inclination of the vehicle body, an impact on the vehicle body (variations in the acceleration applied to the body), the walking distance, and the walking time. The control unit 40 compares information acquired by the three-axis acceleration and angular speed sensor 52 with a predetermined value stored in advance in the storage unit 44, and determines the inclination of the vehicle body as "yes = 0" in the case where the inclination of the vehicle body exceeds such a predetermined value, and as "no = 1" otherwise. The control unit 40 compares information acquired by the three-axis acceleration and angular speed sensor 52 with a predetermined condition stored in advance in the storage unit 44, and determines an impact on the vehicle body as "yes = 0" in the case where such a condition is met, and as "no = 1" otherwise.

The control unit 40 determines the walking distance as "long = 0" based on a history of the walking distance stored in the storage unit 44 in the case where the walking distance is longer than a predetermined distance, and as "short = 1" otherwise. The control unit 40 determines the walking time as "long = 0" based on a history of the walking time stored in the storage unit 44 in the case where the walking time is longer than a predetermined time, and as "short = 1" otherwise.

In FIG. 20, the control unit 40 switches between the assist mode 3 (AM3) and the training mode 3 (TR3), between the assist mode 2 (AM2) and the training mode 2 (TR2), or between the training mode 1 (TR1) and the assist mode 1 (AM1) in FIG. 8 based on the conditions S1 to S6.

The condition S1 and the condition S2 are conditions for switching determination of the operation mode between the training mode 1 (TR1) and the assist mode 1 (AM1). In the case where the manual mode switching unit 76a selects the "training mode 1", the movable handle grasping state is "1 = grasped", the arm swing state is "1 = with arm swing", the fixed handle grasping state is "0 = not grasped", and the mode transition condition is "1 = without abnormality", the condition S1 is met, and the control unit 40 causes the operation mode to transition from the assist mode 1 (AM1) to the training mode 1 (TR1). In the case where the manual mode switching unit 76a selects the "training mode 1", the movable handle grasping state is "1 = grasped", the arm swing state is "1 = with arm swing", the fixed handle grasping state is "0 = not grasped", and the mode transition condition is "0 = with abnormality", the condition S2 is met, and the control unit 40 causes the operation mode to transition from the training mode 1 (TR1) to the assist mode 1 (AM1).

The condition S3 and the condition S4 are conditions for switching determination of the operation mode between the assist mode 2 (AM2) and the training mode 2 (TR2). In the case where the manual mode switching unit 76a selects the "training mode 2", the movable handle grasping state is "1 = grasped", the arm swing state is "0 = without arm swing", the fixed handle grasping state is "0 = not grasped", and the mode transition condition is "1 = without abnormality", the condition S3 is met, and the control unit 40 causes the operation mode to transition from the assist mode 2 (AM2) to the training mode 2 (TR2). In the case where the manual mode switching unit 76a selects the "training mode 2", the movable handle grasping state is "1 = grasped", the arm swing state is "0 = without arm swing", the fixed handle grasping state is "0 = not grasped", and the mode transition condition is "0 = with abnormality", the condition S4 is met, and the control unit 40 causes the operation mode to transition from the training mode 2 (TR2) to the assist mode 2 (AM2).

The condition S5 and the condition S6 are conditions for switching determination of the operation mode between the assist mode 3 (AM3) and the training mode 3 (TR3). In the case where the manual mode switching unit 76a selects the "training mode 2", the movable handle grasping state is "0 = not grasped", the arm swing state is "0 = without arm swing", the fixed handle grasping state is "1 = grasped", and the mode transition condition is "1 = without abnormality", the condition S5 is met, and the control unit 40 causes the operation mode to transition from the assist mode 3 (AM3) to the training mode 3 (TR3). In the case where the manual mode switching unit 76a selects the "training mode 2", the movable handle grasping state is "0 = not grasped", the arm swing state is "0 = without arm swing", the fixed handle grasping state is "1 = grasped", and the mode transition condition is "0 = with abnormality", the condition S6 is met, and the control unit 40 causes the operation mode to transition from the training mode 3 (TR3) to the assist mode 3 (AM3).

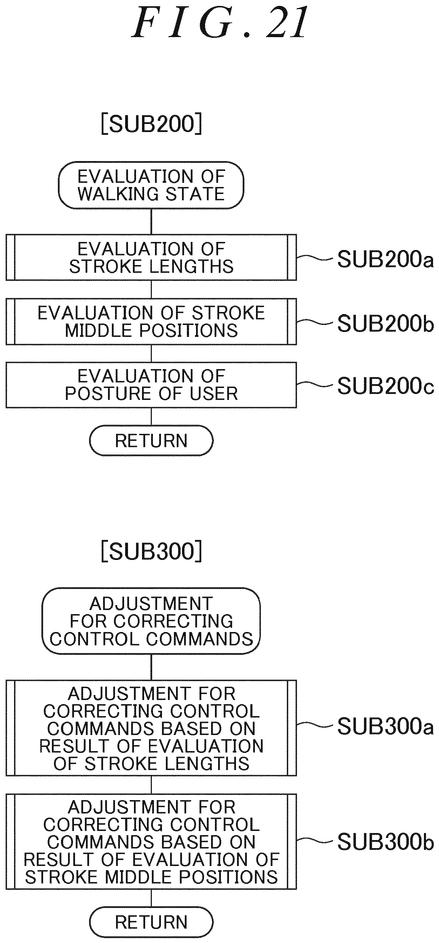

As illustrated in FIG. 21, the evaluation of a walking state (step SUB200) is constituted from evaluation of stroke lengths (step SUB200a), evaluation of stroke middle positions (step SUB200b), and evaluation of the posture of the user (step SUB200c).

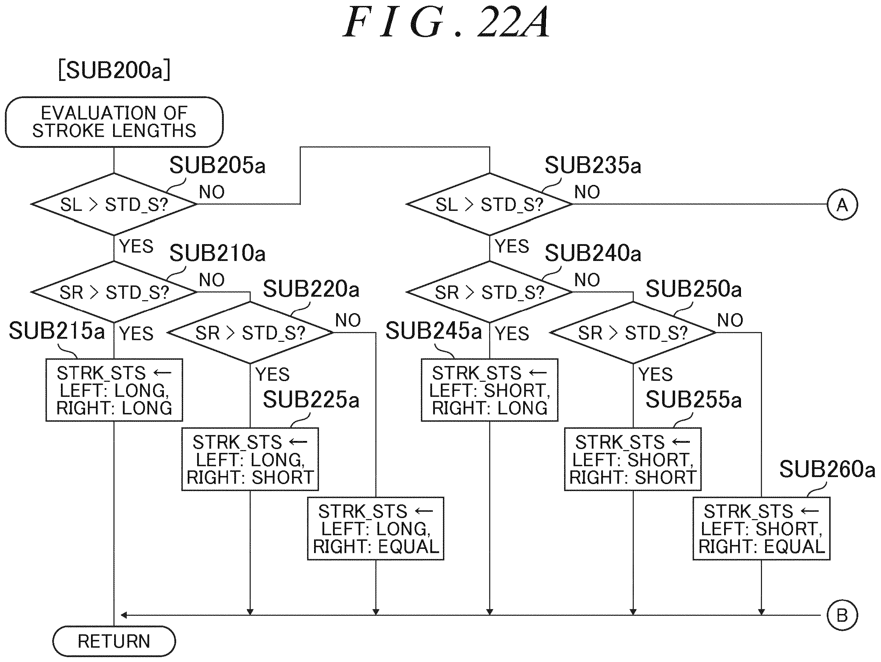

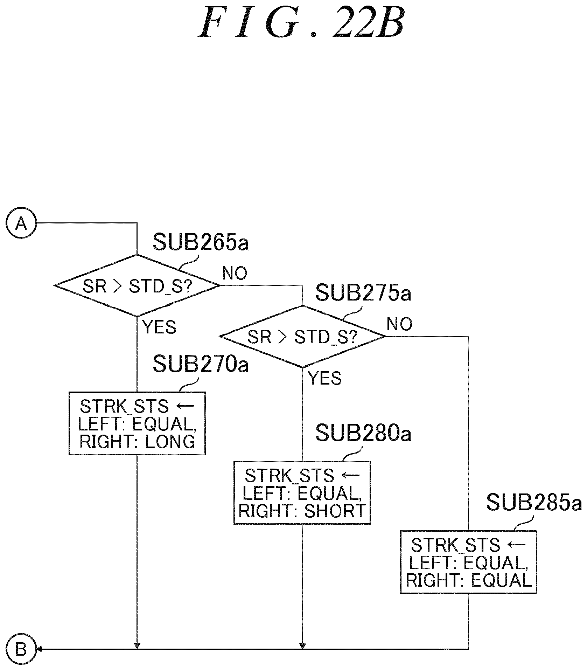

FIG. 22A and FIG. 22B are flowcharts illustrating the procedure of evaluation of stroke lengths. The walking state evaluation unit 40a2 (see FIG. 7) extracts reference stroke information corresponding to the user from the storage unit 44 that stores reference stroke information including a reference stroke length that matches user personal information including gender, age, and body information of the user, and calculates a target stroke length STD_S based on the reference stroke length that is included in the extracted reference stroke information. The target stroke length STD_S may be calculated by making an adjustment by increasing and decreasing the reference stroke length in consideration of the physical strength of the user etc., for example. The walking state evaluation unit 40a2 evaluates, for the calculated target stroke length STD_S, whether or not it is necessary to correct each of an average right stroke length SR and an average left stroke length SL. The average right stroke length SR and the average left stroke length SL are evaluated using the target stroke length STD_S until being calculated through averaging.

STRK_STS indicates the result of evaluation of stroke lengths by the walking state evaluation unit 40a2. "Right" indicates the right stroke length. "Left" indicates the left stroke length. "Long" indicates a case where the stroke length is evaluated as being longer than the target stroke length STD_S. "Short" indicates a case where the stroke length is evaluated as being shorter than the target stroke length STD_S. "Equal" indicates a case where the difference between the stroke length and the target stroke length STD_S is less than a predetermined value.

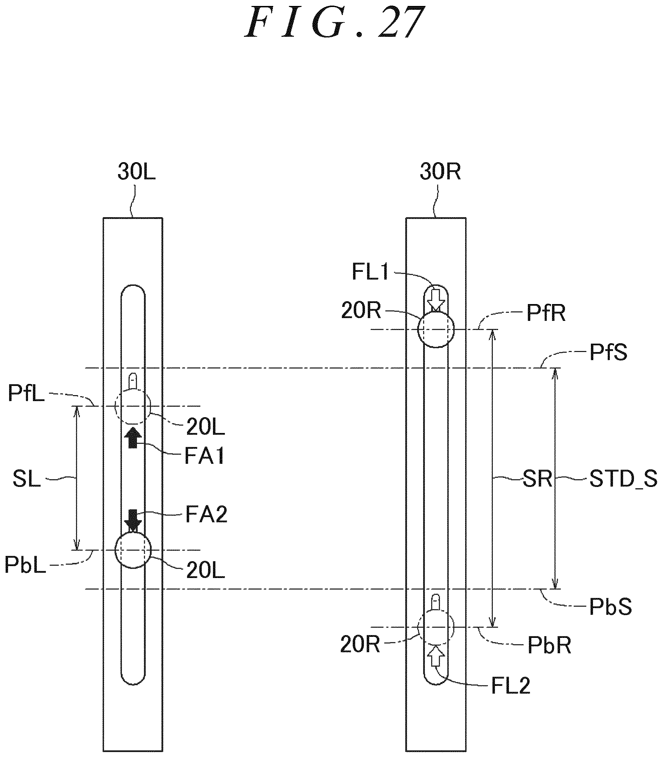

For example, in the case where the average left stroke length SL is evaluated as being shorter than the target stroke length STD_S and the average right stroke length SR is evaluated as being longer than the target stroke length STD_S, the walking state evaluation unit 40a2 determines that it is necessary to correct the stroke lengths (arm swing of the user), and sets STRK_STS to "left: short, right: long" (STRK_STS = left: short, right: long) (see FIG. 27).

In step SUB205a, the walking state evaluation unit 40a2 proceeds to step SUB210a in the case where the average left stroke length SL is evaluated as being longer than the target stroke length STD_S (SL > STD_S) (Yes), and proceeds to step SUB235a in the case where the average left stroke length SL is evaluated as not being longer than the target stroke length STD_S (No).

In step SUB210a, the walking state evaluation unit 40a2 proceeds to step SUB215a in the case where the average right stroke length SR is evaluated as being longer than the target stroke length STD_S (SR > STD_S) (Yes), and proceeds to step SUB220a in the case where the average right stroke length SR is evaluated as not being longer than the target stroke length STD_S (No).

In step SUB215a, the walking state evaluation unit 40a2 sets STRK_STS to "left: long, right: long" (STRK_STS = left: long, right: long), finishes step SUB200a, and returns to step SUB200.

In step SUB220a, the walking state evaluation unit 40a2 proceeds to step SUB225a in the case where the average right stroke length SR is evaluated as being shorter than the target stroke length STD_S (SR < STD_S) (Yes), and proceeds to step SUB230a in the case where the average right stroke length SR is evaluated as not being shorter than the target stroke length STD_S (No).

In step SUB225a, the walking state evaluation unit 40a2 sets STRK_STS to "left: long, right: short" (STRK_STS = left: long, right: short), finishes step SUB200a, and returns to step SUB200.

In step SUB230a, the walking state evaluation unit 40a2 sets STRK_STS to "left: long, right: equal" (STRK_STS = left: long, right: equal), finishes step SUB200a, and returns to step SUB200.

In step SUB235a, the walking state evaluation unit 40a2 proceeds to step SUB240a in the case where the average left stroke length SL is evaluated as being shorter than the target stroke length STD_S (SL < STD_S) (Yes), and proceeds to step SUB265a in the case where the average left stroke length SL is evaluated as not being shorter than the target stroke length STD_S (No).

In step SUB240a, the walking state evaluation unit 40a2 proceeds to step SUB245a in the case where the average right stroke length SR is evaluated as being longer than the target stroke length STD_S (SR > STD_S) (Yes), and proceeds to step SUB250a in the case where the average right stroke length SR is evaluated as not being longer than the target stroke length STD_S (No).

In step SUB245a, the walking state evaluation unit 40a2 sets STRK_STS to "left: short, right: long" (STRK_STS = left: short, right: long), finishes step SUB200a, and returns to step SUB200.

In step SUB250a, the walking state evaluation unit 40a2 proceeds to step SUB255a in the case where the average right stroke length SR is evaluated as being shorter than the target stroke length STD_S (SR < STD_S) (Yes), and proceeds to step SUB260a in the case where the average right stroke length SR is evaluated as not being shorter than the target stroke length STD_S (No).

In step SUB255a, the walking state evaluation unit 40a2 sets STRK_STS to "left: short, right: short" (STRK_STS = left: short, right: short), finishes step SUB200a, and returns to step SUB200.

In step SUB260a, the walking state evaluation unit 40a2 sets STRK_STS to "left: short, right: equal" (STRK_STS = left: short, right: equal), finishes step SUB200a, and returns to step SUB200.

In step SUB265a, the walking state evaluation unit 40a2 proceeds to step SUB270a in the case where the average right stroke length SR is evaluated as being longer than the target stroke length STD_S (SR > STD_S) (Yes), and proceeds to step SUB275a in the case where the average right stroke length SR is evaluated as not being longer than the target stroke length STD_S (No).

In step SUB270a, the walking state evaluation unit 40a2 sets STRK_STS to "left: equal, right: long" (STRK_STS = left: equal, right: long), finishes step SUB200a, and returns to step SUB200.

In step SUB275a, the walking state evaluation unit 40a2 proceeds to step SUB280a in the case where the average right stroke length SR is evaluated as being shorter than the target stroke length STD_S (SR < STD_S) (Yes), and proceeds to step SUB285a in the case where the average right stroke length SR is evaluated as not being shorter than the target stroke length STD_S (No).

In step SUB280a, the walking state evaluation unit 40a2 sets STRK_STS to "left: equal, right: short" (STRK_STS = left: equal, right: short), finishes step SUB200a, and returns to step SUB200.

In step SUB285a, the walking state evaluation unit 40a2 sets STRK_STS to "left: equal, right: equal" (STRK_STS = left: equal, right: equal), finishes step SUB200a, and returns to step SUB200.

The walking state evaluation unit 40a2 evaluates it being necessary to correct the stroke lengths (arm swing) of the user except for the case where STRK_STS is "left: equal, right: equal" (STRK_STS = "left: equal, right: equal"). The walking state evaluation unit 40a2 may evaluate whether or not the deviation between the right stroke length (average right stroke length SR) and the left stroke length (average left stroke length SL) is equal to or more than a predetermined length deviation, and evaluate it being necessary to correct at least one of the right stroke length and the left stroke length.

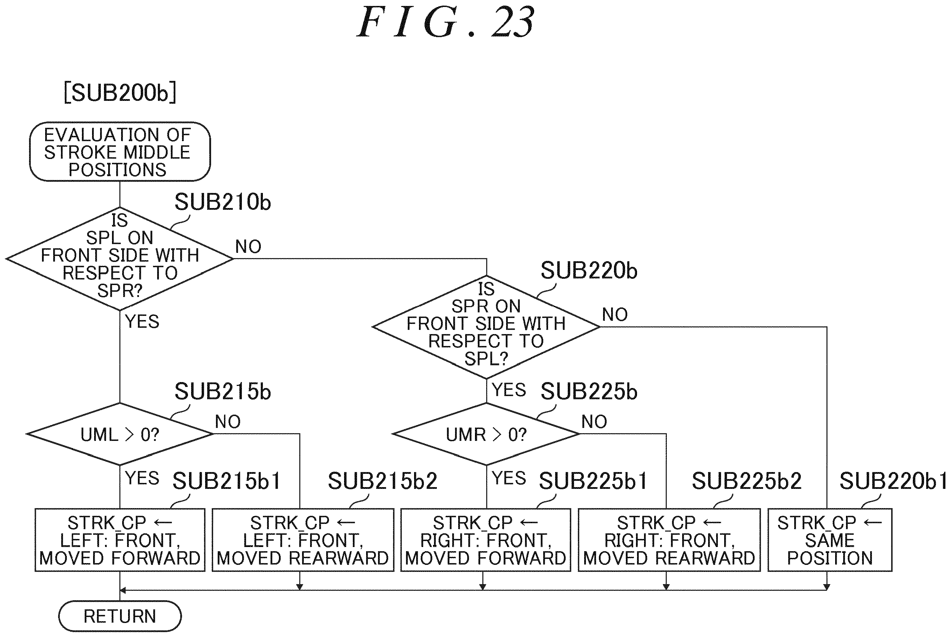

FIG. 23 is a flowchart illustrating the procedure of evaluation of stroke middle positions that are the middle positions, in the front-rear direction, of the stroke ranges of the movable handles (20R and 20L). An average right stroke middle position SPR and an average left stroke middle position SPL are evaluated using a reference stroke middle position STD_P set in advance until being calculated through averaging.

STRK_CP indicates the result of the walking state evaluation unit 40a2 evaluating the positional relationship between the right and left stroke middle positions in the front-rear direction of the rails (30R and 30L). "Left: front" indicates that the average left stroke middle position SPL is positioned on the front side with respect to the average right stroke middle position SPR. "Right: front" indicates that the average right stroke middle position SPR is positioned on the front side with respect to the average left stroke middle position SPL. "Moved forward" indicates that the movable handle (20R, 20L) is moved forward along the rail (30R, 30L). "Moved rearward" indicates that the movable handle (20R, 20L) is moved rearward along the rail (30R, 30L). "Same position" indicates a case where the difference between the average left stroke middle position SPL and the average right stroke middle position SPR is equal to or less than a predetermined value.

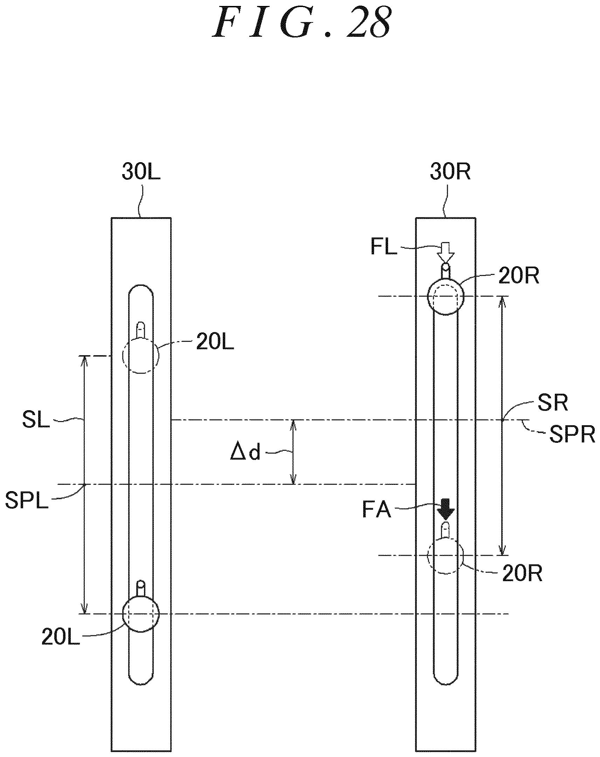

For example, in the case where the average right stroke middle position SPR is evaluated as being positioned on the front side with respect to the average left stroke middle position SPL and the movable handle 20R is evaluated as being moved rearward along the rail 30R, the walking state evaluation unit 40a2 evaluates STRK_CP as "STRK_CP = right: front, moved rearward" (see FIG. 28).

In step SUB210b, the walking state evaluation unit 40a2 proceeds to step SUB215b in the case where the average left stroke middle position SPL is evaluated as being located on the front side with respect to the average right stroke middle position SPR

(Yes), and proceeds to step SUB220b in the case where the average left stroke middle position SPL is evaluated as not being located on the front side with respect to the average right stroke middle position SPR (No).

In step SUB215b, the walking state evaluation unit 40a2 proceeds to step SUB215b1 in the case where a left grasp portion movement speed UML is evaluated as being "positive" (UML > 0) (Yes), and proceeds to step SUB215b2 in the case where the left grasp portion movement speed UML is evaluated as not being "positive" (UML > 0) (No).

In step SUB215b1, the walking state evaluation unit 40a2 sets STRK_CP to "left: front, moved forward" (STRK_CP = left: front, moved forward), finishes step SUB200b, and returns to step SUB200.

In step SUB215b2, the walking state evaluation unit 40a2 sets STRK_CP to "left: front, moved rearward" (STRK_CP = left: front, moved rearward), finishes step SUB200b, and returns to step SUB200.

In step SUB220b, the walking state evaluation unit 40a2 proceeds to step SUB225b in the case where the average right stroke middle position SPR is evaluated as being located on the front side with respect to the average left stroke middle position SPL (Yes), and proceeds to step SUB220b1 in the case where the average right stroke middle position SPR is evaluated as not being located on the front side with respect to the average left stroke middle position SPL (No).

In step SUB225b, the walking state evaluation unit 40a2 proceeds to step SUB225b1 in the case where a right grasp portion movement speed UMR is evaluated as being "positive" (UMR > 0) (Yes), and proceeds to step SUB225b2 in the case where the right grasp portion movement speed UMR is evaluated as not being "positive" (UMR > 0) (No).

In step SUB225b1, the walking state evaluation unit 40a2 sets STRK_CP to "right: front, moved forward" (STRK_CP = right: front, moved forward), finishes step SUB200b, and returns to step SUB200.

In step SUB225b2, the walking state evaluation unit 40a2 sets STRK_CP to "right: front, moved rearward" (STRK CP = right: front, moved rearward), finishes step SUB200b, and returns to step SUB200.

In step SUB220b1, the walking state evaluation unit 40a2 sets STRK_CP to "same position" (STRK CP = same position), finishes step SUB200b, and returns to step SUB200.

In step SUB200c, the walking state evaluation unit 40a2 evaluates the posture of the user from transitions in the results of observation of the right grasp portion inclination direction, the right grasp portion inclination angle, the left grasp portion inclination direction, and the left grasp portion inclination angle. For example, in the case where a state with (right grasp portion inclination direction = forward), (right grasp portion inclination angle ≥ large), (left grasp portion inclination direction = forward), and (left grasp portion inclination angle ≥ large) is observed for a predetermined period, the walking state evaluation unit 40a2 evaluates the user as walking in a hunched posture, which should be corrected.

As illustrated in FIG. 21, the adjustment for correcting control commands (step SUB300) is constituted from adjustment for correcting control commands based on the result of evaluation of stroke lengths (step SUB300a) and adjustment for correcting control commands based on the result of evaluation of stroke middle positions (step SUB300b). FIG. 27 illustrates a process for evaluation and correction of stroke lengths. FIG. 28 illustrates a process for evaluation and correction of stroke middle positions.

The correction adjustment unit 40a3 adjusts the target movement speeds (UR and UL), which are control commands for the grasp portion drive units (32R and 32L), based on the result of evaluation of stroke lengths.

In step SUB305a, the correction adjustment unit 40a3 proceeds to step SUB305a1 in the case where STRK_STS is determined as "STRK_STS = left: long, right: long" (Yes), and proceeds to step SUB315a in the case where STRK_STS is determined as not "STRK_STS = left: long, right: long" (No).

In step S305a1, the correction adjustment unit 40a3 adjusts the target movement speed UR to kL2×UR (UR = kL2×UR), adjusts the target movement speed UL to kL2×UL (UL = kL2×UL), finishes step SUB300a, and returns to step SUB300. The speed coefficient kL2 is a speed coefficient (< 1) for applying a load to movement of the movable handles 20R and 20L, and is a predetermined value stored in advance.

In step SUB315a, the correction adjustment unit 40a3 proceeds to step SUB315a1 in the case where STRK_STS is determined as "STRK_STS = left: long, right: short" (Yes), and proceeds to step SUB320a in the case where STRK_STS is determined as not "STRK_STS = left: long, right: short" (No).

In step S315a1, the correction adjustment unit 40a3 adjusts the target movement speed UR to kA2×UR (UR = kA2×UR), adjusts the target movement speed UL to kL2×UL (UL = kL2×UL), finishes step SUB300a, and returns to step SUB300. The speed coefficient kA2 is a speed coefficient (> 1) for providing assist to movement of the movable handles 20R and 20L, and is a predetermined value stored in advance.

In step SUB320a, the correction adjustment unit 40a3 proceeds to step SUB320a1 in the case where STRK_STS is determined as "STRK_STS = left: long, right: equal" (Yes), and proceeds to step SUB325a in the case where STRK_STS is determined as not "STRK_STS = left: long, right: equal" (No).

In step S320a1, the correction adjustment unit 40a3 adjusts the target movement speed UR to UR (UR = UR), adjusts the target movement speed UL to kL2×UL (UL = kL2×UL), finishes step SUB300a, and returns to step SUB300.

In step SUB325a, the correction adjustment unit 40a3 proceeds to step SUB325a1 in the case where STRK_STS is determined as "STRK_STS = left: short, right: long" (Yes), and proceeds to step SUB330a in the case where STRK_STS is determined as not "STRK_STS = left: short, right: long" (No).

In step S325a1, the correction adjustment unit 40a3 adjusts the target movement speed UR to kL2×UR (UR = kL2×UR), adjusts the target movement speed UL to kA2×UL (UL = kA2×UL), finishes step SUB300a, and returns to step SUB300.

In FIG. 27, a target stroke front end position PfS and a target stroke rear end position PbS are the right stroke front end position and the right stroke rear end position, respectively, which serve as targets calculated based on the target stroke length STD_S on the rails (30R and 30L). A state in which the user moves the movable handle 20R forward and moves the movable handle 20L rearward is represented by the continuous line, and a state in which the user moves the movable handle 20R rearward and moves the movable handle 20L forward is represented by the long dashed double-short dashed line. In the case where STRK_STS is determined as "STRK_STS = left: short, right: long", the average right stroke length SR is longer than the target stroke length STD_S, and the average left stroke length SL is shorter than the target stroke length STD_S. That is, as illustrated in FIG. 27, a right stroke front end position PfR is positioned on the front side with respect to the target stroke front end position PfS, and a right stroke rear end position PbR is positioned on the rear side with respect to the target stroke rear end position PbS. A left stroke front end position PfL is positioned on the rear side with respect to the target stroke front end position PfS, and a left stroke rear end position PbL is positioned on the front side with respect to the target stroke rear end position PbS. The correction adjustment unit 40a3 controls the grasp portion drive unit 32R so as to apply loads (FL1 and FL2) to the right movable handle 20R such that the right stroke front end position PfR is brought to the target stroke front end position PfS and the right stroke rear end position PbR is brought to the target stroke rear end position PbS. The correction adjustment unit 40a3 controls the grasp portion drive unit 32L so as to apply loads (FA1 and FA2) to the left movable handle 20L such that the left stroke front end position PfL is brought to the target stroke front end position PfS and the left stroke rear end position PbL is brought to the target stroke rear end position PbS. Consequently, the arm swing of the user is corrected with the target movement speeds (UR and UL) for the movable handles 20R and 20L adjusted such that the average right stroke length SR and the average left stroke length SL approximate the target stroke length STD_S (target stroke front end position PfS and target stroke rear end position PbS).

In step SUB330a, the correction adjustment unit 40a3 proceeds to step SUB330a1 in the case where STRK_STS is determined as "STRK_STS = left: short, right: short" (Yes), and proceeds to step SUB335a in the case where STRK_STS is determined as not "STRK_STS = left: short, right: short" (No).

In step S330a1, the correction adjustment unit 40a3 adjusts the target movement speed UR to kA2×UR (UR = kA2×UR), adjusts the target movement speed UL to kA2×UL (UL = kA2×UL), finishes step SUB300a, and returns to step SUB300.

In step SUB335a, the correction adjustment unit 40a3 proceeds to step SUB335a1 in the case where STRK_STS is determined as "STRK_STS = left: short, right: equal" (Yes), and proceeds to step SUB340a in the case where STRK_STS is determined as not "STRK_STS = left: short, right: equal" (No).

In step S335a1, the correction adjustment unit 40a3 adjusts the target movement speed UR to UR (UR = UR), adjusts the target movement speed UL to kA2×UL (UL = kA2×UL), finishes step SUB300a, and returns to step SUB300.

In step SUB340a, the correction adjustment unit 40a3 proceeds to step SUB340a1 in the case where STRK_STS is determined as "STRK_STS = left: equal, right: long" (Yes), and proceeds to step SUB345a in the case where STRK_STS is determined as not "STRK_STS = left: equal, right: long" (No).

In step S340a1, the correction adjustment unit 40a3 adjusts the target movement speed UR to kL2×UR (UR = kL2×UR), adjusts the target movement speed UL to UL (UL = UL), finishes step SUB300a, and returns to step SUB300.

In step SUB345a, the correction adjustment unit 40a3 proceeds to step SUB345a1 in the case where STRK_STS is determined as "STRK_STS = left: equal, right: short" (Yes), and proceeds to step SUB345a2 in the case where STRK_STS is determined as not "STRK_STS = left: equal, right: short" (No).

In step S345a1, the correction adjustment unit 40a3 adjusts the target movement speed UR to kA2×UR (UR = kA2×UR), adjusts the target movement speed UL to UL (UL = UL), finishes step SUB300a, and returns to step SUB300.

In step S345a2, the correction adjustment unit 40a3 adjusts the target movement speed UR to UR (UR = UR), adjusts the target movement speed UL to UL (UL = UL), finishes step SUB300a, and returns to step SUB300.

The correction adjustment unit 40a3 adjusts the target movement speeds (UR and UL), which are control commands for the grasp portion drive units (32R and 32L), based on the result of evaluation of stroke middle positions.

In step SUB310b, the correction adjustment unit 40a3 proceeds to step SUB310b1 in the case where STRK_CP is determined as "STRK_CP = left: front, moved forward" (Yes), and proceeds to step SUB315b in the case where STRK_CP is determined as not "STRK_CP = left: front, moved forward" (No).

In step S310b1, the correction adjustment unit 40a3 adjusts the target movement speed UR to UR (UR = UR), adjusts the target movement speed UL to kL3×UL (UL = kL3×UL), finishes step SUB300b, and returns to step SUB300. The speed coefficient kL3 is a speed coefficient (< 1) for applying a load to movement of the movable handles 20R and 20L, and is a predetermined value stored in advance.

In step SUB315b, the correction adjustment unit 40a3 proceeds to step SUB315b1 in the case where STRK_CP is determined as "STRK_CP = left: front, moved rearward" (Yes), and proceeds to step SUB320b in the case where STRK_CP is determined as not "STRK_CP = left: front, moved rearward" (No).

In step S315b1, the correction adjustment unit 40a3 adjusts the target movement speed UR to UR (UR = UR), adjusts the target movement speed UL to kA3×UL (UL = kA3×UL), finishes step SUB300b, and returns to step SUB300. The speed coefficient kA3 is a speed coefficient (> 1) for applying a load to movement of the movable handles 20R and 20L, and is a predetermined value stored in advance.

In step SUB320b, the correction adjustment unit 40a3 proceeds to step SUB320b1 in the case where STRK_CP is determined as "STRK_CP = right: front, moved forward" (Yes), and proceeds to step SUB325b in the case where STRK_CP is determined as not "STRK_CP = right: front, moved forward" (No).

In step S320b1, the correction adjustment unit 40a3 adjusts the target movement speed UR to kL3×UR (UR = kL3×UR), adjusts the target movement speed UL to UL (UL = UL), finishes step SUB300b, and returns to step SUB300.

In step SUB325b, the correction adjustment unit 40a3 proceeds to step SUB325b1 in the case where STRK_CP is determined as "STRK_CP = right: front, moved rearward" (Yes), and proceeds to step SUB325b2 in the case where STRK_CP is determined as not "STRK_CP = right: front, moved rearward" (No).

In step S325b1, the correction adjustment unit 40a3 adjusts the target movement speed UR to kA3×UR (UR = kA3×UR), adjusts the target movement speed UL to UL (UL = UL), finishes step SUB300b, and returns to step SUB300.

In step S325b2, the correction adjustment unit 40a3 adjusts the target movement speed UR to UR (UR = UR), adjusts the target movement speed UL to UL (UL = UL), finishes step SUB300b, and returns to step SUB300.

In FIG. 28, a state in which the user moves the movable handle 20R forward and moves the movable handle 20L rearward is represented by the continuous line, and a state in which the user moves the movable handle 20R rearward and moves the movable handle 20L forward is represented by the long dashed double-short dashed line. In the case where STRK_CP is determined as "STRK_CP = right: front, moved rearward", the average right stroke middle position SPR of the movable handle 20R is positioned a distance Δd on the front side with respect to the average left stroke middle position SPL of the movable handle 20L. The correction adjustment unit 40a3 controls the grasp portion drive unit 32R so as to apply the load FL to the right movable handle 20R (see FIG. 7). In the case where STRK_CP is determined as "STRK_CP = right: front, moved forward", the correction adjustment unit 40a3 controls the grasp portion drive unit 32R so as to apply assist FA to the right movable handle 20R (see FIG. 7). Consequently, the arm swing of the user is corrected with the target movement speeds (UR and UL) for the movable handles 20R and 20L adjusted such that the average right stroke middle position SPR approximates the average left stroke middle position SPL.

The stroke middle positions may be corrected by performing control so as to approximate the stroke middle position on the rear side to the stroke middle position on the front side, instead of performing control so as to approximate the stroke middle position on the front side to the stroke middle position on the rear side. Alternatively, the stroke middle positions may be corrected by performing control so as to approximate each of the average right stroke middle position SPR of the movable handle 20R and the average left stroke middle position SPL of the movable handle 20L to a predetermined position (e.g. an intermediate position), in the front-rear direction, between the stroke middle position on the front side and the stroke middle position on the rear side.

In step SUB300, the correction adjustment unit 40a3 (see FIG. 7) adjusts control commands for the grasp portion drive units 32R and 32L. However, control commands for the drive units 64R and 64L may be adjusted, or a control command for each of the drive units 64R and 64L for the grasp portion drive units 32R and 32L may be adjusted.

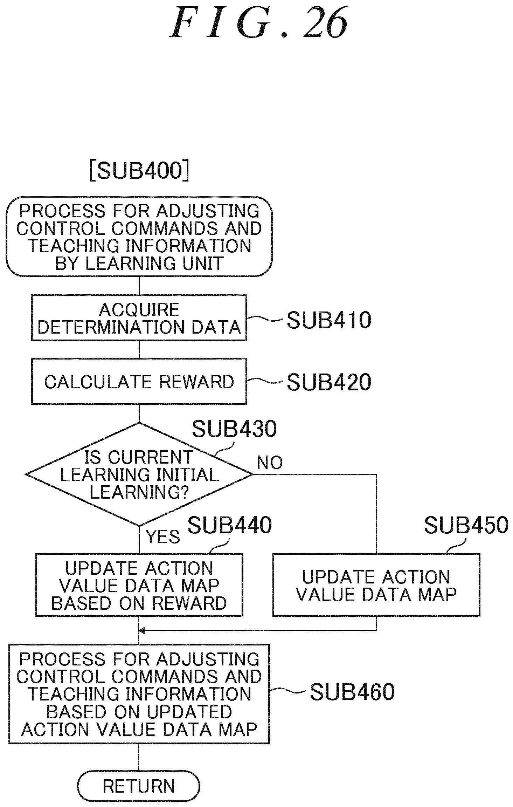

FIG. 26 is a flowchart illustrating the procedure of a process for adjusting control commands (target movement speeds (UR and UL)) and teaching information by the learning unit 40a6 (see FIG. 7).

The learning unit 40a6 performs learning, and is composed of a reward calculation section 40a6A, a function update section 40a6B, and an intention determination section 46a6C. The procedure of the learning will be described below.

The determination data acquisition unit 40a5 acquires, as the determination data, stroke lengths, stroke ranges, stroke middle positions, stroke speeds, etc. calculated based on data observed by the grasp portion state observation unit 40a1.

The learning unit 40a6 learns the control commands that are adjusted by the correction adjustment unit 40a3 and the teaching information that is extracted by the teaching information extraction unit 40a4 in accordance with a training data set constituted of a combination of the state variable that is observed by the grasp portion state observation unit 40a1 and the determination data.

The reward calculation section 40a6A calculates a reward based on the determination data.

The function update section 40a6B updates a function for estimating an appropriate set of the control commands and the teaching information for reducing a deviation and fluctuations from the current state variable based on the reward.

The function update section 40a6B preferably performs reinforcement learning using so-called Q-learning. The Q-learning is a method in which the learning unit 40a6 learns a value (action value) Q(s, a) of selecting an action a (control commands and teaching information) in a certain state s (walking state of the user in the present embodiment).

The intention determination section 40a6C selects the action a with the highest value Q(s, a) in the state s as an optimum action (control commands and teaching information) based on the result of the learning.

The function update section 40a6B updates a function (action value function Q(st, at)) using the following (formula 1). $Q \left(st, at\right) ← Q \left(st, at\right) + α \left(r\left(t+1\right)+γmaxQ\left(s\left(t+1\right),a\right)-Q\left(st, at\right)\right)$ (formula 1) Q(st, at) is an action value function, st is a state at time t, at is an action at time t, α is a learning coefficient, r is a reward, and γ is a reduction rate. The action value function Q means an expected value of the reward. The term with max is obtained by multiplying the value Q of the action a that is the highest in the state s(t + 1) by γ.

It is known that the learning coefficient α and the reduction rate γ are set as 0 < α and γ ≤ 1. When the learning coefficient and the reduction rate are defined as 1 for convenience, however, the function is represented by the following (formula 2). $Q \left(st, at\right) ← r \left(t+1\right) + γ maxQ \left(s\left(t+1\right),a\right)$ (formula 2)

This update (formula 2) indicates that Q(st, at) is increased if the value Q(s(t + 1), max(a(t + 1))) of the best action in a state that results from the action a is larger than the value Q(st, at) of the action a in the state s, and that Q(st, at) is reduced if the value Q(s(t + 1), max(a(t + 1))) is smaller than the value Q(st, at) conversely. That is, the update approximates the value of a certain action in a certain state to the value of the best action in a state that results from such an action. The state that is used in this update (formula 2) corresponds to a state variable that can be acquired from the training data set. The reward is acquired from the reward calculation section 40a6A. The action value Q(st, at) is considered to be stored as a map for each state s and action a (hereinafter "action value map"), for example.

The learning unit 40a6 updates, based on the update (formula 2) and the reward, action values in the action value map corresponding to the current state variable and actions that may be taken.

Step SUB400 (process for adjusting control commands and teaching information by the learning unit) will be described in detail below.

In step SUB410, the determination data acquisition unit 40a5 acquires determination data, and proceeds to step SUB420.

In step SUB420, the reward calculation section 40a6A calculates a reward based on the determination data, and proceeds to step SUB430. The reward is calculated by calculating a deviation between the reference walking state and the target walking state and fluctuations based on the observed determination data, and calculated in accordance with variations in the deviation or the fluctuations, for example. For example, the reward may be calculated as 0 (reference reward) in the case where neither of the deviation and the fluctuations is increased or decreased. The reward may be calculated by adding a predetermined positive value set in advance in the case where one of the deviation and the fluctuations is decreased, and calculated by adding a predetermined positive value that is larger than the above predetermined positive value in the case where both of the deviation and the fluctuations are decreased.

In step SUB430, the learning unit 40a6 proceeds to step SUB450 in the case where it is determined that the current learning is the initial learning (Yes), and proceeds to step SUB440 in the case where it is determined that the current learning is not the initial learning (No).

In step SUB440, the function update section 40a6B updates the action value data map based on the reward using the update (formula 2), and proceeds to step SUB460.

In step SUB450, the function update section 40a6B updates the action value data map, and proceeds to step SUB460.

In step SUB460, the intention determination section 40a6C selects the action a with the highest action value Q(s, a) as an optimum action (control commands and teaching information), finishes a process for adjusting control commands and teaching information by the learning unit 40a6 (step SUB400), and returns to the overall process.

In step SUB420, the reward calculation section 40a6A calculates a reward based on the deviation and the fluctuations. However, a reward may be calculated based on one of the deviation and the fluctuations.

As has been described above, the walking assist device according to the present invention can appropriately correct the walking state of the user.

The walking assist device according to the present invention is not limited to the configuration, the structure, the shape, the process procedure, etc. described in relation to the present embodiment, and may be modified, added, and deleted in various ways without departing from the scope and spirit of the present invention.

In the present embodiment, the walking assist device is a four-wheeled vehicle with two drive wheels. However, the walking assist device may be a three-wheeled vehicle in which two, right and left, wheels serve as drive wheels and the remaining wheel serves as a caster wheel. The present invention is also applicable to a walking cart that assists a user in walking on his/her own, a cart that assists elderly people in walking and that can carry baggage, and a hand cart. In addition, the walking assist device described in relation to the present embodiment includes rails and handles, and the handles are moved in the front-rear direction along the rails. However, handles may be provided at the respective distal ends of pole-like members provided swingably to project from rotary shafts provided on the frame, instead of the rails, and the handles may be swung in the front-rear direction with respect to the frame.

In the present embodiment, the evaluation speeds are calculated through integration. However, the evaluation speeds may be calculated by a different method. The order of the processes in the flowcharts is not limited to the order described in relation to the present embodiment, and is changeable in such a range that the functions and the effects can be maintained.

The grasp portion drive units 32R and 32L that move the movable handles 20R and 20L, respectively, in the front-rear direction with respect to the frame 50 are not limited to the configuration with an electric motor, pulleys, and a wire described in relation to the present embodiment. The movable handles 20R and 20L can be moved in the front-rear direction with respect to the frame 50 using a variety of configurations (see FIG. 2). The configuration, arrangement, etc. of the grasp portion position detection units 34R and 34L (see FIG. 2) that detect the respective positions of the movable handles 20R and 20L are not limited to the configuration, arrangement, etc. described in relation to the present embodiment, and a variety of configurations and arrangements may be used. The configuration, arrangement, etc. of the grasp portion inclination detection units 33R and 33L that detect the respective inclination directions and inclination angles of the movable handles 20R and 20L are not limited to the configuration, arrangement, etc. described in relation to the present embodiment, and a variety of configurations and arrangements may be used (see FIG. 3). The configuration, arrangement, etc. of the grasp portion pressure detection units 25R and 25L that detect respective forces applied to the movable handles 20R and 20L and the grasp portion pressure detection units 25FR and 25FL that detect respective forces applied to the fixed handles 20FR and 20FL are not limited to the configuration, arrangement, etc. described in relation to the present embodiment, and a variety of configurations and arrangements may be used (see FIGS. 3 and 6). An urging unit (such as a spring) is provided for each of the movable handles 20R and 20L see FIGS. 3 and 4. However, the urging unit is not limited to a spring etc., and a variety of elastic members may also be applied.

The object to be observed by the grasp portion state observation unit 40a1 (see FIG. 7) is not limited to the grasp portion state as in the present embodiment, and may be modified, added, and deleted in various ways without departing from the scope and spirit of the present invention. For the walking state evaluation unit 40a2 (see FIG. 7), an image acquisition unit may be provided to the frame 50 (see FIG. 1) to acquire the walking state of the user as an image, and the walking state of the user may be evaluated based on the image.

In the present embodiment, the learning unit 40a6 learns the control commands that are adjusted by the correction adjustment unit 40a3 and the teaching information that is extracted by the teaching information extraction unit 40a4. However, either of the control commands or the teaching information may be learned (see FIG. 7). The function update section 40a6B updates a function for estimating an appropriate set of the control commands and the teaching information for reducing a deviation and fluctuations from the current state variable based on the reward. However, the function update section 40a6B may update a function for estimating an appropriate set of the control commands and the teaching information for reducing either of a deviation or fluctuations (see FIG. 7).

A walking assist device includes: a frame 50; a pair of right and left arm portions 30R and 30L; a pair of right and left grasp portions 20R and 20L provided on the pair of right and left arm portions; a plurality of wheels including a drive wheel 60RL, 60RR; a drive unit 64R, 64L driving the drive wheel; a grasp portion drive unit configured to move the grasp portions; a battery B; a control unit 40 controlling the drive unit; and a grasp portion state detection unit detecting a state of each of the grasp portions. The control unit includes: a grasp portion state observation unit observing a grasp portion state that is a state of the grasp portions; a walking state evaluation unit evaluating a walking state of the user based on the grasp portion state observed using the grasp portion state observation unit; and a correction adjustment unit adjusting a control command for at least one of the drive unit and the grasp portion drive unit based on the walking state evaluated using the walking state evaluation unit.

## Claims

1. A walking assist device (10) comprising:
a frame (50);
a pair of right and left arm portions (30R, 30L) provided on the frame (50);
a pair of right and left grasp portions (20R, 20L) provided on the pair of right and left arm portions (30R, 30L), the grasp portions (20R, 20L) being graspable by a user and movable in a front-rear direction with respect to the frame (50);
a plurality of wheels (60FL, 60FR, 60RR, 60RL) provided at a lower end of the frame (50) and including a drive wheel (60RR, 60RL);
a drive unit (64R, 64L) that drives the drive wheel (60RR, 60RL) to cause the walking assist device (10) to travel forward or rearward;
a grasp portion drive unit (32R, 32L) that is configured to move each of the grasp portions (20R, 20L) in the front-rear direction with respect to the frame (50);
a battery (B) that serves as a power source for the drive unit (64R, 64L) and the grasp portion drive unit (32R, 32L);
a control unit (40) that controls the drive unit (64R, 64L); and
a grasp portion state detection unit (81) that detects a state of each of the grasp portions (20R, 20L), wherein
the control unit (40) includes:
a grasp portion state observation unit (40a1) that observes a grasp portion state, which is a state of each of the grasp portions (20R, 20L), based on a detection signal from the grasp portion state detection unit (81);
a walking state evaluation unit (40a2) that evaluates a walking state of the user based on the grasp portion state observed using the grasp portion state observation unit (40a1); and
a correction adjustment unit (40a3) that adjusts a control command for at least one of the drive unit (64R, 64L) and the grasp portion drive unit (32R, 32L) based on the walking state evaluated using the walking state evaluation unit (40a2).

2. The walking assist device (10) according to claim 1, wherein:
the walking state evaluation unit (40a2) estimates an arm swing state of the user based on the observed grasp portion state; and
the walking state evaluation unit (40a2) estimates the walking state based on the estimated arm swing state of the user.

3. The walking assist device (10) according to claim 2, wherein:
the grasp portion state observation unit (40a1) observes the grasp portion state during a predetermined observation period; and
the walking state evaluation unit (40a2) evaluates the walking state based on the grasp portion state observed during the predetermined observation period.

4. The walking assist device (10) according to claim 2 or 3, wherein
the walking state evaluation unit (40a2) evaluates the walking state based on the grasp portion state during a period excluding:
a predetermined post-start period that is a predetermined period immediately after the user starts walking using the walking assist device (10);
a predetermined pre-end period that is a predetermined period immediately before the user finishes walking using the walking assist device (10); and
a predetermined turn period that is a predetermined period around a right turn or a left turn made by the user using the walking assist device (10).

5. The walking assist device (10) according to any one of claims 1 to 4, wherein:
the grasp portion state includes a right stroke length that is a front-rear stroke length of the right grasp portion with respect to the frame (50) and a left stroke length that is a front-rear stroke length of the left grasp portion with respect to the frame (50);
the walking state evaluation unit (40a2) evaluates it being necessary to make a correction of at least one of the right stroke length and the left stroke length in the case where a deviation between the right stroke length and the left stroke length is equal to or more than a predetermined length deviation; and
the correction adjustment unit (40a3) adjusts the control command for the grasp portion drive unit (32R, 32L) such that the right stroke length and the left stroke length are equal to each other in the case where the walking state evaluation unit (40a2) evaluates it being necessary to make the correction.

6. The walking assist device (10) according to any one of claims 1 to 5, wherein:
the grasp portion state includes a right stroke range that is a front-rear stroke range of the right grasp portion in a position in the front-rear direction with respect to the frame (50), a left stroke range that is a front-rear stroke range of the left grasp portion in a position in the front-rear direction with respect to the frame (50), a right stroke middle position that is a middle position, in the front-rear direction, of the right stroke range, and a left stroke middle position that is a middle position, in the front-rear direction, of the left stroke range;
the walking state evaluation unit (40a2) evaluates it being necessary to make a correction of at least one of the right stroke middle position and the left stroke middle position in the case where a distance, in the front-rear direction, between the right stroke middle position and the left stroke middle position is equal to or more than a predetermined distance deviation; and
the correction adjustment unit (40a3) adjusts the control command for the grasp portion drive unit (32R, 32L) such that the right stroke middle position and the left stroke middle position are the same position in the front-rear direction in the case where the walking state evaluation unit (40a2) evaluates it being necessary to make the correction.

7. The walking assist device (10) according to claim 5, wherein:
the walking state evaluation unit (40a2) extracts, from a storage unit (44) that stores reference stroke information including a reference stroke length that matches user personal information including gender, age, and body information of the user, the reference stroke information corresponding to the user;
the walking state evaluation unit (40a2) calculates a target stroke length based on the reference stroke length included in the extracted reference stroke information;
the walking state evaluation unit (40a2) evaluates, for the calculated target stroke length, whether or not it is necessary to make a correction of each of the right stroke length and the left stroke length; and
in the case where the walking state evaluation unit (40a2) evaluates it being necessary to make the correction, the correction adjustment unit (40a3) adjusts the control command for the grasp portion drive unit (32R, 32L) such that the stroke length, for which the walking state evaluation unit (40a2) evaluates it being necessary to make the correction, is brought to the target stroke length.

8. The walking assist device (10) according to any one of claims 1 to 7, further comprising:
a teaching information extraction unit (40a4) that extracts teaching information including teachings about a correction of the walking state of the user; and
a teaching information output unit (78) that outputs at least one of a sound and an image based on the teaching information, wherein:
the walking state evaluation unit (40a2) estimates a posture of the user during walk using the walking assist device (10) based on the observed grasp portion state, and evaluates the walking state including the estimated posture of the user;
the teaching information extraction unit (40a4) extracts, from a storage unit (44) that stores teaching information including teachings about a correction of the walking state of the user, the teaching information based on the walking state evaluated by the walking state evaluation unit (40a2); and
the teaching information output unit (78) outputs at least one of a sound and an image based on the extracted teaching information.

9. The walking assist device (10) according to claim 8, further comprising:
a determination data acquisition unit (40a5); and
a learning unit (40a6), wherein:
the grasp portion state observation unit (40a1) observes, as a state variable, at least one of positions, in the front-rear direction, of the grasp portions (20R, 20L) with respect to the frame (50), inclination directions and inclination angles of the grasp portions (20R, 20L) with respect to the frame (50), and pressures applied to the grasp portions (20R, 20L);
the determination data acquisition unit (40a5) acquires determination data for determining a deviation between a target walking state, which is based on a reference walking state that is a walking state serving as a reference for the user, and an actual walking state of the user and fluctuations in the actual walking state of the user; and
the learning unit (40a6) learns, in accordance with a training data set constituted of a combination of the state variable and the determination data, at least one of the control command adjusted by the correction adjustment unit (40a3) and the teaching information extracted by the teaching information extraction unit (40a4).

10. The walking assist device (10) according to claim 9, wherein
the state variable includes at least one of:
grasp portion position data that indicate positions of the grasp portions (20R, 20L) with respect to the frame (50), and that are detected by a grasp portion position detection unit (34R, 34L);
grasp portion inclination data that indicate inclination directions and inclination angles of the grasp portions (20R, 20L) with respect to the frame (50), and that are detected by a grasp portion inclination detection unit (33R, 33L) provided to the grasp portions (20R, 20L); and
grasp portion pressure data that indicate a pressure applied from the user to the grasp portions (20R, 20L) as the user grasps the grasp portions (20R, 20L), and that are detected by a grasp portion pressure detection unit (25R, 25L) provided to the grasp portions (20R, 20L).

11. The walking assist device (10) according to claim 9 or 10, wherein:
the learning unit (40a6) learns the control command and the teaching information in accordance with the training data set; and
the learning unit (40a6) has
a reward calculation section (40a6A) that calculates a reward based on the determination data, and
a function update section (40a6B) that updates a function for estimating an appropriate set of the control command and the teaching information for reducing at least one of the deviation and the fluctuations from a current state variable based on the reward.

12. The walking assist device (10) according to claim 11, wherein
the learning unit (40a6) updates an action value data map corresponding to the set of the control command and the teaching information based on the state variable and the reward.

13. The walking assist device (10) according to claim 11 or 12, wherein
the learning unit (40a6) further includes an intention determination section (46a6C) that determines, based on a result of the learning unit (40a6) performing learning in accordance with the training data set, a command for the set of the control command and the teaching information.

14. The walking assist device (10) according to any one of claims 9 to 13, wherein
the learning unit (40a6) is connected to the control unit (40) via a network.

## Patentansprüche

1. Gehhilfevorrichtung (10) mit:
einem Rahmen (50);
einem Paar von rechten und linken Armbereichen (30R, 30L), die an dem Rahmen (50) bereitgestellt sind;
einem Paar von rechten und linken Griffbereichen (20R, 20L), die an dem Paar von rechten und linken Armbereichen (30R, 30L) bereitgestellt sind, wobei die Griffbereiche (20R, 20L) von einem Benutzer greifbar sind und in einer Vorwärts-Rückwärtsrichtung mit Bezug zu dem Rahmen (50) bewegbar sind;
einer Vielzahl von Rädern (60FL, 60FR, 60RR, 60RL), die an einem unteren Ende des Rahmens (50) bereitgestellt sind und die ein Antriebsrad (60RR, 60RL) umfasst;
einer Antriebseinheit (64R, 64L), die das Antriebsrad (60RR, 60RL) antreibt, um zu bewirken, dass die Gehhilfevorrichtung (10) Vorwärts oder Rückwärts fährt;
einer Griffbereichsantriebseinheit (32R, 32L), die eingerichtet ist, jeden der Griffbereiche (20R, 20L) in die Vorwärts-Rückwärtsrichtung mit Bezug zu dem Rahmen (50) zu bewegen;
einer Batterie (B), die als eine Energiequelle für die Antriebseinheit (64R, 64L) und die Griffbereichsantriebseinheit (32R, 32L) dient;
einer Steuerungseinheit (40), die die Antriebseinheit (64R, 64L) steuert; und
einer Griffbereichszustandserfassungseinheit (81), die einen Zustand jedes Griffbereichs (20R, 20L) erfasst, wobei
die Steuerungseinheit (40) umfasst:
eine Griffbereichszustandsbeobachtungseinheit (40a1), die einen Griffbereichszustand basierend auf einem Erfassungssignal von der Griffbereichszustandserfassungseinheit (81) beobachtet, der ein Zustand jedes Griffbereichs (20R, 20L) ist;
eine Gehzustandsbewertungseinheit (40a2), die einen Gehzustand des Benutzers basierend auf dem unter Verwendung der Griffbereichszustandsbeobachtungseinheit (40a1) beobachteten Griffbereichszustand bewertet; und
eine Korrekturanpassungseinheit (40a3), die eine Steuerungsanweisung für zumindest eine von der Antriebseinheit (64R, 64L) und der Griffbereichsantriebseinheit (32R, 32L) basierend auf dem unter Verwendung der Gehzustandsbewertungseinheit (40a2) bewerteten Gehzustand anpasst.

2. Gehhilfevorrichtung (10) nach Anspruch 1, wobei:
die Gehzustandsbewertungseinheit (40a2) einen Armschwingzustand des Benutzers basierend auf dem beobachteten Griffbereichszustand schätzt; und
die Gehzustandsbewertungseinheit (40a2) den Gehzustand basierend auf dem geschätzten Armschwingzustand des Benutzers schätzt.

3. Gehhilfevorrichtung (10) nach Anspruch 2, wobei:
die Griffbereichszustandsbeobachtungseinheit (40a1) den Griffbereichszustand während einer vorbestimmten Beobachtungszeitspanne beobachtet; und
die Gehzustandsbewertungseinheit (40a2) den Gehzustand basierend auf dem während der vorbestimmten Beobachtungszeitspanne beobachteten Griffbereichszustand bewertet.

4. Gehhilfevorrichtung (10) nach Anspruch 2 oder 3, wobei
die Gehzustandsbewertungseinheit (40a2) den Gehzustand basierend auf dem Griffbereichszustand während einer Zeitspanne mit Ausnahme von:
einer vorbestimmten Nach-Startzeitspanne, die eine vorbestimmte Zeitspanne unmittelbar nach dem Beginnen des Gehens des Benutzers unter Verwendung der Gehhilfevorrichtung (10) ist;
einer vorbestimmten Vor-Endzeitspanne, die eine vorbestimmte Zeitspanne unmittelbar bevor der Benutzer das Gehen unter Verwendung der Gehhilfevorrichtung (10) beendet ist; und
einer vorbestimmten Wendezeitspanne, die eine vorbestimmte Zeitspanne um eine Rechtsdrehung oder eine Linksdrehung ist, die der Benutzer unter Verwendung der Gehhilfevorrichtung (10) durchgeführt hat, bewertet.

5. Gehhilfevorrichtung (10) nach einem der Ansprüche 1 bis 4, wobei:
der Griffbereichszustand eine rechte Hublänge, die eine Vorwärts-Rückwärtshublänge des rechten Griffbereichs mit Bezug zu dem Rahmen (50) ist, und eine linke Hublänge, die eine Vorwärts-Rückwärtshublänge des linken Griffbereichs mit Bezug zu dem Rahmen (50) ist, umfasst;
die Gehzustandsbewertungseinheit (40a2) es als notwendig bewertet, eine Korrektur von zumindest einer von der rechten Hublänge und der linken Hublänge in dem Fall durchzuführen, in dem eine Abweichung zwischen der rechten Hublänge und der linken Hublänge gleich oder größer einer vorbestimmten Längenabweichung ist; und
die Korrekturanpassungseinheit (40a3) die Steuerungsanweisung für die Griffbereichsantriebeinheit (32R, 32L) so anpasst, dass die rechte Hublänge und die linke Hublänge gleich sind, in dem Fall, in dem die Gehzustandsbewertungseinheit (40a2) es als notwendig bewertet, eine Korrektur durchzuführen.

6. Gehhilfevorrichtung (10) nach einem der Ansprüche 1 bis 5, wobei:
der Griffbereichszustand einen rechten Hubbereich, der ein Vorwärts-Rückwärtshubbereich des rechten Griffbereichs in einer Position in der Vorwärts-Rückwärtsrichtung mit Bezug zu dem Rahmen (50) ist, einen linken Hubbereich, der ein Vorwärts-Rückwärtshubbereich des linken Griffbereichs in einer Position in der Vorwärts-Rückwärtsrichtung mit Bezug zu dem Rahmen (50) ist, eine mittlere Position des rechten Hubs, die eine mittlere Position in der Vorwärts-Rückwärtsrichtung des rechten Hubbereichs ist, und eine mittlere Position des linken Hubbereichs, die eine mittlere Position in der Vorwärts-Rückwärtsrichtung des linken Hubbereichs ist, umfasst;
die Gehzustandsbewertungseinheit (40a2) es als notwendig bewertet, eine Korrektur von zumindest einer von der mittleren Position des rechten Hubs und der mittleren Position des linken Hubs in dem Fall durchzuführen, in dem eine Distanz in der Vorwärts-Rückwärtsrichtung zwischen der mittleren Position des rechten Hubs und der mittleren Position des linken Hubs gleich oder größer einer vorbestimmten Distanzabweichung ist; und
die Korrekturanpassungseinheit (40a3) die Steuerungsanweisung für die Griffbereichsantriebseinheit (32R, 32L) so steuert, dass die mittlere Position des rechten Hubs und die mittlere Position des linken Hubs an der gleichen Position in der Vorwärts-Rückwärtsrichtung sind, in dem Fall, in dem die Gehzustandsbewertungseinheit (40a2) es als notwendig bewertet, die Korrektur durchzuführen.

7. Gehhilfevorrichtung (10) nach Anspruch 5, wobei:
die Gehzustandsbewertungseinheit (40a2) von einer Speichereinheit (44), die Referenzhubinformationen speichert, die eine Referenzhublänge umfassen, die mit den persönlichen Benutzerinformationen umfassend Geschlecht, Alter und Körperinformationen des Benutzers übereinstimmen, die Referenzhubinformationen entsprechend dem Benutzer extrahiert;
die Gehzustandsbewertungseinheit (40a2) eine Sollhublänge basierend auf der in den extrahierten Referenzhubinformationen umfassten Referenzhublänge berechnet;
die Gehzustandsbewertungseinheit (40a2) für die berechnete Sollhublänge bewertet, ob es notwendig ist, eine Korrektur von jeder von der rechten Hublänge und der linken Hublänge durchzuführen oder nicht; und
in dem Fall, in dem die Gehzustandsbewertungseinheit (40a2) es als notwendig bewertet, die Korrektur durchzuführen, passt die Korrekturanpassungseinheit (40a3) die Steuerungsanweisung für die Griffbereichsantriebseinheit (32R, 32L) so an, dass die Hublänge für die die Gehzustandsbewertungseinheit (40a2) es als notwendig bewertet, die Korrektur durchzuführen, in die Sollhublänge gebracht wird.

8. Gehhilfevorrichtung (10) nach einem der Ansprüche 1 bis 7, umfasst der Weiteren:
eine Lehrinformationsextrahierungseinheit (40a4), die Lehrinformationen umfassend Lehren über eine Korrektur des Gehzustands des Benutzers extrahiert; und
eine Lehrinformationsausgabeeinheit (78), die zumindest eine von einem Ton und einem Bild basierend auf den Lehrinformationen ausgibt, wobei:
die Gehzustandsbewertungseinheit (40a2) eine Haltung des Benutzers während des Gehens unter Verwendung der Gehhilfevorrichtung (10) basierend auf dem beobachteten Griffbereichszustand schätzt und den Gehzustand, der die geschätzte Haltung des Benutzers umfasst, bewertet;
die Lehrinformationsextrahierungseinheit (40a4) von einer Speichereinheit (44), die die Lehrinformationen umfassend Lehren über eine Korrektur des Gehzustands des Benutzers speichert, die Lehrinformation basierend auf dem durch die Gehzustandsbewertungseinheit (40a2) bewerteten Gehzustand extrahiert; und
die Lehrinformationsausgabeeinheit (78) zumindest eine von einem Ton und einem Bild basierend auf den extrahierten Lehrinformationen ausgibt.

9. Gehhilfevorrichtung (10) nach Anspruch 8, umfasst des Weiteren:
eine Bestimmungsdatenbeschaffungseinheit (40a5); und
eine Lerneinheit (40a6), wobei:
die Griffbereichszustandsbeobachtungseinheit (40a1) als eine Zustandsvariable zumindest eine von den Positionen in der Vorwärts-Rückwärtsrichtung der Griffbereiche (20R, 20L) mit Bezug zu dem Rahmen (50), Neigungsrichtungen und Neigungswinkel der Griffbereiche (20R, 20L) mit Bezug zu dem Rahmen (50) und dem an die Griffbereiche (20R, 20L) angelegten Druck beobachtet;
die Bestimmungsdatenbeschaffungseinheit (40a5) Bestimmungsdaten zum Bestimmen einer Abweichung zwischen einem Sollgehzustand, der auf einem Referenzgehzustand basiert, der ein Gehzustand ist, der als eine Referenz für den Benutzer dient, und einem tatsächlichen Gehzustand des Benutzers und Schwankungen in dem tatsächlichen Gehzustand des Benutzers beschafft; und
die Lerneinheit (40a6) gemäß einem Trainingsdatensatz, der aus einer Kombination der Zustandsvariable und den Bestimmungsdaten gebildet wird, zumindest eine von der von der Korrekturanpassungseinheit (40a3) angepassten Steuerungsanweisung und den durch die Lehrinformationsextrahierungseinheit (40a4) extrahierten Lehrinformationen lernt.

10. Gehhilfevorrichtung (10) nach Anspruch 9, wobei
die Zustandsvariable zumindest eine von:
Griffbereichspositionsdaten, die Positionen der Griffbereiche (20R, 20L) mit Bezug zu dem Rahmen (50) angeben, und die durch eine Griffbereichspositionserfassungseinheit (34R, 34L) erfasst werden;
Griffbereichsneigungsdaten die Neigungsrichtungen und Neigungswinkel der Griffbereiche (20R, 20L) mit Bezug zu dem Rahmen (50) angeben, und die durch eine Griffbereichsneigungserfassungseinheit (33R, 33L), die an den Griffbereichen 20R, 20L) bereitgestellt ist, erfasst werden; und
Griffbereichsdruckdaten, die einen von dem Benutzer an die Griffbereiche (20R, 20L) angelegten Druck angeben, wenn der Benutzer die Griffbereiche (20R, 20L) greift, und die durch eine Griffbereichsdruckerfassungseinheit (25R, 25L), die an den Griffbereichen (20R, 20L) bereitgestellt ist, erfasst werden,
umfasst.

11. Gehhilfevorrichtung (10) nach Anspruch 9 oder 10, wobei:
die Lerneinheit (40a6) die Steuerungsanweisung und die Lehrinformationen gemäß dem Trainingsdatensatz lernt; und
die Lerneinheit (40a6) aufweist
einen Belohnungsberechnungsabschnitt (40a6A), der eine Belohnung basierend auf den Bestimmungsdaten berechnet, und
einen Funktionsaktualisierungsabschnitt (40a6B), der eine Funktion zum Schätzen eines geeigneten Satzes aus der Steuerungsanweisung und den Lehrinformationen aktualisiert, um zumindest eine von der Abweichung und den Schwankungen von einer aktuellen Zustandsvariable basierend auf der Belohnung zu reduzieren.

12. Gehhilfevorrichtung (10) nach Anspruch 11, wobei
die Lerneinheit (40a6) eine Aktionswertdatenkarte entsprechend dem Satz aus der Steuerungsanweisung und den Lehrinformationen basierend auf der Zustandsvariable und der Belohnung aktualisiert.

13. Gehhilfevorrichtung (10) nach Anspruch 11 oder 12, wobei
die Lerneinheit (40a6) des Weiteren einen Absichtsbestimmungsabschnitt (46a6C) umfasst, der basierend auf einem Ergebnis der Lerneinheit (40a6), die ein Lernen gemäß einem Trainingsdatensatz durchführt, eine Anweisung für den Satz aus der Steuerungsanweisung und den Lehrinformationen bestimmt.

14. Gehhilfevorrichtung (10) nach einem der Ansprüche 9 bis 13, wobei
die Lerneinheit (40a6) mit der Steuerungseinheit (40) über ein Netzwerk verbunden ist.

## Revendications

1. Dispositif d'aide à la marche (10) comprenant :
un cadre (50) ;
une paire de parties de bras (30R, 30L) droite et gauche prévues sur le cadre (50) ;
une paire de parties de préhension (20R, 20L) droite et gauche prévues sur la paire de parties de bras (30R, 30L) droite et gauche, les parties de préhension (20R, 20L) étant préhensibles par un utilisateur et mobiles dans une direction avant-arrière par rapport au cadre (50) ;
une pluralité de roues (60FL, 60FR, 60RR, 60RL) prévues à une extrémité inférieure du cadre (50) et comportant une roue motrice (60RR, 60RL) ;
une unité d'entraînement (64R, 64L) qui entraîne la roue motrice (60RR, 60RL) pour amener le dispositif d'aide à la marche (10) à avancer ou à reculer ;
une unité d'entraînement de partie de préhension (32R, 32L) qui est configurée pour déplacer chacune des parties de préhension (20R, 20L) dans la direction avant-arrière par rapport au cadre (50) ;
une batterie (B) qui sert de source de puissance pour l'unité d'entraînement (64R, 64L) et l'unité d'entraînement de partie de préhension (32R, 32L) ;
une unité de commande (40) qui commande l'unité d'entraînement (64R, 64L) ; et
une unité de détection d'état de partie de préhension (81) qui détecte un état de chacune des parties de préhension (20R, 20L), dans lequel
l'unité de commande (40) comporte :
une unité d'observation d'état de partie de préhension (40a1) qui observe un état de partie de préhension, qui est un état de chacune des parties de préhension (20R, 20L), sur la base d'un signal de détection provenant de l'unité de détection d'état de partie de préhension (81) ;
une unité d'évaluation d'état de la marche (40a2) qui évalue un état de la marche de l'utilisateur, sur la base de l'état de partie de préhension observé en utilisant l'unité d'observation d'état de partie de préhension (40a1) ; et
une unité de réglage de correction (40a3) qui règle une instruction de commande pour au moins une parmi l'unité d'entraînement (64R, 64L) et l'unité d'entraînement de partie de préhension (32R, 32L), sur la base de l'état de la marche évalué en utilisant l'unité d'évaluation d'état de la marche (40a2).

2. Dispositif d'aide à la marche (10) selon la revendication 1, dans lequel :
l'unité d'évaluation d'état de la marche (40a2) estime un état de balancement de bras de l'utilisateur, sur la base de l'état de partie de préhension observé ; et
l'unité d'évaluation d'état de la marche (40a2) estime l'état de la marche, sur la base de l'état de balancement de bras estimé de l'utilisateur.

3. Dispositif d'aide à la marche (10) selon la revendication 2, dans lequel :
l'unité d'observation d'état de partie de préhension (40a1) observe l'état de partie de préhension pendant une période d'observation prédéterminée ; et
l'unité d'évaluation d'état de la marche (40a2) évalue l'état de la marche, sur la base de l'état de partie de préhension observé pendant la période d'observation prédéterminée.

4. Dispositif d'aide à la marche (10) selon la revendication 2 ou 3, dans lequel :
l'unité d'évaluation d'état de la marche (40a2) évalue l'état de la marche, sur la base de l'état de partie de préhension, pendant une période excluant :
une période post-début prédéterminée, qui est une période prédéterminée, suivant immédiatement l'instant où l'utilisateur a commencé à marcher en utilisant le dispositif d'aide à la marche (10) ;
une période pré-fin prédéterminée, qui est une période prédéterminée, précédant immédiatement l'instant où l'utilisateur a fini de marcher en utilisant le dispositif d'aide à la marche (10) ; et
une période de virage prédéterminée, qui est une période prédéterminée, avant et après un virage à droite ou un virage à gauche effectué par l'utilisateur en utilisant le dispositif d'aide à la marche (10).

5. Dispositif d'aide à la marche (10) selon l'une quelconque des revendications 1 à 4, dans lequel :
l'état de partie de préhension comporte une longueur de course droite qui est une longueur de course avant-arrière de la partie de préhension droite par rapport au cadre (50), et une longueur de course gauche qui est une longueur de course avant-arrière de la partie de préhension gauche par rapport au cadre (50) ;
l'unité d'évaluation d'état de la marche (40a2) évalue la nécessité d'effectuer une correction d'au moins une parmi la longueur de course droite et la longueur de course gauche dans le cas où un écart entre la longueur de course droite et la longueur de course gauche est égal ou supérieur à un écart de longueur prédéterminé ; et
l'unité de réglage de correction (40a3) règle l'instruction de commande pour l'unité d'entraînement de partie de préhension (32R, 32L), de sorte que la longueur de course droite et la longueur de course gauche soient égales l'une à l'autre dans le cas où l'unité d'évaluation d'état de la marche (40a2) évalue la nécessité d'effectuer la correction.

6. Dispositif d'aide à la marche (10) selon l'une quelconque des revendications 1 à 5, dans lequel :
l'état de partie de préhension comporte une plage de course droite, qui est une plage de course avant-arrière de la partie de préhension droite dans une position dans la direction avant-arrière par rapport au cadre (50), une plage de course gauche, qui est une plage de course avant-arrière de la partie de préhension gauche dans une position dans la direction avant-arrière par rapport au cadre (50), une position de milieu de course droite, qui est une position de milieu dans la direction avant-arrière de la plage de course droite, et une position de milieu de course gauche, qui est une position de milieu dans la direction avant-arrière de la plage de course gauche ;
l'unité d'évaluation d'état de la marche (40a2) évalue la nécessité d'effectuer une correction d'au moins une parmi la position de milieu de course droite et la position de milieu de course gauche, dans le cas où une distance dans la direction avant-arrière, entre la position de milieu de course droite et la position de milieu de course gauche, est égale ou supérieure à un écart de distance prédéterminé ; et
l'unité de réglage de correction (40a3) règle l'instruction de commande pour l'unité d'entraînement de partie de préhension (32R, 32L), de sorte que la position de milieu de course droite et la position de milieu de course gauche soient à la même position dans la direction avant-arrière, dans le cas où l'unité d'évaluation d'état de la marche (40a2) évalue la nécessité d'effectuer la correction.

7. Dispositif d'aide à la marche (10) selon la revendication 5, dans lequel :
l'unité d'évaluation d'état de la marche (40a2) extrait d'une unité de stockage (44), qui stocke des informations de course de référence comportant une longueur de course de référence qui concorde avec des informations personnelles d'utilisateur comportant le sexe, l'âge et des informations sur le corps de l'utilisateur, les informations de course de référence correspondant à l'utilisateur ;
l'unité d'évaluation d'état de la marche (40a2) calcule une longueur de course cible, sur la base de la longueur de course de référence incluse dans les informations de course de référence extraites ;
l'unité d'évaluation d'état de la marche (40a2) évalue pour la longueur de course cible calculée, s'il est nécessaire ou non d'effectuer une correction de chacune de la longueur de course droite et de la longueur de course gauche ; et
dans le cas où l'unité d'évaluation d'état de la marche (40a2) évalue la nécessité d'effectuer la correction, l'unité de réglage de correction (40a3) règle l'instruction de commande pour l'unité d'entraînement de partie de préhension (32R, 32L), de sorte que la longueur de course, pour laquelle l'unité d'évaluation d'état de la marche (40a2) évalue la nécessité d'effectuer la correction, soit mise à la longueur de course cible.

8. Dispositif d'aide à la marche (10) selon l'une quelconque des revendications 1 à 7, comprenant en outre :
une unité d'extraction d'informations d'enseignement (40a4) qui extrait des informations d'enseignement comportant des enseignements sur une correction de l'état de la marche de l'utilisateur ; et
une unité de sortie d'informations d'enseignement (78) qui délivre au moins un parmi un son et une image sur la base des informations d'enseignement, dans lequel :
l'unité d'évaluation d'état de la marche (40a2) estime une posture de l'utilisateur pendant la marche en utilisant le dispositif d'aide à la marche (10) sur la base de l'état de partie de préhension observé, et évalue l'état de la marche comportant la posture estimée de l'utilisateur ;
l'unité d'extraction d'informations d'enseignement (40a4) extrait d'une unité de stockage (44), qui stocke des informations d'enseignement comportant des enseignements sur une correction de l'état de la marche de l'utilisateur, les informations d'enseignement basées sur l'état de la marche évalué par l'unité d'évaluation d'état de la marche (40a2) ; et
l'unité de sortie d'informations d'enseignement (78) délivre au moins un parmi un son et une image sur la base des informations d'enseignement extraites.

9. Dispositif d'aide à la marche (10) selon la revendication 8, comprenant en outre :
une unité d'acquisition de données de détermination (40a5) ; et
une unité d'apprentissage (40a6), dans lequel
l'unité d'observation d'état de partie de préhension (40a1) observe, en tant que variable d'état, au moins une parmi des positions dans la direction avant-arrière des parties de préhension (20R, 20L) par rapport au cadre (50), des directions d'inclinaison et des angles d'inclinaison des parties de préhension (20R, 20L) par rapport au cadre (50), et des pressions appliquées aux parties de préhension (20R, 20L) ;
l'unité d'acquisition de données de détermination (40a5) acquiert des données de détermination pour déterminer un écart entre un état cible de la marche, qui est basé sur un état de référence de la marche qui est un état de la marche servant de référence pour l'utilisateur, et un état réel de la marche de l'utilisateur et des fluctuations de l'état réel de la marche de l'utilisateur ; et
l'unité d'apprentissage (40a6) apprend, suivant un jeu de données de formation constitué d'une combinaison de la variable d'état et des données de détermination, au moins une parmi l'instruction de commande réglée par l'unité de réglage de correction (40a3) et les informations d'enseignement extraites par l'unité d'extraction d'informations d'enseignement (40a4).

10. Dispositif d'aide à la marche (10) selon la revendication 9, dans lequel
la variable d'état comporte au moins une parmi :
des données de position de partie de préhension, qui indiquent des positions des parties de préhension (20R, 20L) par rapport au cadre (50), et qui sont détectées par une unité de détection de position de partie de préhension (34R, 34L) ;
des données d'inclinaison de partie de préhension, qui indiquent des directions d'inclinaison et des angles d'inclinaison des parties de préhension (20R, 20L) par rapport au cadre (50), et qui sont détectées par une unité de détection d'inclinaison de partie de préhension (33R, 33L) prévue pour les parties de préhension (20R, 20L) ; et
des données de pression de partie de préhension, qui indiquent une pression appliquée par l'utilisateur aux parties de préhension (20R, 20L) lorsque l'utilisateur saisit les parties de préhension (20R, 20L), et qui sont détectées par une unité de détection de pression de partie de préhension (25R, 25L) prévue pour les parties de préhension (20R, 20L).

11. Dispositif d'aide à la marche (10) selon la revendication 9 ou 10, dans lequel :
l'unité d'apprentissage (40a6) apprend l'instruction de commande et les informations d'enseignement conformément au jeu de données de formation ; et
l'unité d'apprentissage (40a6) dispose
d'une section de calcul des récompenses (40a6A), qui calcule une récompense sur la base des données de détermination, et
d'une section de mise à jour des fonctions (40a6B), qui met à jour une fonction pour estimer un jeu approprié de l'instruction de commande et des informations d'enseignement pour réduire au moins un parmi l'écart et les fluctuations à partir d'une variable d'état actuelle sur la base de la récompense.

12. Dispositif d'aide à la marche (10) selon la revendication 11, dans lequel
l'unité d'apprentissage (40a6) met à jour une carte des données de valeur d'action correspondant au jeu de l'instruction de commande et des informations d'enseignement, sur la base de la variable d'état et de la récompense.

13. Dispositif d'aide à la marche (10) selon la revendication 11 ou 12, dans lequel
l'unité d'apprentissage (40a6) comporte en outre une section de détermination d'intention (46a6C) qui détermine, sur la base d'un résultat de l'unité d'apprentissage (40a6) réalisant l'apprentissage suivant le jeu de données de formation, une instruction pour le jeu de l'instruction de commande et des informations d'enseignement.

14. Dispositif d'aide à la marche (10) selon l'une quelconque des revendications 9 à 13, dans lequel
l'unité d'apprentissage (40a6) est connectée à l'unité de commande (40) via un réseau.
